# EUROPEAN PATENT APPLICATION

(11) **EP 3 611 161 A1**
(43) Date of publication of application: **19.02.2020**
(21) Application number: 18777447.6
(22) Date of filing: 30.03.2018
(51) Int. Cl.: C07C 311/18, A61K 31/18, A61K 31/27, A61K 31/445, A61K 31/4453, A61K 31/4462, A61K 31/4465, A61K 31/5375, A61P 31/12, A61P 31/14, C07C 311/29, C07D 211/28, C07D 295/13

(54) **BIARYL SULFONAMIDE DERIVATIVE HAVING FILOVIRUS CELL ENTRY INHIBITION ACTIVITY**

(30) Priority: 31.03.2017 JP 2017071729
(71) Applicant: National University Corporation Hokkaido University, Sapporo-shi, Hokkaido 060-0808 (JP)
(72) Inventor: TAKADA, Ayato, Sapporo-shi Hokkaido 060-0808 (JP); SAKAITANI, Masahiro, Sapporo-shi Hokkaido 060-0808 (JP); FURUYAMA, Wakako, Sapporo-shi Hokkaido 060-0808 (JP)
(74) Representative: Forstmeyer, Dietmar
(86) International application number: PCT/JP2018/013581
(87) International publication number: WO 2018/181884

(57) **Abstract**

The objective of the present invention is to provide a novel compound inhibiting the entry of a filovirus into a cell, a medical composition, an anti-filovirus agent, or a filovirus entry inhibitor containing the compound, as well as a method for inhibiting a filovirus cell entry process *in vitro,* a method for treating a filovirus infectious disease, and the like. The objective has been achieved by providing: the compound represented by formula (I) or a hydrate thereof; or a pharmaceutically acceptable salt of the compound or a hydrate thereof.

## Description

### [Technical Field]

The present invention relates to a novel compound that inhibits an invasion by filovirus into a cell, a pharmaceutical composition comprising said compound, an anti-filovirus agent or an inhibitor to cell invasion by a filovirus, or a method of inhibiting cell invasion process by filovirus *in vitro,* a method for treating filovirus infection.

### [Background Arts]

*Filoviridae,* a family of virus having single minus-strand RNA as viral genome, includes genera of *Ebolavirus* and *Marburgvirus.* Viruses of *Filoviridae* (herein refers to as "filovirus") are known to cause acute infections with high fatality in human and monkeys. Filovirus includes viruses of the genera *Ebolavirus* (herein refers to as "Ebola virus") and *Marburgvirus* (herein refers to as "Marburg virus"), and which cause infections known as Ebola hemorrhagic fever and Marburg hemorrhagic fever, respectively.

Nowadays, five genealogical species of Ebola virus: Zaire, Sudan, Tai Forest, Bundibugyo and Reston, and only one species of Marburg virus are known. Ebola hemorrhagic fever and Marburg hemorrhagic fever are repeatedly prevalent in sporadic manner in Africa. Particularly, the epidemic outbreak of Ebola hemorrhagic fever in West Africa from 2013 to 2015 was caused by Zaire. Moreover, because of the unprecedented pandemic outbreak of Ebola hemorrhagic fever in West Africa in 2014 and its spread to Europe and America, the necessity for the development of prophylactic and therapeutic drugs for Ebola virus and its exigency became being aware, and such drugs have been developed in hurry. However, for now, no effective prophylactic/therapeutic drugs against filovirus including Ebola and Marburg viruses have been in practical use.

Non-patent Reference 1 describes that an antibody medicine using a cocktail of various monoclonal antibodies was used for Ebola virus-infected patients in practice, which resulted in a decrease in plasma Ebola virus load over time, though this is unapproved drugs.

Non-patent Reference 2 describes that, in clinical trial in Africa, a nucleic acid analogue Favipiravir (Avigan® tablets) which is a small molecular compound developed as an influenza therapeutic was used for Ebola virus-infected patients in practice, which did not confirm a sufficient therapeutic result.

Non-patent Reference 3 describes that a novel synthetic adenosine analogue BCX4430 inhibited viral RNA polymerase function and thereby inhibited filovirus infection in human cells.

Non-patent Reference 4 describes that a novel small molecule compound GS-5734 which is a monophosphoramide acid prodrug of an adenosine analogue has anti-viral activity against Ebola virus.

Non-patent Reference 5 describes that the effectiveness of T-705 (Favipiravir), a pyrazine carboxamide derivative against Ebola virus (Zaire), was validated and the results indicated that it suppressed replication of said virus in cells and led to a rapid virus clearance in mouse.

Non-patent Reference 6 describes that two novel invasion inhibitors that inhibit Ebola virus infection (MBX2254 and MBX2270) were identified.

### [Prior Art References]

### [Non-patent References]

[Non-patent Reference 1] Lyon GM, et al. Clinical care of two patients with Ebola virus disease in the United States. N. Engl. J. Med., 371(25):2402-2409, 2014
[Non-patent Reference 2] Sissoko D, et al. Experimental treatment with favipiravir for Ebola virus disease (the JIKI Trial): a historically controlled, single-arm proof-of-concept trial in Guinea. PLOS Med., 13(3):e1001967, 2016
[Non-patent Reference 3] Warren TK, et al. Protection against filovirus diseases by a novel broad-spectrum nucleoside analogue BCX4430. Nature, 508(7496):402-405, 2014
[Non-patent Reference 4] Warren TK, et al. Therapeutic efficacy of the small molecule GS-5734 against Ebola virus in rhesus monkeys. Nature, 531(7594):381-385, 2016
[Non-patent Reference 5] Oestereich L, et al. Successful treatment of advanced Ebola virus infection with T-705 (favipiravir) in a small animal model. Antiviral Res., 105:17-21, 2014
[Non-patent Reference 6] Basu A, et al. Novel small molecule entry inhibitors of Ebola virus. J. Infect. Dis., 212(Suppl 2):S425-S434, 2015

### [Summary of the Invention]

### [Problems to be Solved by the Invention]

The present invention is aimed at providing a novel compound that inhibits the invasion by filovirus into a cell, a pharmaceutical composition comprising said compound, an anti-filovirus agent or an inhibitor to the cell invasion by filovirus, or a method of inhibiting the cell invasion process by filovirus *in vitro,* and a method for treating filovirus infection, etc.

### [Means to Solve the Problems]

Because the antigenicity of filoviruses greatly differ between species, the majority of monoclonal antibodies ever generated worldwide is specific to Zaire, including the antibodies described in Non-patent Reference 1, and not effective to other filoviruses. Moreover, the development of antibody medicine harbors problems of heavy restrictions by the limit of mass production and cost. On the other hand, Favipiravir was expected to be an effective RNA polymerase inhibitor against wide variety of RNA viruses including filovirus. Although its anti-viral effect was confirmed in a mouse model of Ebola virus infection, as described in Non-patent Reference 2, the clinical trial in Africa did not confirm a sufficient anti-viral effect. Moreover, it has been confirmed that Favipiravir has a side effect, and it is therefore known that the use of Favipiravir is limited even as an influenza therapeutic.

Here, the inventors aimed at putting an effective next-generation prophylactic/therapeutic drug against filoviruses into practical use. The inventors focused on the mechanism of cell invasion by filovirus and continued a research, and in due course found that a biaryl sulfonamide derivative inhibited the cell invasion process by filovirus. The inventors continued the investigation based on such findings, analyzed structure-activity correlations for various biaryl sulfonamide derivatives, optimized structures of these compounds, and further continued the investigation to complete the present invention.

Namely, the present invention relates to those listed below:
[1] A compound of Formula (I): 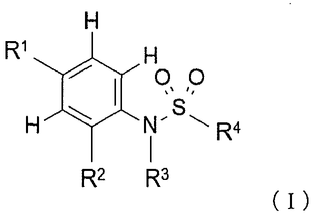 wherein
R¹ is H, Hal, Alk or OAlk,
R² is NO₂, Alk or Ar,
R³ is 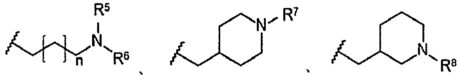
or 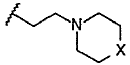 wherein,
n is any of 0 to 5,
R⁵ to R⁸ are each independently H, Alk or COOAlk,
X is CH₂ or O,
R⁴ is 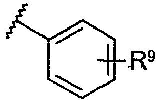
or a naphthyl group, wherein,
R⁹ is H, Hal, Alk or OAlk,
wherein,
Alk is each independently a linear or branched alkyl group having 1 to 10 carbon atoms,
Hal is each independently a halogen,
Ar is each independently an aryl group which may optionally be substituted with Hal,
or a hydrate thereof or a pharmaceutically acceptable salt of the compound or a hydrate thereof.
[2] The compound of [1] or a hydrate thereof or a pharmaceutically acceptable salt of the compound or a hydrate thereof, wherein n is 0 or 1.
[3] The compound of [1] or [2] or a hydrate thereof or a pharmaceutically acceptable salt of the compound or a hydrate thereof, wherein Alk is a linear or branched alkyl group having 1 to 4 carbon atoms.
[4] The compound of any one of [1] to [3] or a hydrate thereof or a pharmaceutically acceptable salt of the compound or a hydrate thereof, wherein Ar is an aryl group substituted with Hal.
[5] The compound of any one of [1] to [4] or a hydrate thereof or a pharmaceutically acceptable salt of the compound or a hydrate thereof, wherein Hal is F.
[6] The compound of any one of [1] to [5] or a hydrate thereof or a pharmaceutically acceptable salt of the compound or a hydrate thereof, wherein R⁴ is a phenyl group, a fluorinated phenyl group, a phenyl group substituted with a methyl group or methoxy group, 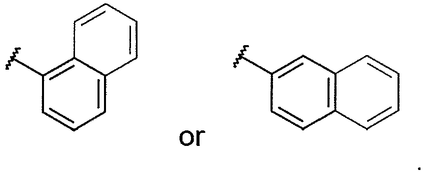
[7] The compound of [1] or a hydrate thereof or a pharmaceutically acceptable salt of the compound or a hydrate thereof, wherein the compound is of the following chemical structure formulae:

**[Table 1-1]**

| No. | Name | Chemical structural formula |
|---|---|---|
| 4-21 | N-(4-methoxy-2-phenyl-phenyl)-N-[1-ethyl-4-piperidyl]methyl]benzensulfonamide | 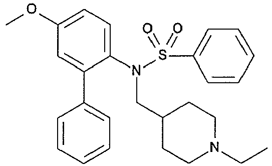 |
| 4-17 | N-[3-(diethylamino)propyl]-N-(4-methoxy-2-phenyl-phenyl)benzensulfonamide | 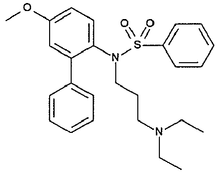 |
| 4-20 | N-(4-methoxy-2-phenyl-phenyl)-N-[1-methyl-4-piperidyl]methyl]benzensulfonamide | 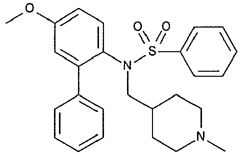 |
| 4-27 | N-[3-(dimethylamino)propyl]-N-(4-methoxy-2-phenyl-phenyl)benzensulfonamide | 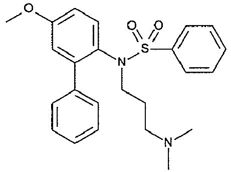 |
| 4-51 | N-[2-(4-fluorophenyl)-4-methoxyphenyl)-N-(1-ethyl-4-piperidylmethyl)benzensulfonamide | 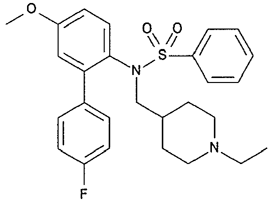 |
| 4-54 | N-[2-(3-fluorophenyl)-4-methoxyphenyl)-N-(1-ethyl-4-piperidylmethyl)benzensulfonamide | 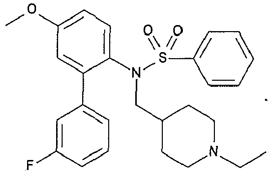 |

**[Table 1-2]**

| | | |
|---|---|---|
| 4-63 | 3-fluoro-N-(4-methoxy-2-phenyl-phenyl)-N-(1-ethyl-4-piperidylmethyl)benzensulfonamide | 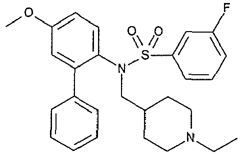 |
| 4-36 | N-[1-isopropyl-4-piperidyl]methyl]-N-(4-methoxy-2-phenyl-phenyl)benzensulfonamide | 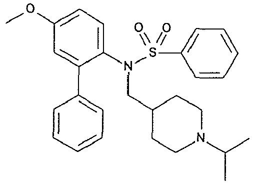 |
| 4-25 | N-(4-methoxy-2-phenyl-phenyl)-N-[1-ethyl-3-piperidyl]methyl]benzensulfonamide | 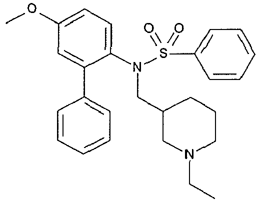 |
| 4-57 | N-[2-(2-fluorophenyl)-4-methoxyphenyl)-N-(1-ethyl-4-piperidylmethyl)benzensulfonamide | 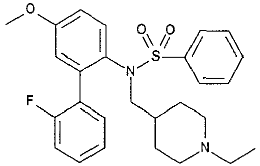 |
| 4-66 | 2-fluoro-N-(4-methoxy-2-phenyl-phenyl)-N-(1-ethyl-4-piperidylmethyl)benzensulfonamide | 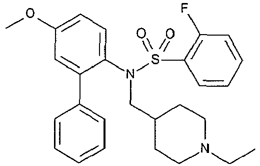 |
| 4-42 | N-(4-methoxy-2-phenyl-phenyl)-N-[1-methyl-3-piperidyl]methyl]benzensulfonamide | 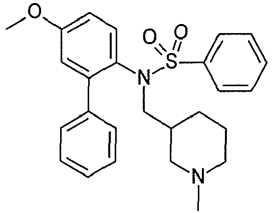 |

**[Table 1-3]**

| | | |
|---|---|---|
| 4-60 | 4-fluoro-N-(4-methoxy-2-phenyl-phenyl)-N-(1-ethyl-4-piperidylmethyl)benzensulfonamide | 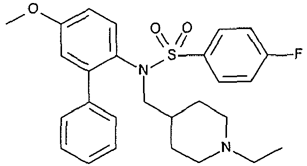 |
| 4-14 | 4-methoxy-N-[3-(diethylamino)propyl]-N-(2-phenyl-phenyl)benzensulfonamide | 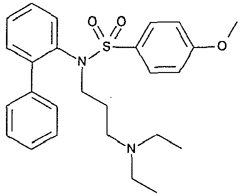 |
| 4-1 | N-[3-(diethylamino)propyl]-N-(4-methoxy-2-nitrophenyl)benzensulfonamide | 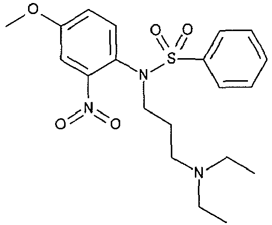 |
| 4-11 | 4-methoxy-N-[3-(diethylamino)propyl]-N-(4-methoxy-2-nitrophenyl)benzensulfonamide | 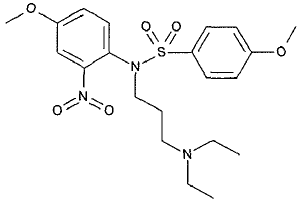 |
| 4-33 | N-[3-(dimethylamino)ethyl]-N-(4-methoxy-2-phenyl-phenyl)benzensulfonamide | 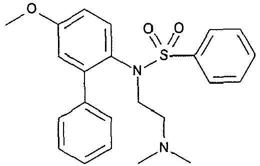 |
| 4-8 | N-[3-(diethylamino)propyl]-N-(2-phenyl-phenyl)benzensulfonamide | 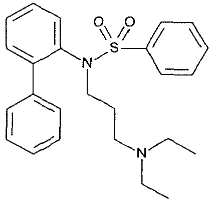 |

**[Table 1-4]**

| | | |
|---|---|---|
| 4-9 | N-[3-(diethylamino)propyl]-N-(4-methoxy 2-nitro-phenyl)naphthalene-1-sulfonamide | 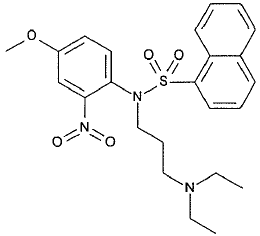 |
| 4-28 | N-[3-(diethylamino)ethyl]-N-(4-methoxy-2-phenyl-phenyl)benzensulfonamide | 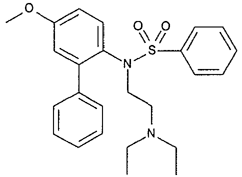 |
| 4-4 | 3-fluoro-N-[3-(diethylamino)propyl]-N-(4-methoxy-2-nitrophenyl)benzensulfonamide | 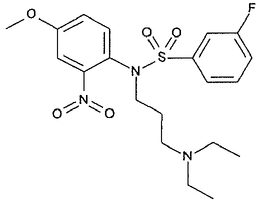 |
| 4-30 | N-(4-methoxy-2-phenyl-phenyl)-N-[3-(methylamino)propyl]-benzensulfonamide | 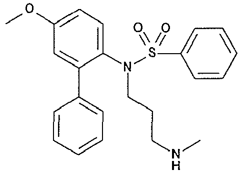 |
| 4-7 | 2-methyl-N-[3-(diethylamino)propyl]-N-(4-methoxy-2-nitrophenyl)benzensulfonamide | 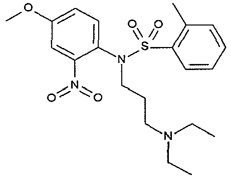 |
| 4-32 | N-(3-aminopropyl)-N-(4-methoxy-2-phenyl-phenyl)benzensulfonam ide | 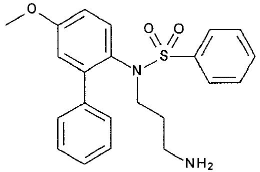 |

**[Table 1-5]**

| | | |
|---|---|---|
| 4-5 | 2-fluoro-N-[3-(diethylamino)propyl]-N-(4-methoxy-2-nitrophenyl)benzensulfonamide | 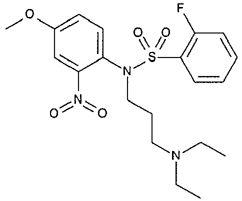 |
| 4-3 | 4-fluoro-N-[3-(diethylamino)propyl]-N-(4-methoxy-2-nitrophenyl)benzensulfonamide | 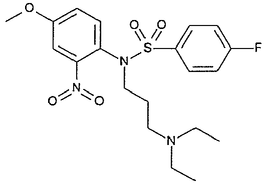 |
| 4-19 | N-(4-methoxy-2-phenyl-phenyl)-N-(4-piperidylmethyl)benzensulfonamide | 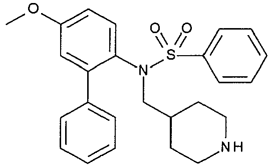 |
| 4-6 | 3-methyl-N-[3-(diethylamino)propyl]-N-(4-methoxy-2-nitrophenyl)benzensulfonamide | 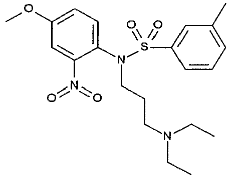 |
| 4-45 | N-(4-isopropyl-2-phenyl-phenyl)-N-(1-ethyl-4-piperidylmethyl)benzensulfonamide | 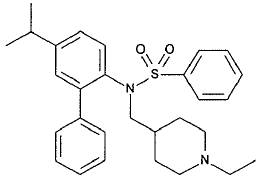 |
| 4-10 | N-[3-(diethylamino)propyl]-N-(4-methoxy 2-nitro-phenyl)naphthalene-2-sulfonamide | 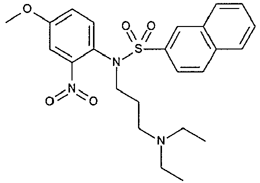 |

**[Table 1-6]**

| | | |
|---|---|---|
| 4-23 | N-(4-methoxy-2-phenyl-phenyl)-N-(3-piperidylmethyl)benzensulfonamide | 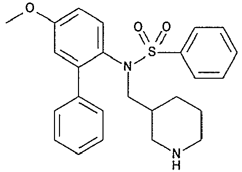 |
| 4-15 | N-[3-(diethylamino)propyl]-N-(2-isopropyl-phenyl)benzensulfonamide | 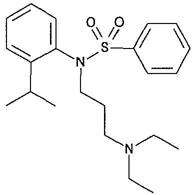 |
| 4-35 | N-(4-methoxy-2-phenyl-phenyl)-N-[2-(1-piperidyl)ethyl]benzensulfonamide | 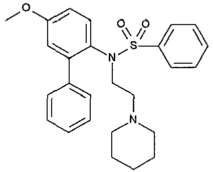 |
| 4-29 | t-butyl-N-[3-[N-(benzensulfonyl)-4-methoxy-2-phenyl-anilino]propyl]-N-methylcarbamate | 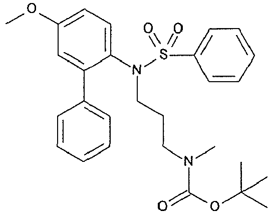 |
| 4-34 | N-(4-methoxy-2-phenyl-phenyl)-N-(2-morpholinoethyl)benzensulfonamide | 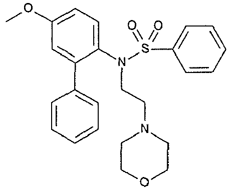 |
| 4-16 | N-[3-(diethylamino)propyl]-N-(2-t-butylphenyl)benzensulfonamide | 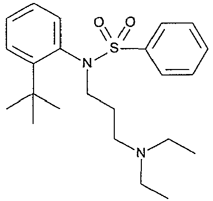 |

**[Table 1-7]**

| | | |
|---|---|---|
| 4-31 | t-butyl-N-[3-[N-(benzensulfonyl)-4-methoxy-2-phenyl-anilino]propyl]carbamate | 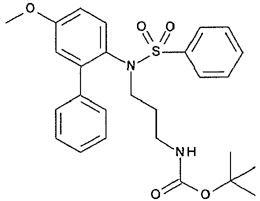 |
| 4-22 | t-butyl-3-[[N-(benzensulfonyl)-4-methoxy-2-phenyl-anilino]methyl]piperidine-1-carboxylate | 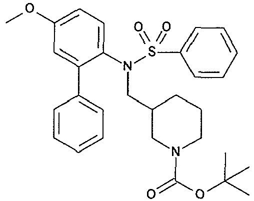 |
| 4-39 | N-(4-ethoxy-2-phenyl-phenyl)-N-[1-ethyl-4-piperidyl]methyl]benzensulfonamide | 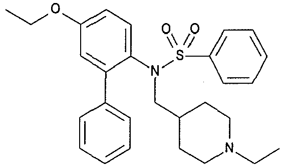 |
| 4-13 | N-[3-(diethylamino)propyl]-N-(2-phenyl-phenyl)naphthalene-1-sulfonamide | 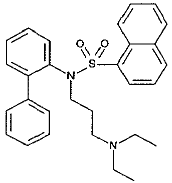 |
| 4-18 | t-butyl-4-[[N-(benzensulfonyl)-4-methoxy-2-phenyl-anilino]methyl]piperidine-1-carboxylate | 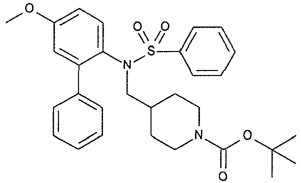 |
| 4-2 | N-[3-(diethylamino)propyl]-N-(2-nitrophenyl)benzensulfonamide | 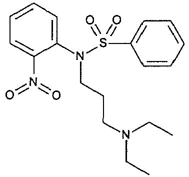 |

**[Table 1-8]**

| | | |
|---|---|---|
| 4-48 | N-(4-t-butyl-2-phenyl-phenyl)-N-(1-ethyl-4-piperidylmethyl)benzensulfonamide | 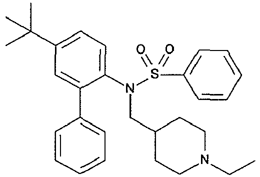 |
| 4-67 | N-[3-(dimethylamino)propyl]-N-[2-(4-fluorophenyl)-4-methoxyphenyl)benzensulfonamide | 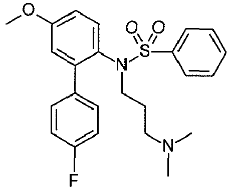 |
| 4-68 | N-[3-(diethylamino)propyl]-N-[2-(4-fluorophenyl)-4-methoxyphenyl)benzensulfonamide | 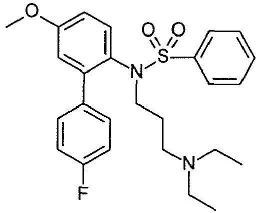 |

[8] A pharmaceutical composition comprising the compound of any one of [1] to [7] or a hydrate thereof or a pharmaceutically acceptable salt of the compound or a hydrate thereof.
[9] The pharmaceutical composition of [8], wherein the pharmaceutical composition is for prophylaxis and/or treatment of a viral infection.
[10] The pharmaceutical composition of [9], wherein the viral infection is an acute viral infection.
[11] The pharmaceutical composition of [10], wherein the acute viral infection is Ebola hemorrhagic fever.
[12] The pharmaceutical composition of [9], wherein the virus is a filovirus.
[13] The pharmaceutical composition of [12], wherein the filovirus is Ebola virus.
[14] An antiviral agent comprising the compound of any one of [1] to [7] or a hydrate thereof or a pharmaceutically acceptable salt of the compound or a hydrate thereof.
[15] A viral cell-invasion inhibitor comprising the compound of any one of [1] to [7] or a hydrate thereof or a pharmaceutically acceptable salt of the compound or a hydrate thereof.
[16] A method of inhibiting viral cell-invasion processes *in vitro* using the compound of any one of [1] to [7] or a hydrate thereof or a pharmaceutically acceptable salt of the compound or a hydrate thereof.

### [Effects by the Invention]

The compounds of the present invention can inhibit the invasion by filoviruses including Ebola and Marburg viruses into cells, and thereby can inhibit replication of filoviruses in cells. Moreover, the inhibitory effect of the compounds of the present invention against the invasion by filoviruses into cells is specific to filoviruses and accompanying low toxicity, and thus the compounds of the present invention can be used for prophylaxis and therapy for infections associated with filovirus, e.g., Ebola hemorrhagic fever and Marburg hemorrhagic fever, with extremely high selectivity. Moreover, the compound of the present invention can inhibit the invasion into cells by a filovirus regardless of its species as long as it is filovirus, and thus can be used in general as an antiviral agent against filovirus. Furthermore, the compound of the present invention is a biaryl sulfonamide derivative which corresponds to a small molecule compound, and therefore can be industrially produced at lower cost as compared to antibody medicines, can be supplied to domestic and oversea markets including African countries that are principal epidemic areas of the infections related to filovirus.

### [Brief Description of Drawings]

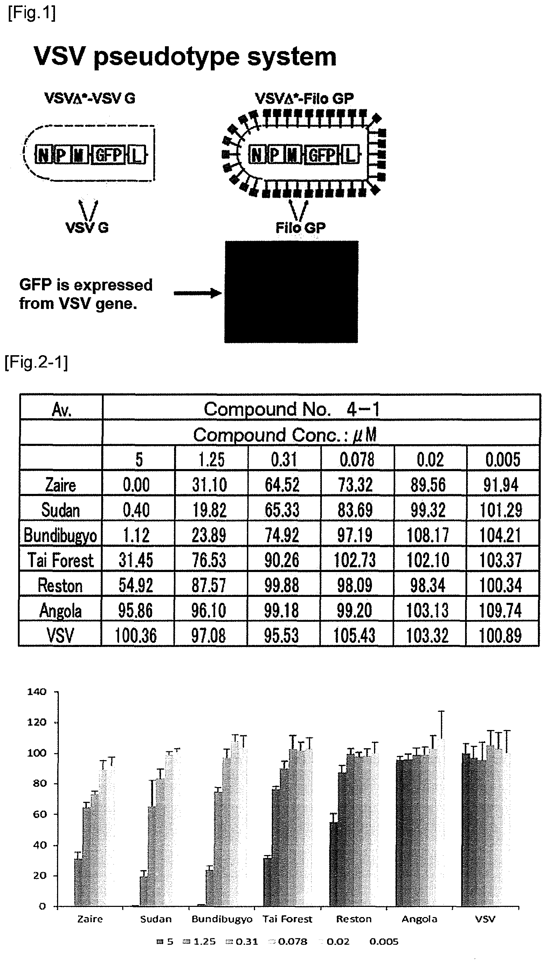

[Fig.1] Fig.1 shows a schematic diagram of vesicular stomatitis virus (VSV) pseudotype system that enables the measurement of the inhibitory activity to the cell invasion by filovirus.

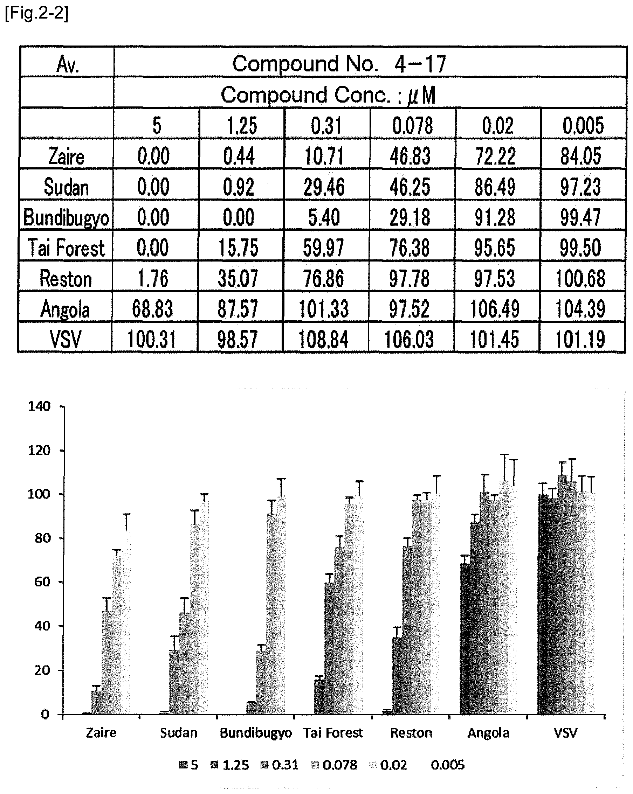

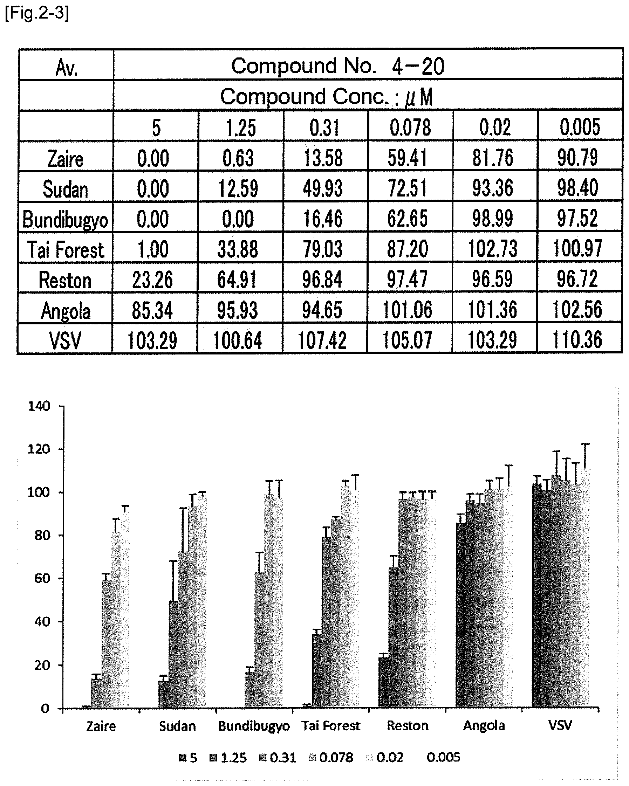

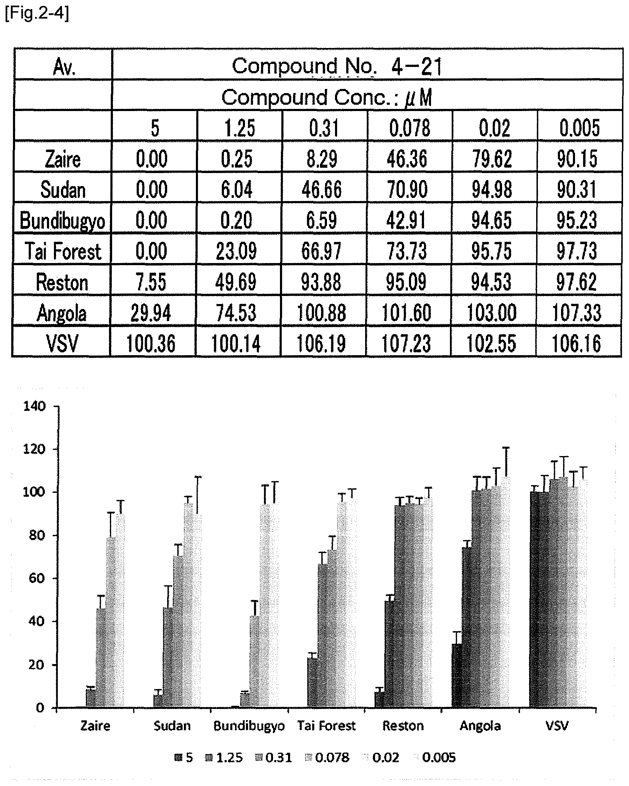

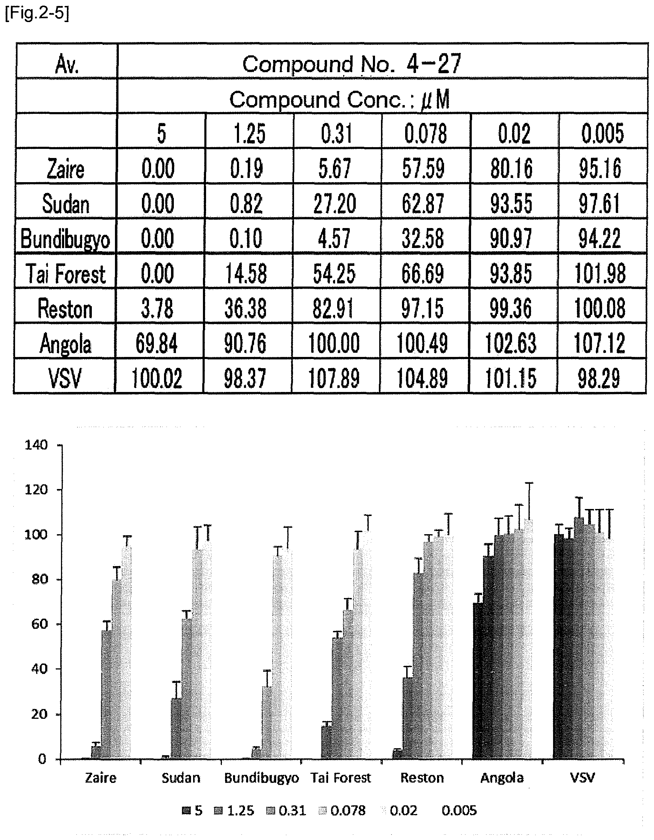

[Fig.2] Fig.2-1 to 2-5 show measurement results of cell-invasion inhibitory activities of various compounds of the present invention (4-1, 4-17, 4-20, 4-21 and 4-27) against various Ebola viruses (Zaire, Sudan, Bundibugyo, Tai Forest and Reston) and a Marburg virus (Angola). The upper row indicates the inhibitory activity of each compound at each concentration (average value of the measurements obtained from three assays), the lower row indicates a graph in which the inhibitory activities were plotted (error bars indicating standard deviations). The inhibitory activity is indicated as a relative value (%) to the measurement obtained for a control in which the compound was not added.

### [Description of Embodiments]

Hereinbelow, the present invention will be explained in details.

In the present specification, unless defined otherwise, all technical terms and scientific terms used herein have the same meanings as those which are usually understood by a skilled person. All patents, applications, published applications and other publications cited herein are herein incorporated by reference in their entirety. When there is a contradiction herein between the cited publication and the description of the present specification, the description of the present specification shall control.

### <1> Compounds of the present invention

The present invention in one aspect relates to a compound of the following Formula (I): 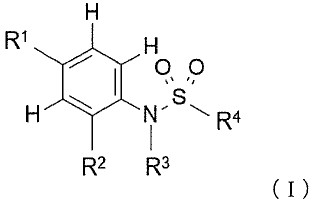 wherein,
R¹ is H, Hal, Alk or OAlk,
R² is NO₂, Alk or Ar,
R³ is 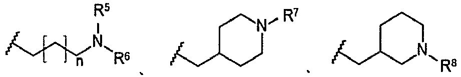
or 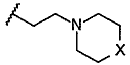 wherein,
n is any of 0 to 5,
R⁵ to R⁸ are each independently H, Alk or COOAlk,
X is CH₂ or O,
R⁴ is 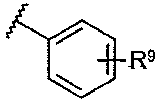
or a naphthyl group, wherein,
R⁹ is H, Hal, Alk or OAlk,
or a hydrate thereof or a pharmaceutically acceptable salt of the compound or a hydrate thereof.

"Alk" denotes a linear or branched alkyl group having 1 to 10 carbon atoms, preferably a linear or branched alkyl group having 1 to 4 carbon atoms. Specifically, it includes such as, for example, a methyl group, ethyl group, isopropyl group, t-butyl group, n-butyl group, isobutyl group or s-butyl group, preferably such as a methyl group, ethyl group, isopropyl group or t-butyl group.

"OAlk" denotes an alkoxy group in which a linear or branched alkyl group having 1 to 10 carbon atoms is bound to an oxygen, preferably a linear or branched alkoxy group having 1 to 4 carbon atoms. Specifically, it includes such as, for example, a methoxy group, etoxy group, isopropoxy group, n-butoxy group, isobutoxy group, s-butoxy group or t-butoxy group, preferably a methoxy group or etoxy group.

"COOAlk" denotes an alkoxycarbonyl group in which a linear or branched alkyl having 1 to 10 carbon atoms is bound to an ester, preferably a linear or branched alkoxycarbonyl group having 1 to 4 carbon atoms. Specifically, it includes such as, for example, methoxycarbonyl group, etoxycarbonyl group, isopropoxycarbonyl group, n-butoxycarbonyl group, isobutoxycarbonyl group, s-butoxycarbonyl group or t-butoxycarbonyl group, preferably t-butoxycarbonyl group.

"Hal" means a halogen such as F, Cl, Br, I. A preferred halogen is F.

"Ar" denotes an aryl group which may be substituted with any substituent. A preferred substituent is Hal. In the invention of the present application, an "aryl" or "aryl group" refers to an aromatic cyclic group derived from aromatic hydrocarbons such as a monocyclic ring, a condensed ring or a polycyclic ring in which monocycles are bound by single bonds, and preferably includes such as phenyl group, biphenyl group and naphthyl group.

R¹ is H, Hal, Alk or OAlk, preferably Alk or OAlk. In certain embodiment, R¹ is preferably H, ethyl group, isopropyl group, t-butyl group, methoxy group or etoxy group.

R² is NO₂, Alk or Ar, preferably Ar. In certain embodiment, R² is preferably NO₂, isopropyl group, t-butyl group, aryl group or fluorinated aryl group.

R³ is 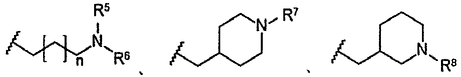 or 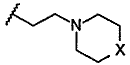 , and it is preferably 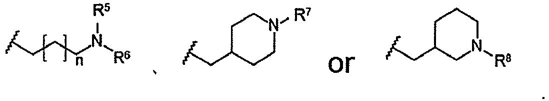
n is any of 0 to 5, preferably 0 or 1.
NR⁵R⁶ is preferably NH₂, NHCH₃, N(CH₃)₂, N(CH₂CH₃)₂, NHCOO-t-butyl or NCH₃COO-t-butyl.
R⁵ to R⁸ are each independently H, Alk or COOAlk, preferably Alk. In certain embodiment, R⁵ to R⁸ are preferably H, a methyl group, ethyl group, isopropyl group or COO-t-butyl.
Y is CH₂ or O, preferably CH₂.
R⁴ is 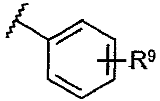
or a naphthyl group, and preferably

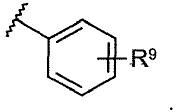

In certain embodiment, R⁴ is preferably a phenyl group, a fluorinated phenyl group, a phenyl substituted with methyl group or methoxy group,

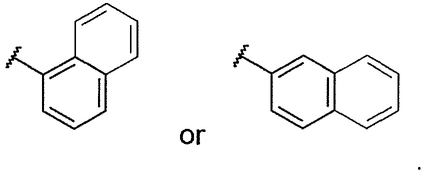

R⁹ is H, Hal, Alk or OAlk, preferably H or Hal, more preferably H or F. In certain embodiment, R⁹ is preferably H, F, a methyl group or methoxy group.

Combining preferred embodiments regarding the substituents described above to each other will provide particularly preferred embodiments of the compound of the present invention.

The compound of Formula (I) or a salt thereof may be an anhydrate or may form a solvate such as a hydrate. "Solvation" here refers to a phenomenon that a solute molecule or ion in solution strongly attracts its adjacent solvent molecules to form one molecular population, and called hydration when the solvent is water, for example. A solvate may be a hydrate or non-hydrate. As a non-hydrate, alcohols (e.g., methanol, ethanol, n-propanol), dimethylformamide, etc. can be used.

The compound of the present invention can be used for prophylaxis and therapy of an infection associated with filovirus, as mentioned above. Therefore, a compound of Formula (I) or a solvate thereof or a pharmaceutically acceptable salt of the compound or a solvate thereof is also encompassed in the present invention. When a "compound of the present invention" is referred herein, unless described otherwise, it is understood that not only the compound of Formula (I) but also a solvate thereof or a pharmaceutically acceptable salt of the compound or a solvate thereof are also encompassed.

Pharmaceutically acceptable salts of the compound of Formula (I) include, for example, a hydrochloride, hydrobromide, hydroiodide, phosphate, phosphonate, sulfate, sulfonates such as methanesulfonate and p-toluenesulfonate, carbonates such as acetate, citrate, malate, tartrate, succinate and salicylate, or alkali metal salts such as sodium salt and potassium salt; alkali earth metal salts such as magnesium salt and calcium salt; and ammonium salts such as ammonium salt, alkyl ammonium salt, dialkyl ammonium salt, trialkyl ammonium salt and tetraalkyl ammonium salt. These salts can be produced by methods known in the art such as, for example, bringing the compound into contact with an acid or base that can be used in the production of a pharmaceutical product.

When the compound of the present invention is obtained as a free body, it can be converted to a state of a salt that may be formed by the compound or a hydrate or solvate thereof according to a conventional method.

When the compound of the present invention is obtained as a salt, hydrate or solvate of the compound, it can be converted to a free salt of the compound according to a conventional method.

The compounds of the present invention encompass any of the compounds of Formula (I) including any isotope. A compound of the present invention including (an) isotope(s) is that in which at least one atom has been substituted with different atom having the same atomic number (the proton number) and different mass number (the sum of the numbers of protons and neutrons). Examples of atoms that have isotopes which can be included in the compounds of the present invention include H, C, N, O, P, S, F, Cl, etc., which include ²H,³H,¹³C,¹⁴C,¹⁵N,¹⁷O,¹⁸O,³⁵S,¹⁸F, etc., respectively. In particular, radioisotopes that emit radiation or positive electrons and collapse are useful, e.g., for tissue distribution examination of a pharmaceutical product or a compound.

Stable isotopes can be used safely because they will not collapse, and are not radioactive. A compound of the present invention can be converted to that which includes an isotope according to a conventional method by replacing a reagent used in synthesis with a reagent which comprise corresponding isotope.

Moreover, the compounds of the present invention encompass forms of prodrugs known in the art. Here, a "prodrug" of a compound of the present invention means a derivative of the compound of Formula (I) which, upon administration, will be converted to the compound of Formula (I) or a pharmaceutically acceptable salt thereof by enzymatic or nonenzymatic degradation under physiological condition. A prodrug may be inactive when it was administered to a patient, but it is present in the living organism as a converted active compound of Formula (I).

Prodrugs known in the art include, for example, those which change to the desired drug form at particular pH or by enzymatic effect. A typical prodrug is a compound which produces a free acid in a living organism, for example, a compound having a hydrolyzable ester residue which is hydrolyzed *in vivo* to produce a free acid. Such hydrolyzable ester residues include, but not limited to, for example, a residue having a carboxyl moiety in which a free hydrogen in the carboxyl group is substituted with C₁-C₄ alkyl group, C₂-C₇ alkanoyloxymethyl group, C₄-C₉ 1-(alkanoyloxy)ethyl group, C₅-C₁₀ 1-methyl-1-(alkanoyloxy)-ethyl group, C₃-C₆ alkoxycarbonyloxymethyl group, C₄-C₇ 1-(alkoxycarbonyloxy)ethyl group, C₅-C₈ 1-methyl-1-(alkoxycarbonyloxy)ethyl group, C₃-C₉ N-(alkoxycarbonyl)aminomethyl, C₄-C₁₀ 1-(N-(alkoxycarbonyl)amino)ethyl group, 3-phthalidyl group, 4-crotonolactonyl group, γ-butyrolactone-4-yl group, di-N,N-(C₁₋₂)alkylamino(C₂₋₃)alkyl group (for example, N,N-dimethylamimoethyl group), carbamoyl-(C₁₋₂)alkyl group, N,N-di(C₁₋₂)alkylcarbamoyl-(C₁₋₂)alkyl group, piperidino(C₂₋₃)alkyl group, pyrrolidino(C2-3)alkyl group, or morpholino(C₂₋₃)alkyl group.

The compounds described in the tables below are the compounds whose cell invasion inhibitory activity has actually been demonstrated by the inventors. Accordingly, preferred compounds among the compounds of the present invention include the group of compounds described in the tables below:

**[Table 2-1]**

| No. | Name | Chemical structural formula |
|---|---|---|
| 4-21 | N-(4-methoxy-2-phenyl-phenyl)-N-[1-ethyl-4-piperidyl]methyl]benzensulfonamide | 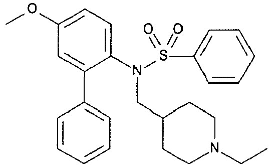 |
| 4-17 | N-[3-(diethylamino)propyl]-N-(4-methoxy-2-phenyl-phenyl)benzensulfonamide | 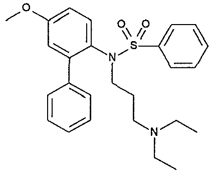 |
| 4-20 | N-(4-methoxy-2-phenyl-phenyl)-N-[1-methyl-4-piperidyl]methyl]benzensulfonamide | 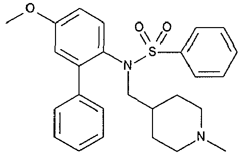 |
| 4-27 | N-[3-(dimethylamino)propyl]-N-(4-methoxy-2-phenyl-phenyl)benzensulfonamide | 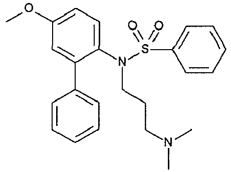 |
| 4-51 | N-[2-(4-fluorophenyl)-4-methoxyphenyl)-N-(1-ethyl-4-piperidylmethyl)benzensulfonamide | 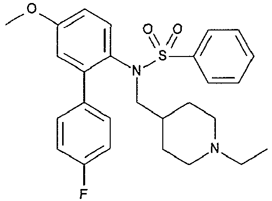 |
| 4-54 | N-[2-(3-fluorophenyl)-4-methoxyphenyl)-N-(1-ethyl-4-piperidylmethyl)benzensulfonamide | 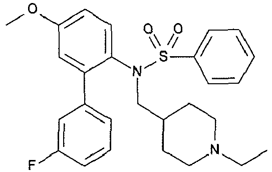 |

**[Table 2-2]**

| | | |
|---|---|---|
| 4-63 | 3-fluoro-N-(4-methoxy-2-phenyl-phenyl)-N-(1-ethyl-4-piperidylmethyl)benzensulfonamide | 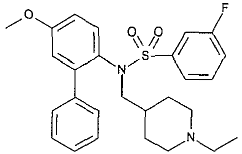 |
| 4-36 | N-[1-isopropyl-4-piperidyl]methyl]-N-(4-methoxy-2-phenyl-phenyl)benzensulfonamide | 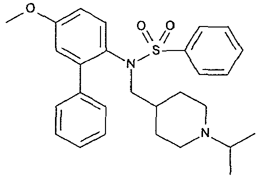 |
| 4-25 | N-(4-methoxy-2-phenyl-phenyl)-N-[1-ethyl-3-piperidyl]methyl]benzensulfonamide | 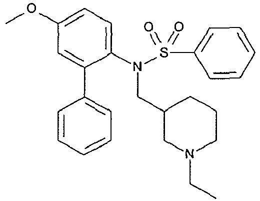 |
| 4-57 | N-[2-(2-fluorophenyl)-4-methoxyphenyl)-N-(1-ethyl-4-piperidylmethyl)benzensulfonamide | 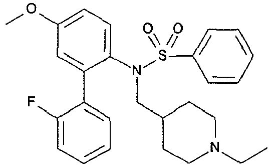 |
| 4-66 | 2-fluoro-N-(4-methoxy-2-phenyl-phenyl)-N-(1-ethyl-4-piperidylmethyl)benzensulfonamide | 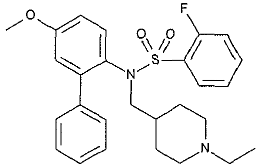 |
| 4-42 | N-(4-methoxy-2-phenyl-phenyl)-N-[1-methyl-3-piperidyl]methyl]benzensulfonamide | 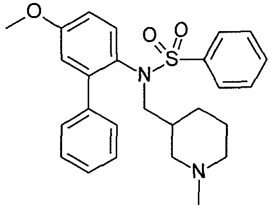 |

**[Table 2-3]**

| | | |
|---|---|---|
| 4-60 | 4-fluoro-N-(4-methoxy-2-phenyl-phenyl)-N-(1-ethyl-4-piperidylmethyl)benzensulfonamide | 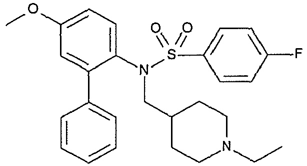 |
| 4-14 | 4-methoxy-N-[3-(diethylamino)propyl]-N-(2-phenyl-phenyl)benzensulfonamide | 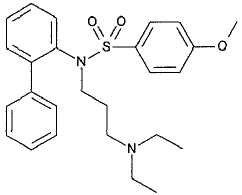 |
| 4-1 | N-[3-(diethylamino)propyl]-N-(4-methoxy-2-nitrophenyl)benzensulfonamide | 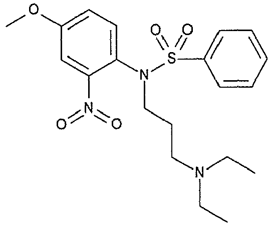 |
| 4-11 | 4-methoxy-N-[3-(diethylamino)propyl]-N-(4-methoxy-2-nitrophenyl)benzensulfonamide | 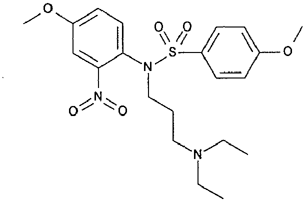 |
| 4-33 | N-[3-(dimethylamino)ethyl]-N-(4-methoxy-2-phenyl-phenyl)benzensulfonamide | 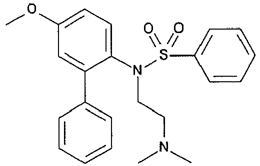 |
| 4-8 | N-[3-(diethylamino)propyl]-N-(2-phenyl-phenyl)benzensulfonamide | 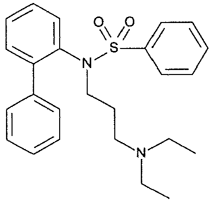 |

**[Table 2-4]**

| | | |
|---|---|---|
| 4-9 | N-[3-(diethylamino)propyl]-N-(4-methoxy 2-nitro-phenyl)naphthalene-1-sulfonamide | 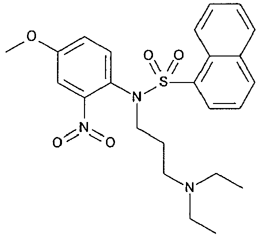 |
| 4-28 | N-[3-(diethylamino)ethyl]-N-(4-methoxy-2-phenyl-phenyl)benzensulfonamide | 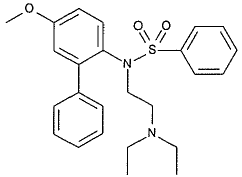 |
| 4-4 | 3-fluoro-N-[3-(diethylamino)propyl]-N-(4-methoxy-2-nitrophenyl)benzensulfonamide | 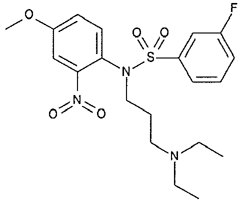 |
| 4-30 | N-(4-methoxy-2-phenyl-phenyl)-N-[3-(methylamino)propyl]-benzensulfonamide | 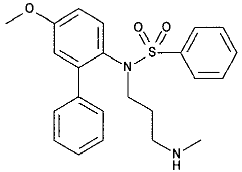 |
| 4-7 | 2-methyl-N-[3-(diethylamino)propyl]-N-(4-methoxy-2-nitrophenyl)benzensulfonamide | 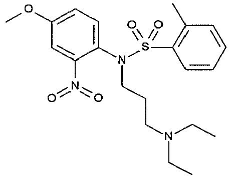 |
| 4-32 | N-(3-aminopropyl)-N-(4-methoxy-2-phenyl-phenyl)benzensulfonamide | 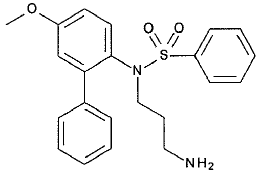 |

**[Table 2-5]**

| | | |
|---|---|---|
| 4-5 | 2-fluoro-N-[3-(diethylamino)propyl]-N-(4-methoxy-2-nitrophenyl)benzensulfonamide | 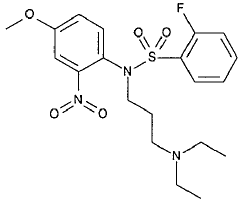 |
| 4-3 | 4-fluoro-N-[3-(diethylamino)propyl]-N-(4-methoxy-2-nitrophenyl)benzensulfonamide | 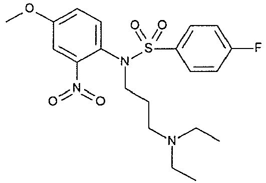 |
| 4-19 | N-(4-methoxy-2-phenyl-phenyl)-N-(4-piperidylmethyl)benzensulfonamide | 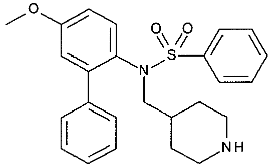 |
| 4-6 | 3-methyl-N-[3-(diethylamino)propyl]-N-(4-methoxy-2-nitrophenyl)benzensulfonamide | 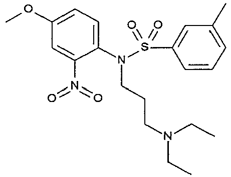 |
| 4-45 | N-(4-isopropyl-2-phenyl-phenyl)-N-(1-ethyl-4-piperidylmethyl)benzensulfonamide | 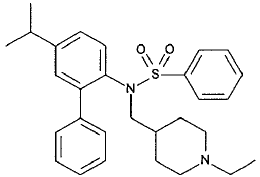 |
| 4-10 | N-[3-(diethylamino)propyl]-N-(4-methoxy 2-nitro-phenyl)naphthalene-2-sulfonamide | 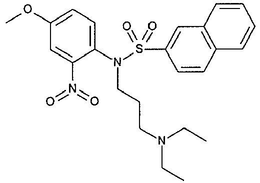 |

**[Table 2-6]**

| | | |
|---|---|---|
| 4-23 | N-(4-methoxy-2-phenyl-phenyl)-N-(3-piperidylmethyl)benzensulfonamide | 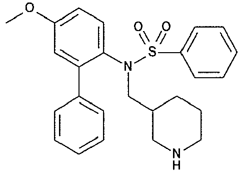 |
| 4-15 | N-[3-(diethylamino)propyl]-N-(2-isopropyl-phenyl)benzensulfonamide | 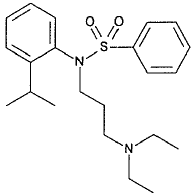 |
| 4-35 | N-(4-methoxy-2-phenyl-phenyl)-N-[2-(1-piperidyl)ethyl]benzensulfonamide | 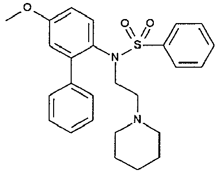 |
| 4-29 | t-butyl-N-[3-[N-(benzensulfonyl)-4-methoxy-2-phenyl-anilino]propyl]-N-methylcarbamate | 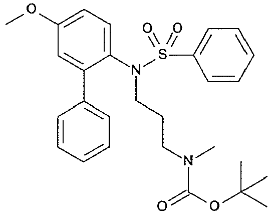 |
| 4-34 | N-(4-methoxy-2-phenyl-phenyl)-N-(2-morpholinoethyl)benzensulfonamide | 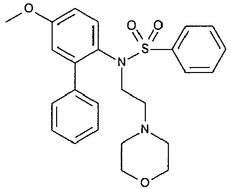 |
| 4-16 | N-[3-(diethylamino)propyl]-N-(2-t-butylphenyl)benzensulfonamide | 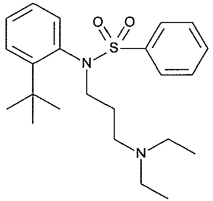 |

**[Table 2-7]**

| | | |
|---|---|---|
| 4-31 | t-butyl-N-[3-[N-(benzensulfonyl)-4-methoxy-2-phenyl-anilino]propyl]carbamate | 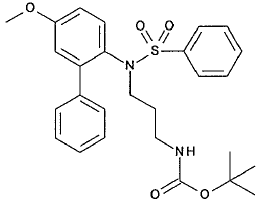 |
| 4-22 | t-butyl-3-[[N-(benzensulfonyl)-4-methoxy-2-phenyl-anilino]methyl]piperidine-1-carboxylate | 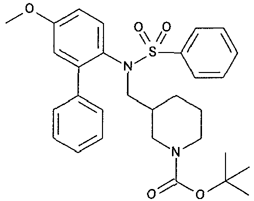 |
| 4-39 | N-(4-ethoxy-2-phenyl-phenyl)-N-[1-ethyl-4-piperidyl]methyl]benzensulfonamide | 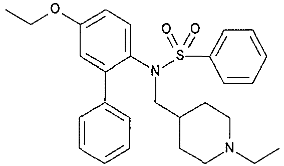 |
| 4-13 | N-[3-(diethylamino)propyl]-N-(2-phenyl-phenyl)naphthalene-1-sulfonamide | 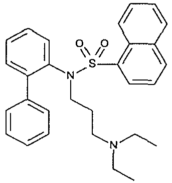 |
| 4-18 | t-butyl-4-[[N-(benzensulfonyl)-4-methoxy-2-phenyl-anilino]methyl]piperidine-1-carboxylate | 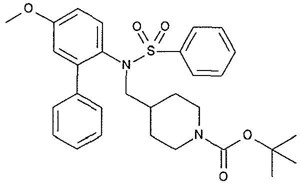 |
| 4-2 | N-[3-(diethylamino)propyl]-N-(2-nitrophenyl)benzensulfonamide | 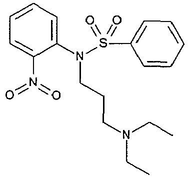 |

**[Table 2-8]**

| | | |
|---|---|---|
| 4-48 | N-(4-t-butyl-2-phenyl-phenyl)-N-( 1-ethyl-4-piperidylmethyl)benzensulfonamide | 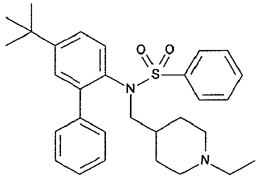 |
| 4-67 | N-[3-(dimethylamino)propyl]-N-[2-(4-fluorophenyl)-4-methoxyphenyl)benzensulfonamide | 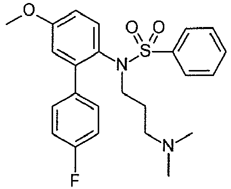 |
| 4-68 | N-[3-(diethylamino)propyl]-N-[2-(4-fluorophenyl)-4-methoxyphenyl)benzensulfonamide | 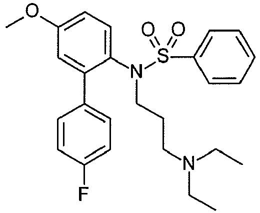 |

Particularly preferred compounds of the present invention include the group of the compounds described in the tables below:

**[Table 3-1]**

| No. | Name | Chemical structural formula |
|---|---|---|
| 4-21 | N-(4-methoxy-2-phenyl-phenyl)-N-[1-ethyl-4-piperidyl]methyl]benzensulfonamide | 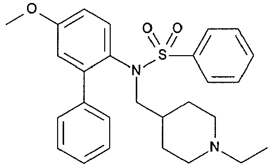 |
| 4-17 | N-[3-(diethylamino)propyl]-N-(4-methoxy-2-phenyl-phenyl)benzensulfonamide | 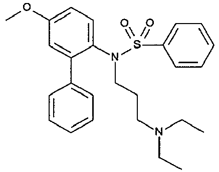 |
| 4-20 | N-(4-methoxy-2-phenyl-phenyl)-N-[1-methyl-4-piperidyl]methyl]benzensulfonamide | 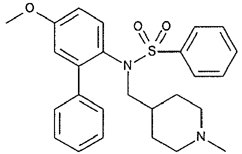 |
| 4-27 | N-[3-(dimethylamino)propyl]-N-(4-methoxy-2-phenyl-phenyl)benzensulfonamide | 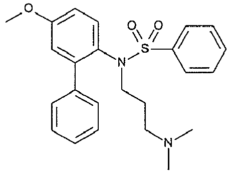 |
| 4-51 | N-[2-(4-fluorophenyl)-4-methoxyphenyl)-N-(1-ethyl-4-piperidylmethyl)benzensulfonamide | 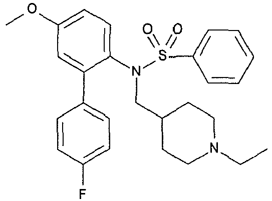 |
| 4-54 | N-[2-(3-fluorophenyl)-4-methoxyphenyl)-N-(1-ethyl-4-piperidylmethyl)benzensulfonamide | 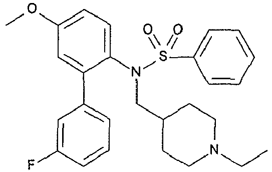 |

**[Table 3-2]**

| | | |
|---|---|---|
| 4-63 | 3-fluoro-N-(4-methoxy-2-phenyl-phenyl)-N-(1-ethyl-4-piperidylmethyl)benzensulfonamide | 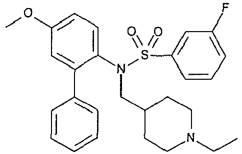 |
| 4-36 | N-[1-isopropyl-4-piperidyl]methyl]-N-(4-methoxy-2-phenyl-phenyl)benzensulfonamide | 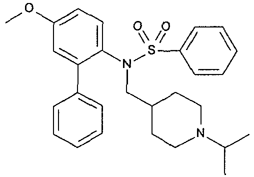 |
| 4-25 | N-(4-methoxy-2-phenyl-phenyl)-N-[1-ethyl-3-piperidyl]methyl]benzensulfonamide | 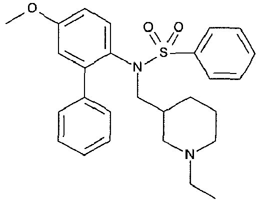 |
| 4-57 | N-[2-(2-fluorophenyl)-4-methoxyphenyl)-N-(1-ethyl-4-piperidylmethyl)benzensulfonamide | 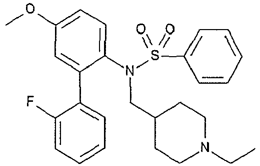 |
| 4-66 | 2-fluoro-N-(4-methoxy-2-phenyl-phenyl)-N-(1-ethyl-4-piperidylmethyl)benzensulfonamide | 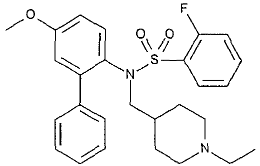 |
| 4-42 | N-(4-methoxy-2-phenyl-phenyl)-N-[1-methyl-3-piperidyl]methyl]benzensulfonamide | 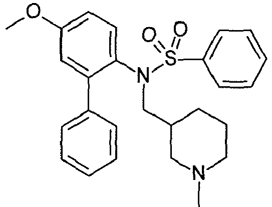 |

**[Table 3-3]**

| | | |
|---|---|---|
| 4-60 | 4-fluoro-N-(4-methoxy-2-phenyl-phenyl)-N-(1-ethyl-4-piperidylmethyl)benzensulfonamide | 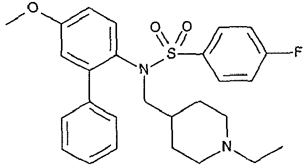 |
| 4-14 | 4-methoxy-N-[3-(diethylamino)propyl]-N-(2-phenyl-phenyl)benzensulfonamide | 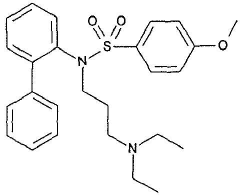 |
| 4-1 | N-[3-(diethylamino)propyl]-N-(4-methoxy-2-nitrophenyl)benzensulfonamide | 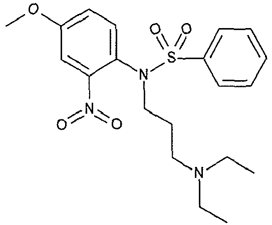 |
| 4-11 | 4-methoxy-N-[3-(diethylamino)propyl]-N-(4-methoxy-2-nitrophenyl)benzensulfonamide | 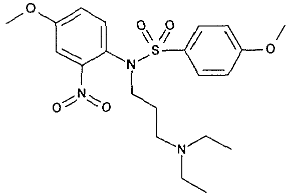 |
| 4-67 | N-[3-(dimethylamino)propyl]-N-[2-(4-fluorophenyl)-4-methoxyphenyl)benzensulfonamide | 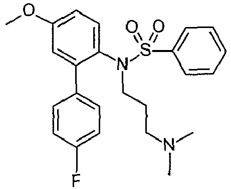 |

**[Table 3-4]**

| | | |
|---|---|---|
| 4-68 | N-[3-(diethylamino)propyl]-N-[2-(4-fluorophenyl)-4-methoxyphenyl)benzensulfonamide | 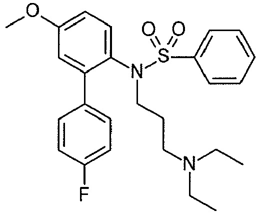 |

Further preferred compounds of the present invention include the group of compounds described in the tables below:

**[Table 4-1]**

| No. | Name | Chemical structural formula |
|---|---|---|
| 4-21 | N-(4-methoxy-2-phenyl-phenyl)-N-[1-ethyl-4-piperidyl]methyl]benzensulfonamide | 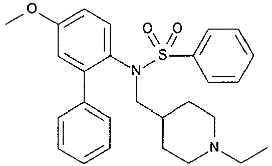 |
| 4-17 | N-[3-(diethylamino)propyl]-N-(4-methoxy-2-phenyl-phenyl)benzensulfonamide | 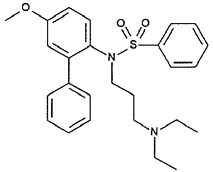 |
| 4-20 | N-(4-methoxy-2-phenyl-phenyl)-N-[1-methyl-4-piperidyl]methyl]benzensulfonamide | 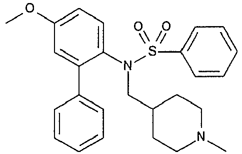 |
| 4-27 | N-[3-(dimethylamino)propyl]-N-(4-methoxy-2-phenyl-phenyl)benzensulfonamide | 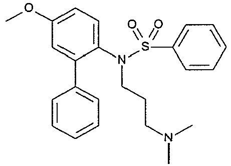 |
| 4-51 | N-[2-(4-fluorophenyl)-4-methoxyphenyl)-N-(1-ethyl-4-piperidylmethyl)benzensulfonamide | 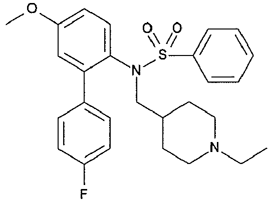 |
| 4-67 | N-[3-(dimethylamino)propyl]-N-[2-(4-fluorophenyl)-4-methoxyphenyl)benzensulfonamide | 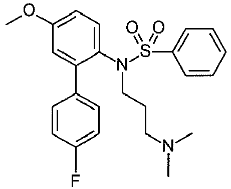 |

**[Table 4-2]**

| | | |
|---|---|---|
| 4-68 | N-[3-(diethylamino)propyl]-N-[2-(4-fluorophenyl)-4-methoxyphenyl)benzensulfonamide | 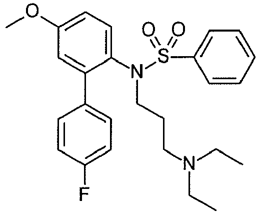 |

In certain embodiment, the compounds of the present invention preferably include the group of compounds described in the tables below:

**[Table 5-1]**

| No. | Name | Chemical structural formula |
|---|---|---|
| 4-21 | N-(4-methoxy-2-phenyl-phenyl)-N-[1-ethyl-4-piperidyl]methyl]benzensulfonamide | 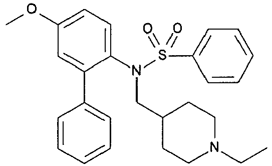 |
| 4-17 | N-[3-(diethylamino)propyl]-N-(4-methoxy-2-phenyl-phenyl)benzensulfonamide | 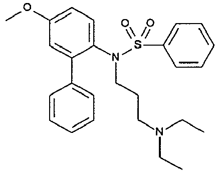 |
| 4-20 | N-(4-methoxy-2-phenyl-phenyl)-N-[1-methyl-4-piperidyl]methyl]benzensulfonamide | 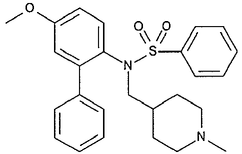 |
| 4-27 | N-[3-(dimethylamino)propyl]-N-(4-methoxy-2-phenyl-phenyl)benzensulfonamide | 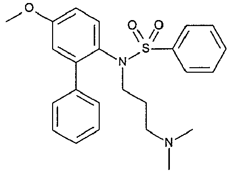 |
| 4-1 | N-[3-(diethylamino)propyl]-N-(4-methoxy-2-nitrophenyl)benzensulfonamide | 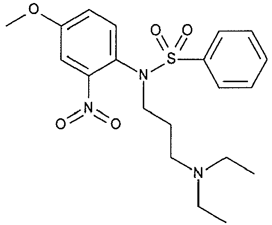 |
| 4-67 | N-[3-(dimethylamino)propyl]-N-[2-(4-fluorophenyl)-4-methoxyphenyl)benzensulfonamide | 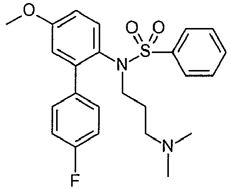 |

**[Table 5-2]**

| | | |
|---|---|---|
| 4-68 | N-[3-(diethylamino)propyl]-N-[2-(4-fluorophenyl)-4-methoxyphenyl)benzensulfonamide | 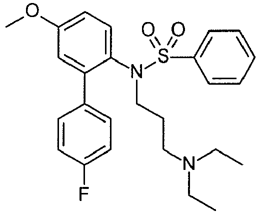 |

### <Representative method for production>

The compounds of the present invention can be produced according to, for example, the following methods, though methods for producing compounds of the present invention are not limited to these. Moreover, the order of reaction processes such as the introduction of substituents, etc., can be changed as necessary. Besides, raw materials to be used in production may be those which are commercially available or may be produced by conventional methods as necessary.

In the formulae representing the following reaction processes, R¹ to R⁴ are as defined in Formula (I).

Other abbreviations used in the following reaction formulae have usual meanings as a skilled person in the art can understand.

The names of reagents and solvents corresponding to the abbreviations and chemical equations which are generally used in the following general synthetic methods and Working Examples are described below.
- EtOAc: ethyl acetate
- Mel: methyl iodide
- Etl: ethyl iodide
- i-Prl: isopropyl iodide
- MeOH: methanol
- NaH: sodium hydride
- TEA: triethylamine
- DMAP: N,N-dimethyl-4-aminopyridine
- THF: tetrahydrofuran
- DMF: N,N-dimethylformamide
- DMSO: dimethylsulfoxide

The compounds of the present invention can be produced according to the following scheme.

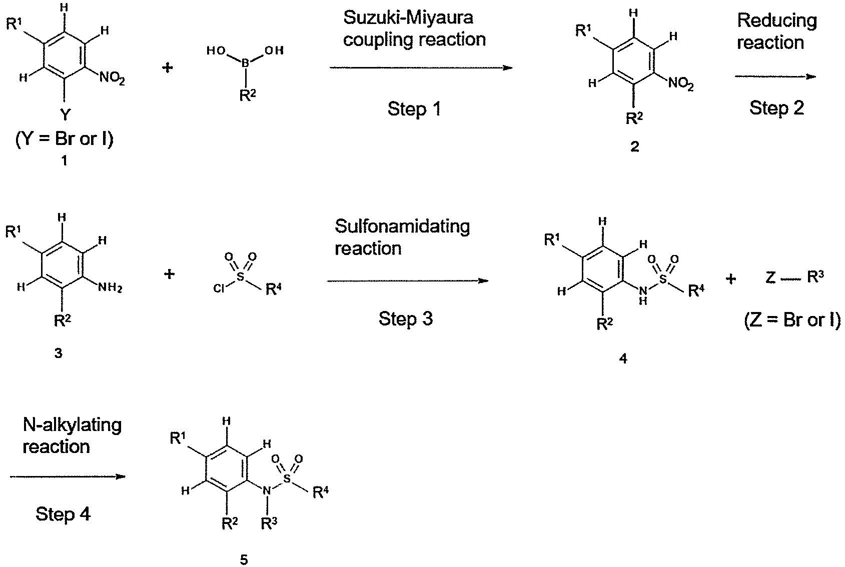

### [Step 1]

Step 1 is Suzuki-Miyaura coupling reaction.

This step is a chemical reaction in which cross-coupling of an arylboronic acid and a halogenated aryl via an action of a palladium catalyst or nickel catalyst and a nucleophile such as a base in the presence of an additive gives an asymmetrical biaryl (biphenyl derivative). Such a reaction is well known in the art, and a skilled person could carry out the reaction referring to the description of, e.g., Miyaura, N.; Suzuki, A. J. Chem. Soc., Chem. Commun. 1979, 866.

As a substrate, a halogenated aryl can be used.

As an organic boron compound, an organic boronic acid, boronic acid ester, trifluoroboronic acid, organic cyclic triolborate, and 1,8-diaminonaphthalene borate can be used. The organic boronic acid, e.g., aryl boronic acid, is preferred because it is relatively stable against water or air and easily handled.

As a palladium catalyst, palladium catalysts that are usually used in the art can be used, including, but not limited to, e.g., Pd (PPh₃)₄, Pd (OAc)₂, PdCl₂, PdCl₂ (PPh₃)₂, PdCl₂ (dppf)₂, Pd (NO₃)₂, PdCl₂CH₃CN)₂, PdCl₂ (PhCN)₂, Pd (acac)₂, Pd (dba)₂, Pd₂ (dba)₃, Pd[P (t-Bu)₂ (4-(Me₂N)-Ph)]₂Cl₂, Pd₂ (η-Allyl)₂Cl₂. Pd (OAc)₂ is preferred.

As a nickel catalyst, nickel catalysts that are usually used in the art can be used, including, but not limited to, e.g., Ni (COD)₂.

Bases include inorganic bases such as sodium hydroxide, potassium hydroxide, sodium carbonate, potassium carbonate, cesium carbonate, sodium hydride, potassium hydride and calcium hydride, or organic bases such as t-BuOK, t-BuONa, pyridine, TEA, DIPEA, LDA, LiHMDS and n-BuLi. Sodium carbonate is preferred.

As a solvent, toluene, tetrahydrofuran, N,N-dimethylformamide, water, methanol, ethanol, propanol, xylene, acetone, acetonitrile, benzene, chloroform, 2-butanone, hexane, heptane, pentane, cyclohexane, dichloromethane, dioxane, ethyl acetate, or a mixed solvent thereof can be used.

As an additive, copper iodide and tetrabutylammonium iodide can be used.

The reaction temperature is between room temperature to the boiling point of the solvent, and further adapted up to 300 °C by using a microwave synthesizer. It is preferably between 120 and 180 °C by using a microwave synthesizer, and the reaction time is 10 minutes.

After the reaction was finished, water was poured over the reaction solution, which is then extracted with ethyl acetate, and the organic layer is washed with water, saturated saline, etc., and dried over desiccant such as sodium sulfate. After removing desiccant, the solvent is removed by, e.g., concentration under reduced pressure to give a crude product.

Purifying the obtained crude product by, e.g., silica gel column chromatography and drying it under reduced pressure can give a biphenyl derivative, which is used in next steps.

### [Step 2]

Step 2 is a catalytic reduction of a nitro group to an amine.

In this step, the reaction of an aromatic compound having a nitro group (the biphenyl compound obtained in Step 1) with a reducing agent that can be a hydrogen source in the presence of a hydrogenating catalyst such as a nickel catalyst or palladium catalyst reduces the nitro group within the compound to an amino group.

As a hydrogenating catalyst to be used in the reaction, palladium catalysts such as, for example, 5 % palladium carbon and 10 % palladium carbon, as well as nickel catalysts can be used. Preference is given to 10 % palladium carbon.

Under a general nitro-group reducing condition, hydrogen gas is often used as a hydrogen source, though 1,4-cyclohexadiene can also be used as the hydrogen source for reaction in this step. Accordingly, hydrogen gas or 1,4-cyclohexadiene, preferably 1,4-cyclohexadiene can be used as the hydrogen source. The reaction can be carried out, for example, referring to the method described in Bioorganic and medicinal chemistry letters,12,22,3309-3312; 2002. It is used in 10 equivalences.

As a solvent, an alcohol solvent such as, e.g., methanol and ethanol can be used. Preferably the solvent is methanol.

The reaction temperature is between room temperature to the boiling point of the solvent, and further includes a reaction condition up to 300 °C by using a microwave synthesizer. It is preferably between 120 and 180 °C by using a microwave synthesizer, and the reaction time is 10 minutes.

For purification, after the reaction was finished, palladium carbon is removed by filtration and the reaction solution is distilled-off, and the obtained residue is purified by silica gel column chromatography, dried under reduced pressure, and the obtained aniline derivative is used in next steps.

### [Step 3]

Step 3 is a sulfonamidation process by a reaction between the aniline derivative obtained in Step 2 and sulfuryl chloride.

In this step, the reaction of the aniline derivative obtained in Step 2 with sulfuryl chloride in the presence of a pyridine solvent enables the synthesis of a sulfonamidated derivative.

The reaction temperature is between room temperature to the boiling point of the solvent, and further includes a reaction condition up to 300 °C by using a microwave synthesizer. It is preferably between 120 and 180 °C under microwave irradiation, and the reaction time is 10 minutes.

After the reaction is finished, water is poured over the reaction solution, which is extracted with an organic solvent such as ethyl acetate, the organic layer is washed with aqueous solution of 1N hydrochloric acid, saturated saline, dried over desiccant such as sodium sulfate. After removing the desiccant, the residue obtained by concentration under reduced pressure is purified by silica gel column chromatography, dried under reduced pressure, and the obtained sulfonamidated derivative is used in next steps.

### [Step 4]

This is an alkylation process of the sulfonamide to an amino group.

This step can be carried out by reacting the sulfonamidated derivative obtained in Step 3 with an alkylating agent in the presence of a base.

As an alkylating agent, R³I or R³Br (wherein, R³ is the same as that in the above-described Formula (I)) can be used.

The base that can be used is not particularly limited as long as it is a base known in the art, and includes, e.g., inorganic bases such as sodium hydroxide, potassium hydroxide, sodium carbonate, potassium carbonate, cesium carbonate, sodium hydride, potassium hydride and calcium hydride, or organic bases such as t-BuOK, t-BuONa, pyridine, TEA, DIPEA, LDA, LiHMDS and n-BuLi. Preferably the base is sodium carbonate or cesium carbonate.

The solvent that can be used is not particularly limited as long as it does not have any adverse effect on the reaction, and includes such as, e.g., for example, toluene, xylene, n-hexane, cyclohexane, DMF, DMA, EtOAc, DMSO, dichloromethane, carbon tetrachloride, THF, dioxane, acetonitrile, and mixtures thereof. Preferably, the solvent is DMF.

The reaction temperature is between room temperature to the boiling point of the solvent, and further includes a reaction condition up to 300 °C by using a microwave synthesizer. It is preferably between 120 and 180 °C under microwave irradiation, and the reaction time is 10 minutes.

After the reaction is finished, water is poured over the reaction solution, which is extracted with an organic solvent such as ethyl acetate, and the organic layer is washed with water, saturated saline, and dried over desiccant such as sodium sulfate. After removing the desiccant, the residue is concentrated under reduced pressure, purified by silica gel column chromatography, and dried under reduced pressure to give the intended compound of the present invention.

### <Synthesis of raw material compounds>

The raw material compounds for the compounds of the present invention can easily obtained by purchasing commercially available compounds or synthesizing them using known methods.

An example of a method for producing the compound of Formula (I) regarding to the present invention was shown above, and the isolation and purification of the compounds of interest indicated in the above-mentioned reaction steps can be carried out by applying ordinal chemical operations such as an extraction, concentration, distilling-off, crystallization, filtration, recrystallization, and various chromatography.

### <2> Pharmaceutical compositions of the present invention

The compounds of the present invention can inhibit invasion by a virus, filovirus in particular, into cells as mentioned above, and can therefore be suitably used in prophylaxis and/or treatment of a disease caused by viral infection. Accordingly, the present invention, in one aspect, relates to a pharmaceutical composition comprising a compound of the present invention or a solvate thereof or a pharmaceutically acceptable salt of the compound or a solvate thereof. Specifically, the compounds of the present invention have remarkably low IC₅₀ value in inhibiting the invasion by a virus, filovirus in particular. The inventors have found that some of the compounds have suitable characteristics as an effective ingredient for a pharmaceutical composition for their low cardio-toxicity and high metabolic stability in a living organism.

The pharmaceutical composition of the present invention may further comprise, adding to the compound of the present invention, a pharmaceutically acceptable carrier. The term a "pharmaceutically acceptable carrier" herein means one or more compatible solid or liquid excipients/diluents or encapsulating ingredients which is suitable for administration to a subject (e.g., a mammal) who can suffer from filovirus infection. The term "acceptable" herein means that an ingredient within the composition and the compound of interest can be mixed by a way such that neither of these will not cause a reaction that substantially decreases the pharmaceutical efficacy of the composition under normal conditions of use. A pharmaceutically acceptable carrier must naturally have a sufficiently high purity and sufficiently low toxicity such that it is suitable for administration to a a subject to be treated, preferably an animal, more preferably an mammal.

Examples of ingredients that can be used as pharmaceutically acceptable carriers include, such as, saccharides such as lactose, glucose and sucrose; starches such as corn starch and potato starch; cellulose and its derivatives such as sodium carboxymethyl cellulose, ethyl cellulose and methyl cellulose; tragacanth gum powder; malt; gelatin; talc; solid lubricants such as stearic acid and magnesium stearate; calcium sulfate; plant oils such as peanut oil, cottonseed oil, sesame oil, olive oil, corn oil, plant oil and cacao seed oil; polyalcohols such as propylene glycol, glycerin, sorbitol, mannitol and polyethylene glycol; alginic acid; emulsifiers such as TWEEN; wetting agents such as lecithin; coloring agents; flavoring agents; tableting agents; stabilizing agents; antioxidants; antiseptics; pyrogen-free water; isotonic saline; and phosphate buffered solution.

When a pharmaceutical composition of the present invention is used as a therapeutic or prophylaxis for viral infection, methods of administration include, such as, oral, rectal, parenteral (intravenous, intramuscular, subcutaneous), intracisternal, intravaginal, intraperitoneal, intravesical, topical (infusion, powder, ointment, gel or cream) administration and inhalation (buccal or nose spray).

Their dosage forms include, for example, tablets, capsules, granules, powder, pills, aqueous or non-aqueous oral solution and suspension, and a non-oral solution filled in a container adapted for subdividing into individual dosages. Moreover, dosage forms can be adapted to various methods of administration encompassing formulations for controlled release such as subcutaneous implant.

The preparations described above are produced by well-known methods using additives such as excipients, lubricants (coatings), binding agents, disintegrating agents, stabilizing agents, corrigents and dilutions.

Excipients include, but not limited to, such as, for example, starch such as starch, potato starch and corn starch, lactose, crystalline cellulose and calcium hydrogen-phosphate.

Coatings include, but not limited to, such as, for example, ethyl cellulose, hydroxypropyl cellulose, hydroxypropyl methyl cellulose, shellac, talc, carnauba wax and paraffin.

Binding agents include, but not limited to, such as, for example, polyvinyl pyrrolidone, macrogol and compounds similar to the above-described diluents.

Disintegrating agents include, but not limited to, such as, for example, compounds similar to the above-described diluents and chemically modified starch such as croscarmellose sodium, carboxymethyl starch sodium, cross-linked polyvinyl pyrrolidone and celluloses.

Stabilizing agents include, but not limited to, such as, for example, hydroxybenzoates such as methylparaben and propylparaben; alcohols such as chlorobutanol, benzyl alcohol, phenylethyl alcohol; benzalkonium chloride; phenols such as phenol and cresol; thimerosal; dehydroacetic acid; and sorbic acid.

Corrigents include, but not limited to, such as, for example, sweeteners, acidulants and flavorings, etc. which are usually used.

Moreover, as a solvent for producing a liquid agent, for example, ethanol, phenol, chlorocresol, purified water, distilled water can be used, though not limited thereto.

Surfactants and emulsifying agents include, but not limited to, such as, for example, polysorbate 80, polyoxyl stearate 40, lauromacrogol.

The mechanism by which the compound of the present invention inhibits the invasion by a virus, filovirus in particular, into cells is not clear. While not being bound to a theory, it can be considered, for example, that it acts on glycoproteins on the surface of the virus and thereby inhibits their function.

When a pharmaceutical composition of the present invention is used as a therapeutic or prophylaxis for viral infection, the amount of the compound of the present invention or a salt thereof or solvate used differs depending on symptoms, age, body weight, relative physical conditions, the presence of other drug administration, methods for administration, etc. For example, for a patient (a warm-blooded animal, human in particular), the amount that is generally effective is, as an effective ingredient (the compound of the present invention), in a case of oral formulation, preferably between 0.001 and 1000 mg per day per 1 kg of body weight, further preferably between 0.01 and 300 mg per day per 1 kg of body weight. The amount used per one day is, for an adult patient of a normal body weight, preferably in a range between preferably 1 and 800 mg. In a case of parenteral formulation, preferably between 0.001 and 1000 mg per day per 1 kg of body weight, further preferably between 0.01 and 300mg per day per 1 kg of body weight. It is desirable to administering this amount by dividing it into one or several doses, depending on symptoms.

The pharmaceutical composition of the present invention is for prophylaxis and/or treatment of a viral infection, preferably, for prophylaxis/treatment of an acute viral infection, more preferably, for prophylaxis and/or treatment of Ebola hemorrhagic fever and/or Marburg hemorrhagic fever, further preferably, for prophylaxis and/or treatment of Ebola hemorrhagic fever.

The pharmaceutical composition of the present invention is for prophylaxis and/or treatment of a viral infection, wherein the virus is preferably filovirus, and, more preferably, the virus is Ebola virus or Marburg virus, and further preferably the virus is Ebola virus.

### <3> Antiviral agents of the present invention

The compounds of the present invention can inhibit a virus, filovirus in particular, from invading into a cell, as mentioned above, which disables the virus for infecting the cell, resulting in the suppression of viral proliferation. Therefore, the present invention relates, in one aspect, to an antiviral agent comprising a compound of the present invention or a solvate thereof or a pharmaceutically acceptable salt of the compound or a solvate thereof. The antiviral agent means an agent which has an effect of suppressing viral proliferation *in vitro* and/or *in vivo.* Preferably, the virus is filovirus, and more preferably the virus is Ebola virus or Marburg virus, and further preferably the virus is Ebola virus.

### <4> Viral cell-invasion inhibitor of the present invention

The present invention, in one aspect, relates to a viral cell-invasion inhibitor comprising a compound of the present invention or a solvate thereof or a pharmaceutically acceptable salt of the compound or a solvate thereof. The viral cell-invasion inhibitor means an agent which has an effect of inhibiting the invasion by a virus into cells *in vitro* and/or *in vivo.* Preferably, the virus is filovirus, and more preferably the virus is Ebola virus or Marburg virus, and further preferably the virus is Ebola virus.

### <5> Method of the present invention of inhibiting viral cell-invasion processes in vitro

The present invention, in one aspect, relates to a method of inhibiting viral cell-invasion processes *in vitro,* comprising a step of bringing one or more compounds of the present invention into contact with the virus *in vitro.*

### <6> Method of the present invention for preventing and/or treating viral infection

The present invention, in one aspect, relates to a method for preventing and/or treating a viral infection in a subject, comprising a step of administrating an effective amount of one ore more compounds of the present invention to a subject in need thereof. The viral infection is preferably an acute viral infection, more preferably Ebola hemorrhagic fever and/or Marburg hemorrhagic fever, further preferably, Ebola hemorrhagic fever. When a pharmaceutical composition of the present invention is used as a therapeutic or prophylactic for viral infection, the amount of the compound of the present invention used can differ depending on symptoms, age, body weight, relative physical conditions, the presence of other drug administration, methods for administration, etc. For example, for a patient (a warm-blooded animal, human, in particular), the amount that is generally effective is, as an effective ingredient (the compound of the present invention), in a case of oral formulation, preferably 0.001 and 1000 mg per day per 1 kg of body weight, further preferably between 0.01 and 300 mg per 1 kg of body weight. The amount used per one day is, for example, when the subject is human, for an adult patient of a normal body weight, preferably in a range between 1 and 800 mg. In a case of parenteral formulation, it is preferably between 0.001 and 1000 mg per day per 1 kg of body weight, further preferably between 0.01 and 300 mg per 1 kg of body weight. It is desirable to administering this amount by dividing it into one or several doses, depending on symptoms.

### [Examples]

Hereinbelow, the present invention will be further explained by Working Examples, though the present invention are not to be limited to those Working Examples.

Microwave reaction was carried out using Initiator from Biotage Japan Ltd. using a snap-capped reaction vial. The setting for the maximum output includes air cooling of the reaction vessel to avoid temperature rise due to microwave.

For purification of synthesized compounds, Isolera Prime from Biotage Japan Ltd. was used, using SNAP cartridge from Biotage Japan Ltd. as purification column.

Mass spectrum data was obtained using Waters SQ Detector2.

NMR analysis was carried out using JNM-EC500 (500MHz), JNM-ECX400P (400MHz) or JNM-ECX400(400MHz) (all from JEOL), NMR data was indicated in ppm (parts per million) (δ), with reference to deuterium lock signal from sample solvent.

Commercially obtained reagents were used without further purification. Room temperature refers to a temperature range between about 20 and 30 °C. All non-aqueous reactions were carried out under nitrogen or argon atmosphere in an anhydrous solvent. For concentration under reduced pressure or distilling-off of solvents, rotary evaporators were used.

In preparation of a compound, when an unfavorable side reaction was likely to occur, functional groups were protected with protecting groups as necessary, which were removed after preparing the target molecule. The selection of the protecting groups and the attaching and detaching operations were performed by, for example, a method described in Greene and Wuts, "Protective Groups in Organic Synthesis" (5th ed., John Wiley & Sons 2014).

### [Working Example 1]

### Compound No.: 1-1

### 4-ethoxy-1-nitro-2-phenylbenzene

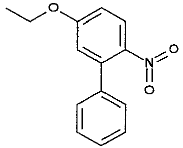

60 % sodium hydride (60 mg, 1.50 mmol) was dispersed in anhydrous tetrahydrofuran (1.5 mL), and to this solution added ethanol (88 µL, 1.50 mmol) and stirred at room temperature for 10 min to produce sodium etoxide.

4-fluoro-1-nitro-2-phenyl-benzene synthesized according to a known method (325 mg, 1.50 mmol) was dissolved in anhydrous DMF (4.0 mL), and this solution was added dropwise, stirred at room temperature for 30 min.

After confirming the disappearance of the raw materials with TLC, water (50 mL) was poured into the reaction solution, which was then extracted with ethyl acetate (30 mL), and the organic layer was washed with water and saturated saline, then dried over sodium sulfate. After removing the desiccant, the residue obtained by concentration under reduced pressure was purified by silica gel column chromatography (Biotage Ultra, 10 g, 0 -> 10 % ethyl acetate/hexane, elution with 15-column volume) to obtain the title compound, 4-ethoxy-1-nitro-2-phenylbenzene as yellow oil (161.6 mg, 44 % yield).
1H-NMR(500MHz,CDCl3) δ: 7.98 (d, J = 8.6 Hz, 1H), 7.46-7.41 (m, 3H), 7.32-7.31 (m, 2H), 7.15 (dd, J = 8.9 Hz, 2.6 Hz, 1H), 7.13 (d, J = 2.3 Hz, 1H)
LCMS: m/z244(M+H)⁺, 266(M+Na)⁺

### [Working Example 2]

### Compound No.: 1-2

### 4-isopropoxy-1-nitro-2-phenylbenzene

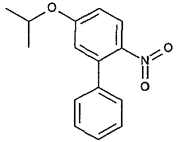

Under similar conditions to those in Working Example 1, using isopropanol instead of ethanol,
the title compound, 4-isopropoxy-1-nitro-2-phenylbenzene was obtained as pale yellow oil (46.6 mg, 20 %).
1H-NMR(500MHz,CDCl3) δ: 7.98 (d, J = 9.2 Hz, 1H), 7.44-7.38 (m, 3H), 7.31 (dd, J = 7.7 Hz, 1.4 Hz, 2H), 6.91 (dd, J = 8.9 Hz, 2.6 Hz, 1H), 6.84 (d, J = 2.9 Hz, 1H), 4.67 (m, 1H), 1.39 (d, J = 6.3 Hz, 6H)
LCMS: m/z258(M+H)⁺, 280(M+Na)⁺

### [Working Example 3]

### Compound No.: 1-3

### 4-t-butoxy-1-nitro-2-phenylbenzene

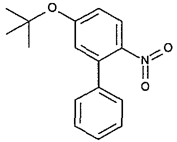

Under similar conditions to those in Working Example 1, using t-butanol instead of ethanol, the title compound, 4-t-butoxy-1-nitro-2-phenylbenzene was obtained as pale yellow oil (20.4 mg, 16 %).
1H-NMR(400MHz,CDCl3) δ: 7.90 (d, J = 9.0 Hz, 1H), 7.45-7.38 (m, 3H), 7.31-7.28 (m, 2H), 7.03 (dd, J = 9.0 Hz, 2.7 Hz, 1H), 6.98 (d, J = 2.7 Hz, 1H), 1.45 (s, 9H) LCMS: m/z294(M+Na)⁺, 272(M+H)⁺

### [Working Example 4]

### Compound No.: 1-4

### 2-(4-fluorophenyl)-4-methoxy-1-nitro-benzene

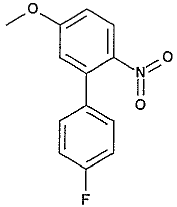

Commercially available 2-iod-4-methoxynitrobenzene (279.0 mg, 1.00 mmol), 4-fluorophenyl boronic acid (279.8 mg, 2.00 mmol), PdCl₂ (PPh₃)₂ (70.19 mg, 0.100 mmol), potassium carbonate (276.4 mg, 2.00 mmol), DMF (15mL), water (3mL) were added to 20 mL snap-capped reaction vial (Biotage Japan Ltd.), and, after an aluminum cap was fitted, this suspension was heated and stirred at 120°C for 10 min using microwave synthesizer.

After cooling to room temperature, the reaction suspension was pored into water (80 mL), which was then extracted with ethyl acetate (30 mL), and the organic layer was washed with water and saturated saline, then dried over sodium sulfate. After removing the desiccant, the residue obtained by concentration under reduced pressure was purified by silica gel column chromatography (Biotage KP-SIL, 25 g, 5 -> 10 % ethyl acetate/hexane, elution with 15-column volume) to give the title compound, 2-(4-fluorophenyl)-4-methoxy-1-nitro-benzene as pale yellow oil (226.2 mg, 91 % yield).
1H-NMR(400MHz,CDCl3) δ: 8.01 (d, J = 9.1 Hz, 1H), 7.28 (m, 2H), 7.12 (t, J = 8.6 Hz, 2H), 6.95 (dd, J = 9.1 Hz, 3.2 Hz, 1H), 6.83 (d, J = 2.7 Hz, 1H), 3.91 (S, 3H)
LCMS: m/z No detectable peaks.

### [Working Example 5]

### Compound No.: 1-5

### 2-(3-fluorophenyl)-4-methoxy-1-nitro-benzene

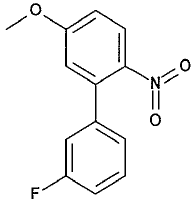

Under similar conditions to those in Working Example 4, using 3-fluorophenyl boronic acid instead of 4-fluorophenyl boronic acid, the title compound, 2-(3-fluorophenyl)-4-methoxy-1-nitro-benzene was obtained as pale yellow oil (227.7 mg, 93 %).
1 H-NMR(500MHz,CDCl3) δ: 8.02 (d, J = 8.6 Hz, 1H), 7.38 (m, 1H), 7.12-7.01 (m, 3H), 6.96 (dd, J = 8.9 Hz, 2.6 Hz, 1H), 6.83 (d, J = 2.9 Hz, 1H), 3.91 (s, 3H)
LCMS: m/z No detectable peaks.

### [Working Example 6]

### Compound No.: 1-6

### 2-(2-fluorophenyl)-4-methoxy-1-nitro-benzene

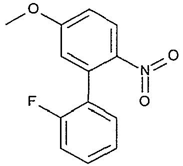

Under similar conditions to those in Working Example 4, using 2-fluorophenyl boronic acid instead of 4-fluorophenyl boronic acid, the title compound, 2-(2-fluorophenyl)-4-methoxy-1-nitro-benzene was obtained as pale yellow oil (223.8 mg, 94 %).
1H-NMR(400MHz,CDCl3) δ: 8.12 (d, J = 9.1 Hz, 1H), 7.40 (m, 1H), 7.32 (ddd, J = 7.5 Hz, 7.5 Hz, 1.8 Hz, 1H), 7.23 (dd, J = 7.5 Hz, 1.1 Hz, 1H), 7.12 (t, J = 9.3 Hz, 1H), 6.99 (dd, J = 9.1 Hz, 2.7 Hz, 1H), 6.85 (d, J = 2.7 Hz, 1H), 3.91 (s, 3H)
LCMS: m/z No detectable peaks.

### [Working Example 7]

### Compound No.: 2-1

### 4-ethoxy-2-phenyl-aniline

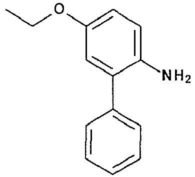

Compound No. 1-1 (30 mg, 0.12 mmol), 1,3-cyclohexadiene (0.112ml,1.20 mmol), 10 % palladium carbon (6mg), methanol (2 mL) were added to 20 mL snap-capped reaction vial (Biotage Japan Ltd.), and after an aluminum cap was fitted, heated and stirred at 130°C for 10 min using microwave synthesizer.

After cooling to room temperature, the reaction suspension was filtrated to remove 10 % palladium carbon. The solvent was distilled-off under reduced pressure, the residue was purified by silica gel column chromatography (Biotage ULTRA, 10 g, 5 -> 20 % ethyl acetate/hexane, elution with 15-column volume) to give the title compound, 4-ethoxy-2-phenyl-aniline as pale yellow oil (11.5 mg, 65 % yield).

1H-NMR(500MHz,CDCl3) δ: 7.48-7.43 (m, 4H), 7.35 (m, 1H), 6.77 (dd, J = 8.6 Hz, 2.9 Hz, 1H), 6.75 (d, J = 2.3 Hz, 1H), 6.72 (d, J = 8.6 Hz, 1H), 3.99 (q, J = 6.9 Hz, 2H), 3.49 (brs, 2H), 1.39 (t, J = 6.9 Hz, 3H)
LCMS: m/z 214(M+H)⁺

### [Working Example 8]

### Compound No.: 2-2

### 4-isopropoxy-2-phenyl-aniline

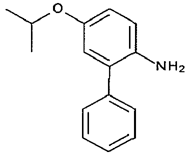

Under similar conditions to those in Working Example 7, using Compound No. 1-2 instead of Compound No. 1-1, an attempt was made to obtain the title compound, 4-isopropoxy-2-phenyl-aniline. However, degradation preferentially took place and the title compound could not be obtained.

### [Working Example 9]

### Compound No.: 2-3

### 4-t-butoxy-2-phenyl-aniline

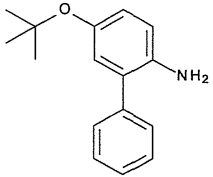

Under similar conditions to those in Working Example 7, using Compound No. 1-3 instead of Compound No. 1-1, the title compound, 4-t-butoxy-2-phenyl-aniline was obtained as pale yellow oil (3.8 mg, 21 %).

1H-NMR(500MHz,CDCl3) δ: 7.46-7.42 (m, 4H), 7.35 (m, 1H), 6.83-6.80 (m, 2H), 6.67 (m, 1H), 3.59 (brs, 2H), 1.31 (s, 9H)
LCMS: m/z 242(M+H)⁺

### [Working Example 10]

### Compound No.: 2-4

### 2-(2-fluorophenyl)-4-methoxy-aniline

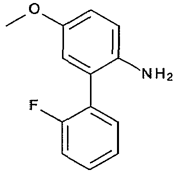

Under similar conditions to those in Working Example 7, using Compound No. 1-6 instead of Compound No. 1-1, the title compound, 2-(2-fluorophenyl)-4-methoxy-aniline was obtained as pale yellow oil (135.0 mg, 65 %).
1H-NMR(400MHz,CDCl3) δ: 7.39-7.33 (m, 2H), 7.25-7.15 (m, 2H), 6.82 (dd, J = 8.8 Hz, 2.9 Hz, 1H), 6.76 (d, J = 8.6 Hz, 1H), 6.72 (d, J = 2.7 Hz, 1H), 3.77 (s, 3H), 3.42 (br s, 2H)
LCMS: m/z 218(M+H)⁺

### [Working Example 11]

### Compound No.: 3-1

### 3-fluoro-N-(4-methoxy-2-nitro-phenyl)benzensulfonamide

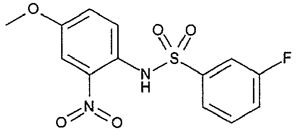

Commercially obtained 4-methoxy-2-nitroaniline (200.0 mg,1.18 mmol), 3-fluorobenzenesulfonylchloride (0.190 mL, 1.42 mmol), DMAP (15.0 mg,0.12 mmol), and 4 ml of pyridine were added to 20 mL snap-capped reaction vial (Biotage Japan Ltd.), and after an aluminum cap was fitted, heated and stirred at 160 °C for 20 min using microwave synthesizer.

The reaction suspension was poured into 1N hydrochloric acid (100 mL), which was then extracted with ethyl acetate (30 mL), and the organic layer was washed with water and saturated saline, then dried over sodium sulfate. After removing the desiccant, the residue obtained by concentration under reduced pressure was purified by silica gel column chromatography (Biotage KP-SIL, 25 g, 10 -> 20 % ethyl acetate/hexane, elution with 15-column volume) to give the title compound, 3-fluoro-N-(4-methoxy-2-nitro-phenyl)benzensulfonamide as pale yellow oil (102.5 mg, 26 % yield).
1H-NMR(500MHz,CDCl3) δ: 9.23 (br s, 1H), 7.76 (d, J = 9.2 Hz, 1H), 7.51 (ddd, J = 8.0 Hz, 1.4 Hz, 1.4 Hz, 1H), 7.49 (d, J = 2.9 Hz, 1H), 7.45-7.40 (m, 2H), 7.25 (ddd, J = 8.5 Hz, 8.5 Hz, 2.3 Hz, 1H), 7.20 (dd, J = 9.2 Hz, 2.9 Hz, 1H), 3.82 (s, 3H)
LCMS: m/z 349(M+Na)⁺

### [Working Example 12]

### Compound No.: 3-2

### 2-fluoro-N-(4-methoxy-2-nitro-phenyl)benzensulfonamide

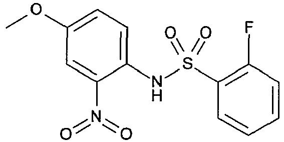

Under similar conditions to those in Working Example 11, using 2-fluorobenzenesulfonylchloride instead of 3-fluorobenzenesulfonylchloride, the title compound, 2-fluoro-N-(4-methoxy-2-nitro-phenyl)benzensulfonamide was obtained as pale yellow oil (180.0 mg, 46 %).
1H-NMR(500MHz,CDCl3) δ: 7.83 (ddd, J = 7.5 Hz, 7.4 Hz, 1.5 Hz, 1H), 7.70 (d, J = 9.2 Hz, 1H), 7.55 (m, 1H), 7.51 (d, J = 2.9 Hz, 1H), 7.22 (dd, J = 7.5Hz, 7.4 Hz, 1H), 7.12 (dd, J = 9.5 Hz, 9.5 Hz, 1H), 7.11 (d, J = 9.2 Hz, 1H), 5.98 (brs, 1H), 3.78 (s, 3H)
LCMS: m/z 349(M+Na)⁺

### [Working Example 13]

### Compound No.: 3-3

### 3-methyl-N-(4-methoxy-2-nitro-phenyl)benzensulfonamide

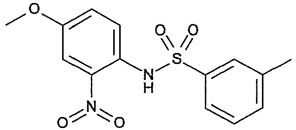

Under similar conditions to those in Working Example 11, using 3-methylbenzenesulfonylchloride instead of 3-fluorobenzenesulfonylchloride, the title compound, 3-methyl-N-(4-methoxy-2-nitro-phenyl)benzensulfonamide was obtained as yellow oil (320.5 mg, 83 %).
1H-NMR(500MHz,CDCl3) δ: 9.26 (brs, 1H), 7.78 (d, J = 9.2 Hz, 1H), 7.55 (brs, 1H), 7.51 (d, J = 8.0 Hz, 1H), 7.49 (d, J = 2.9 Hz, 1H), 7.35 (d, J = 7.5 Hz, 1H), 7.30 (dd, J = 7.7 Hz, 7.7 Hz, 1H), 7.18 (dd, J = 9.2 Hz, 3.4 Hz, 1H), 3.82 (s, 3H), 2.35 (s, 3H)
LCMS: m/z 345(M+Na)⁺

### [Working Example 14]

### Compound No.: 3-4

### 2-methyl-N-(4-methoxy-2-nitro-phenyl)benzensulfonamide

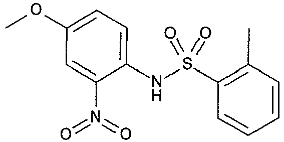

Under similar conditions to those in Working Example 11, using 2-methylbenzenesulfonylchloride instead of 3-fluorobenzenesulfonylchloride, the title compound, 2-methyl-N-(4-methoxy-2-nitro-phenyl)benzensulfonamide was obtained as yellow oil (379.4 mg, 78 %).
1H-NMR(500MHz,CDCl3) δ: 9.62 (br s, 1H), 7.93 (dd, J = 8.0 Hz, 1.2 Hz, 1H), 7.65 (d, J = 9.2 Hz, 1H), 7.53 (d, J = 2.9 Hz, 1H), 7.45 (ddd, J = 7.6 Hz, 7.6 Hz, 1.3 Hz, 1H), 7.28 (dd, J = 7.2 Hz, 3.7 Hz, 2H), 7.12 (dd, J = 9.2 Hz, 2.9 Hz, 1H), 3.79 (s, 3H), 2.62 (s, 3H) LCMS: m/z 345(M+Na)⁺

### [Working Example 15]

### Compound No.: 3-5

### N-(4-methoxy-2-nitro-phenyl)naphthalene-1-sulfonamide

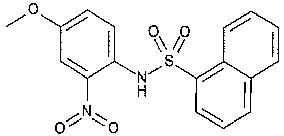

Under similar conditions to those in Working Example 11, using 1-naphthylsulfonylchloride instead of 3-fluorobenzenesulfonylchloride, the title compound, N-(4-methoxy-2-nitro-phenyl)naphthalene-1-sulfonamide was obtained as orange powder (295.0 mg, 69 %).
1H-NMR(500MHz,CDCl3) δ: 9.80 (brs, 1H), 8.59 (d, J = 8.6 Hz, 1H), 8.24 (dd, J = 7.5 Hz, 1.2 Hz, 1H), 8.03 (d, J = 8.0 Hz, 1H), 7.88 (d, J = 8.0 Hz, 1H), 7.71 (d, J = 9.2 Hz, 1H), 7.67 (ddd, 7.9 Hz, 7.9 Hz, 1.6 Hz, 1H), 7.58 (dd, J = 7.7 Hz, 7.7 Hz, 1H), 7.47 (dd, J = 7.7 Hz, 7.7 Hz, 1H), 7.36 (d, J = 3.4 Hz, 1H), 7.07 (dd, J = 9.5 Hz, 3.2 Hz, 1H), 3.72 (s, 3H)
LCMS: m/z 381(M+Na)⁺

### [Working Example 16]

### Compound No.: 3-6

### 3-methoxy-N-(4-methoxy-2-nitro-phenyl)benzensulfonamide

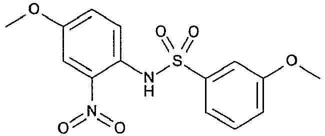

Under similar conditions to those in Working Example 11, using 3-methoxybenzenesulfonylchloride instead of 3-fluorobenzenesulfonylchloride, the title compound, 3-methoxy-N-(4-methoxy-2-nitro-phenyl)benzensulfonamide was obtained as pale yellow powder (362.2 mg, 90 %).
1H-NMR(400MHz,CDCl3) δ: 9.23 (br s, 1H), 7.76 (d, J = 9.1 Hz, 1H), 7.46 (d, J = 2.7 Hz, 1H), 7.30 (dd, J = 7.7 Hz, 7.7 Hz, 1H), 7.25 (ddd, J = 7.7 Hz, 1.4 Hz, 1.4 Hz, 1H), 7.19 (dd, J = 1.9 Hz, 2.0 Hz, 1H), 7.17 (dd, J = 9.0 Hz, 3.2 Hz, 1H), 7.04 (ddd, J = 8.2 Hz, 2.7 Hz, 1.4 Hz, 1H), 3.80 (s, 3H), 3.76 (s, 3H)
LCMS: m/z 361 (M+Na)⁺

### [Working Example 17]

### Compound No.: 3-7

### N-(4-methoxy-2-phenyl-phenyl)benzensulfonamide

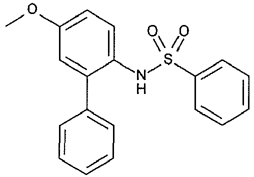

Under similar conditions to those in Working Example 11, using 4-ethoxy-2-phenylaniline synthesized by a known method and benzenesulfonylchloride, the title compound, N-(4-methoxy-2-phenyl-phenyl)benzensulfonamide was obtained as colorless oil (315.0 mg, 92 %).
1H-NMR(500MHz,CDCl3) δ: 7.64 (d, J = 8.6 Hz, 1H), 7.52 (dddd, J = 7.5 Hz, 7.5 Hz, 1.2 Hz, 1.2 Hz, 1H), 7.43 (dd, J = 8.6 Hz, 1.2 Hz, 2H), 7.34 (dd, J = 8.0 Hz, 8.0 Hz, 2H), 7.30 (ddd, J = 7.2 Hz, 1.9 Hz, 1.9 Hz, 1H), 7.25 (dd, J = 7.2 Hz, 7.2 Hz, 2H), 6.90 (dd, J = 8.9 Hz, 3.2 Hz, 1H), 6.69 (d, J = 6.9 Hz, 2H), 6.61 (d, J = 2.9 Hz, 1H), 6.43 (br s, 1H), 3.77 (s, 3H)
LCMS: m/z 362(M+Na)⁺

### [Working Example 18]

### Compound No.: 3-8

### N-(4-ethoxy-2-phenyl-phenyl)benzensulfonamide

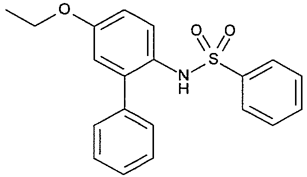

Under similar conditions to those in Working Example 11, using Compound No. 2-1 and benzenesulfonylchloride, the title compound, N-(4-ethoxy-2-phenyl-phenyl)benzensulfonamide was obtained as colorless oil (227.7 mg, 88 %).
1H-NMR(500MHz,CDCl3) δ: 7.64 (d, J = 9.2 Hz, 1H), 7.52 (dd, J = 7.5 Hz, 7.5 Hz, 1H), 7.43 (dd, J = 9.2 Hz, 1.2 Hz, 2H), 7.36-7.29 (m, 3H), 7.27-7.24 (m, 2H), 6.90 (dd, J = 9.2 Hz, 2.9 Hz, 1H), 6.68 (d, J = 6.9 Hz, 2H), 6.61 (d, J = 2.9 Hz, 1H), 6.41 (br s, 1H), 3.99 (q, J = 6.9 Hz, 2H), 1.39 (t, J = 6.9 Hz, 3H)
LCMS: m/z 729(2M+Na)⁺, 376(M+Na)⁺

### [Working Example 19]

### Compound No.: 3-9

### N-(4-ethyl-2-phenyl-phenyl) benzensulfonamide

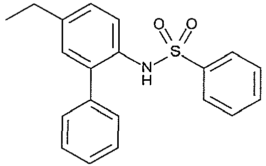

Under similar conditions to those in Working Example 11, using 4-ethyl-2-phenylaniline synthesized according to a known method, the title compound, N-(4-ethyl-2-phenyl-phenyl)benzensulfonamide was obtained as colorless oil (209.4 mg, 94 %).
1H-NMR(50OMHz,CDCl3) δ: 7.65 (d, J = 8.6 Hz, 1H), 7.55-7.52 (m, 3H), 7.38 (ddd, J = 6.9 Hz, 6.8 Hz, 2.1 Hz, 2H), 7.35-7.28 (m, 3H), 7.18 (dd, J = 8.3 Hz, 2.0 Hz, 1H), 6.93 (d, J = 1.7 Hz, 1H), 6.79 (ddd, J = 6.3 Hz, 1.9 Hz, 1.9 Hz, 2H), 6.55 (br s, 1H), 2.61 (q, J = 7.5 Hz, 2H), 1.21 (t, J = 7.4 Hz, 3H)
LCMS: m/z 697(2M+Na)⁺, 360(M+Na)⁺

### [Working Example 20]

### Compound No.: 3-10

### N-(4-isopropyl-2-phenyl-phenyl)benzensulfonamide

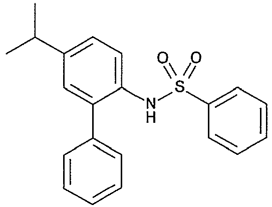

Under similar conditions to those in Working Example 11, using 4-isopropyl-2-phenylaniline synthesized according to a known method, the title compound, N-(4-isopropyl-2-phenyl-phenyl)benzensulfonamide was obtained as colorless oil (85.0 mg, 94 %).
1H-NMR(400MHz,CDCl3) δ: 7.63 (d, J = 8.2 Hz, 1H), 7.55 (dddd, J = 8.8 Hz, 8.8 Hz, 1.4 Hz, 1.4 Hz, 3H), 7.39 (ddd, J = 7.5 Hz, 7.5 Hz, 2.0 Hz, 2H), 7.33-7.29 (m, 3H), 7.21 (dd, J = 8.2 Hz, 2.3 Hz, 1H), 6.95 (d, J = 2.3 Hz, 1H), 6.82 (ddd, J = 5.9 Hz, 1.7 Hz, 1.7 Hz, 2H), 6.54 (br s, 1H), 2.87 (m, 1H), 1.22 (d, J = 6.8 Hz, 6H)
LCMS: m/z 374(M+Na)⁺, 352(M+H)⁺, 725(2M+Na)⁺

### [Working Example 21]

### Compound No.: 3-11

### N-(4-t-butyl-2-phenyl-phenyl)benzensulfonamide

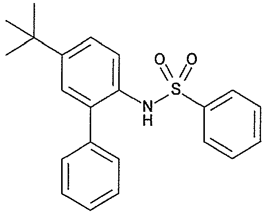

Under similar conditions to those in Working Example 11, using 4-t-butyl-2-phenylaniline synthesized according to a known method, the title compound, N-(4-t-butyl-2-phenyl-phenyl)benzensulfonamide was obtained as colorless oil (175.0 mg, 90 %).
1H-NMR(500MHz,CDCl3) δ: 7.64 (d, J = 8.6 Hz, 1H), 7.59 (dd, J = 8.6 Hz, 1.2 Hz, 2H), 7.55 (dd, J = 7.5 Hz, 7.5 Hz, 1H), 7.41-7.37 (m, 3H), 7.36-7.31 (m, 3H), 7.12 (d, J = 2.3 Hz, 1H), 6.86 (dd, J = 7.5 Hz, 1.7 Hz, 2H), 6.60 (br s, 1H), 1.30 (s, 9H)
LCMS: m/z 753(2M+Na)⁺, 388(M+Na)⁺

### [Working Example 22]

### Compound No.: 3-12

### N-[2-(4-fluorophenyl)-4-methoxy-phenyl)benzensulfonamide

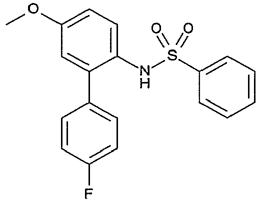

Under similar conditions to those in Working Example 11, using 2-(4-fluorophenyl)-4-methoxy-aniline synthesized according to a known method, the title compound, N-[2-(4-fluorophenyl)-4-methoxy-phenyl)benzensulfonamide was obtained as pale yellow oil (115.7 mg, 89 %).
1H-NMR(500MHz,CDCl3) δ: 7.62 (d, J = 8.6 Hz, 1H), 7.54 (br t, J = 7.5 Hz, 1H), 7.47 (dd, J = 8.3 Hz, 1.4 Hz, 2H), 7.38 (br t, J = 6.9 Hz, 2H), 6.97-6.90 (m, 3H), 6.67 (dd, J = 8.6 Hz, 5.2 Hz, 2H), 6.59 (d, J = 2.9 Hz, 1H), 6.26 (s, 1H), 3.78 (s, 3H)
LCMS: m/z No peaks.

### [Working Example 23]

### Compound No.: 3-13

### N-[2-(3-fluorophenyl)-4-methoxy-phenyl)benzensulfonamide

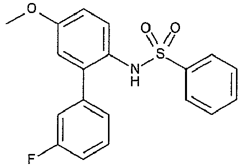

Under similar conditions to those in Working Example 11, using 2-(3-fluorophenyl)-4-methoxy-aniline synthesized according to a known method, the title compound, N-[2-(3-fluorophenyl)-4-methoxy-phenyl)benzensulfonamide was obtained as colorless oil (133.5 mg, 91 %).
1H-NMR(500MHz,CDCl3) δ: 7.66 (d, J = 9.2 Hz, 1H), 7.55 (br t, J = 7.5 Hz, 1H), 7.43 (dd, J = 8.6 Hz, 1.6 Hz, 2H), 7.36 (dd, J = 7.7 Hz, 7.7 Hz, 2H), 7.26 (ddd, J = 7.9 Hz, 7.9 Hz, 5.7 Hz, 1H), 7.01 (ddd, J = 8.3 Hz, 8.3 HZ, 2.3 Hz, 1H), 6.93 (dd, J = 9.2 Hz, 2.9 Hz, 1H), 6.59 (dd, J = 6.3 Hz, 2.9 Hz, 2H), 6.32 (brs, 1H), 6.21 (ddd, J = 9.5 Hz, 2.1 Hz, 2.1 Hz, 1H), 3.79 (s, 3H)
LCMS: m/z No peaks.

### [Working Example 24]

### Compound No.: 3-14

### N-[2-(2-fluorophenyl)-4-methoxy-phenyl)benzensulfonamide

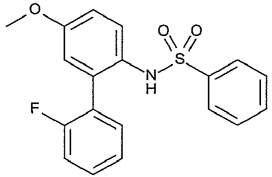

Under similar conditions to those in Working Example 11, using Compound No. 2-4, the title compound, N-[2-(2-fluorophenyl)-4-methoxy-phenyl)benzensulfonamide was obtained as colorless oil (78.6 mg, 87 %).
1H-NMR(400MHz,CDCl3) δ: 7.62 (d, J = 9.1 Hz, 1H), 7.44 (br t, J = 7.3 Hz, 1H), 7.33 (dd, J = 8.6 Hz, 1.4 Hz, 2H), 7.30 (m, 1H), 7.23 (dd, J = 7.9 Hz, 7.9 Hz, 2H), 7.05 (ddd, J = 9.2 Hz, 9.1 Hz, 1.2 Hz, 1H), 6.95 (dd, J = 9.1 Hz, 2.7 Hz, 1H), 6.94 (dd, J = 7.5 Hz, 1.1 Hz, 1H), 6.63 (d, J = 2.7 Hz, 1H), 6.50 (ddd, J = 7.6 Hz, 7.6 Hz, 1.7 Hz, 1H), 6.37 (d, J = 3.6 Hz, 1H), 3.79 (s, 3H)
LCMS: m/z 737(2M+Na)⁺, 380(M+Na)⁺

### [Working Example 25]

### Compound No.: 3-15

### 4-fluoro-N-(4-methoxy-2-phenyl-phenyl)benzensulfonamide

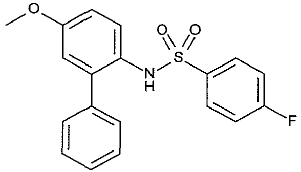

Under similar conditions to those in Working Example 11, using 4-methoxy-2-phenylaniline and 4-fluorobenzenesulfonylchloride synthesized according to known synthetic methods, the title compound, 4-fluoro-N-(4-methoxy-2-phenyl-phenyl)benzensulfonamide was obtained as colorless oil (237.4 mg, 85 %).
1H-NMR(500MHz,CDCl3) δ: 7.59 (d, J = 9.2 Hz, 1H), 7.37-7.35 (m, 2H), 7.31-7.23 (m, 3H), 6.95 (dd, J = 8.6 Hz, 8.6 Hz, 2H), 6.88 (dd, J = 9.2 Hz, 2.9 Hz, 1H), 6.72 (d, J = 7.5 Hz, 2H), 6.61 (d, J = 2.9 Hz, 1H), 6.46 (br s, 1H), 3.75 (s, 3H)
LCMS: m/z 380(M+Na)⁺

### [Working Example 26]

### Compound No.: 3-16

### 3-fluoro-N-(4-methoxy-2-phenyl-phenyl)benzensulfonamide

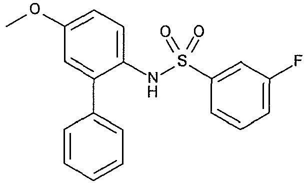

Under similar conditions to those in Working Example 11, using 4-methoxy-2-phenylaniline and 3-fluorobenzenesulfonylchloride synthesized according to known synthetic methods, the title compound, 3-fluoro-N-(4-methoxy-2-phenyl-phenyl)benzensulfonamide was obtained as colorless oil (232.3 mg, 83 %).
1H-NMR(400MHz,CDCl3) δ: 7.63 (d, J = 9.0 Hz, 1H), 7.36-7.27 (m, 4H), 7.23 (ddd, J = 8.2 Hz, 2.6 Hz, 1.0 Hz, 1H), 7.18 (dd, J = 7.6 Hz, 1.6 Hz, 1H), 7.07 (ddd, J = 7.6 Hz, 2.0 Hz, 2.0 Hz, 1H), 6.92 (dd, J = 9.0 Hz, 2.7 Hz, 1H), 6.73 (dd, J = 7.9 Hz, 1.6 Hz, 2H), 6.64 (d, J = 2.7 Hz, 1H), 6.51 (brs, 1H), 3.80 (s, 3H)
LCMS: m/z 380(M+Na)⁺

### [Working Example 27]

### Compound No.: 3-17

### 2-fluoro-N-(4-methoxy-2-phenyl-phenyl)benzensulfonamide

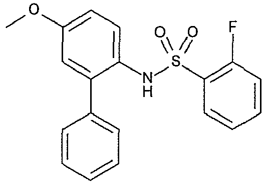

Under similar conditions to those in Working Example 11, using 4-methoxy-2 phenylaniline and 2-fluorobenzenesulfonylchloride synthesized according to known synthetic methods, the title compound, 2-fluoro-N-(4-methoxy-2-phenyl-phenyl)benzensulfonamide was obtained as colorless oil (219.6 mg, 78 %).
1H-NMR(400MHz,CDCl3) δ: 7.65 (ddd, J = 7.5 Hz, 7.5 Hz, 1.4 Hz, 1H), 7.53-7.47 (m, 2H), 7.42-7.34 (m, 3H), 7.14 (ddd, J = 7.9 Hz, 7.9 Hz, 0.9 Hz, 1H), 7.00 (dd, J = 7.6 Hz, 1.8 Hz, 2H), 6.95 (d, J = 8.5 Hz, 1H), 6.84 (dd, J = 9.0 Hz, 3.1 Hz, 2 H), 6.05 (d, J = 2.7 Hz, 1H), 3.76 (s, 3H) LCMS: m/z 380(M+Na)⁺, 737(2M+Na)⁺

### [Working Example 28]

### Compound No.: 4-1

### N-[3-(diethylamino)propyl]-N-(4-methoxy-2-nitro-phenyl)benzensulfonamide

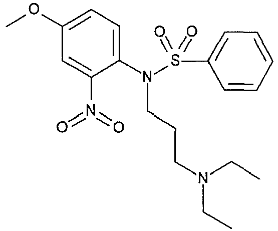

N-(4-methoxy-2-nitro-phenyl)benzensulfonamide (50.0mg,0.16 mmol) synthesized according to a known synthetic method, N,N-diethylaminopropylchloride (50.0mg,0.16 mmol) synthesized according to a known synthetic method, sodium carbonate (25.0mg,0.24 mmol), anhydrous DMF (2.5 mL) were added to a 5 mL snap-capped reaction vial (Biotage Japan Ltd.), and after an aluminum cap was fitted, heated and stirred at 120°C for 10 min using microwave synthesizer.

The reaction suspension was poured over water (100 mL), which was extracted with ethyl acetate (30 mL), and the organic layer was washed with water and saturated saline, then dried over sodium sulfate. After removing the desiccant, the residue obtained by concentration under reduced pressure was purified by silica gel column chromatography (Biotage KP-SIL, 10 g, 0 -> 10 % methanol/chloroform, elution with 15-column volume) to give the title compound, N-[3-(diethylamino)propyl]-N-(4-methoxy-2-nitro-phenyl)benzensulfonamide as pale yellow oil (63.5 mg, 92 % yield).
1H-NMR(500MHz,CDCl3) δ:7.55- 7.63 (m, 3H), 7.45-7.8 (m, 2H), 7.35 (brs, 1H),), 7.00 (d, J = 7.5 Hz, 1H), 6.93 (d, J = 7.5 Hz, 1H), 3.86 (s, 3H), 3.72 (brs, 1H), 3.53 (brs, 1H), 2.48 (m, 6H), 1.81 (brs, 1H), 1.73 (brs, 1H), 0.95 (t, J =7.0 Hz, 6H)
LCMS: m/z 422(M+H)⁺

### [Working Example 29]

### Compound No.: 4-2

### N-[3-(diethylamino)propyl]-N-(2-nitro-phenyl)benzensulfonamide

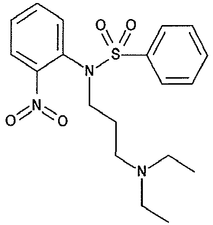

Under similar conditions to those in Working Example 28, using N-(2-nitrophenyl)benzensulfonamide synthesized according to a known synthetic method, the title compound, N-[3-(diethylamino)propyl]-N-(2-nitro-phenyl)benzensulfonamide was obtained as pale yellow oil (63.5 mg, 92 %).
1H-NMR(500MHz,CDCl3) δ: 7.87 (d, J = 7.5 Hz, 1H), 7.60 (m, 3H), 748 (m, 4H), 7.04 (d, J = 7.5 Hz, 1H), 3.72 (brs, 1H), 3.60 (brs, 1H), 2.50 (m, 6H), 1.78 (brs, 2H), 0.98 (t, J =8.0 Hz, 6H) LCMS: m/z 392(M+H)⁺

### [Working Example 30]

### Compound No.: 4-3

### 4-fluoro-N-[3-(diethylamino)propyl]-N-(4-methoxy-2-nitro-phenyl)benzensulfonamide

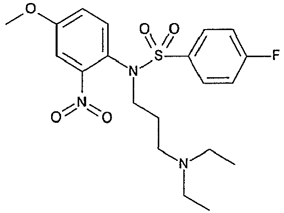

Under similar conditions to those in Working Example 28, using 4-fluoro-N-(4-methoxy-2-nitrophenyl)benzensulfonamide synthesized according to a known synthetic method, the title compound, 4-fluoro-N-[3-(diethylamino)propyl]-N-(4-methoxy-2-nitro-phenyl)benzensulfonamide was obtained as pale yellow oil (33.8 mg, 62 %).
1H-NMR(400MHz,CDCl3) δ: 7.63 (dd, J = 8.8 Hz, 5.2 Hz, 2H), 7.35 (d, J = 2.7 Hz, 1H), 7.14 (dd, J = 8.5 Hz, 8.5 Hz, 2H), 7.03 (dd, J = 9.0 Hz, 3.1 Hz, 1H), 6.97 (d, J = 9.0 Hz, 1H), 3.87 (s, 3H), 3.69 (br s, 1H), 3.56 (br s, 1H), 2.52 (dd, J = 13.7 Hz, 6.5 Hz, 6H), 1.80 (br s, 2H), 0.99 (t, J = 7.2 Hz, 6H)
LCMS: m/z 440(M+H)⁺

### [Working Example 31]

### Compound No.: 4-4

### 3-fluoro-N-[3-(diethylamino)propyl]-N-(4-methoxy-2-nitro-phenyl)benzensulfonamide

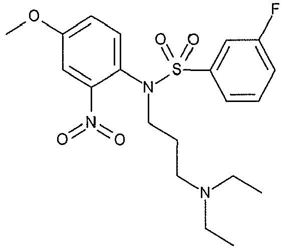

Under similar conditions to those in Working Example 28 using Compound No. 3-1, the title compound, 3-fluoro-N-[3-(diethylamino)propyl]-N-(4-methoxy-2-nitrophenyl)benzensulfonamide was obtained as pale yellow oil (74.6 mg, 92 %).
1H-NMR(500MHz,CDCl3) δ: 7.46 (ddd, J = 7.7 Hz, 7.7 Hz, 5.2 Hz, 1H), 7.41 (d, J = 8.0 Hz, 1H), 7.37 (d, J = 2.9 Hz, 1H), 7.32 (ddd, J = 8.0 Hz, 2.0 Hz, 2.0 Hz, 1H), 7.28 (m, 1H), 7.03 (dd, J = 8.9 Hz, 3.2 Hz, 1H), 6.98 (d, J = 9.2 Hz, 1H), 3.87 (s, 3H), 3.69 (br s, 1H), 3.60 (br s, 1H), 2.52 - 2.44 (m, 6H), 1.80-1.72 (m, 2H), 0.97 (t, J = 7.2 Hz, 6H)
LCMS: m/z 440(M+H)⁺, 901(2M+Na)⁺

### [Working Example 32]

### Compound No.: 4-5

### 2-fluoro-N-[3-(diethylamino)propyl]-N-(4-methoxy-2-nitro-phenyl)benzensulfonamide

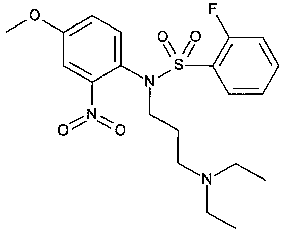

Under similar conditions to those in Working Example 28 using Compound No. 3-2, the title compound, 2-fluoro-N-[3-(diethylamino)propyl]-N-(4-methoxy-2-nitrophenyl)benzensulfonamide was obtained as pale yellow oil (48.5 mg, 84 %).
1H-NMR(500MHz,CDCl3) δ: 7.60 (ddd, J = 7.5 Hz, 7.4 Hz, 1.5 Hz, 1H), 7.55 (m, 1H), 7.35 (d, J = 2.9 Hz, 1H), 7.20-7.14 (m, 3H), 7.03 (dd, J = 8.6 Hz, 2.9 Hz, 1H), 3.85 (s, 3H), 3.78 (br s, 2H), 2.52 (dd, J = 14.3 Hz, 6.9 Hz,6H), 1.84-1.79 (m, 2H), 1.00 (t, J = 7.2 Hz, 6H)
LCMS: m/z 440(M+H)⁺

### [Working Example 33]

### Compound No.: 4-6

### 3-methyl-N-[3-(diethylamino)propyl]-N-(4-methoxy-2-nitro-phenyl)benzensulfonamide

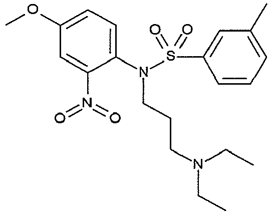

Under similar conditions to those in Working Example 28 using Compound No. 3-3, the title compound, 3-methyl-N-[3-(diethylamino)propyl]-N-(4-methoxy-2-nitrophenyl)benzensulfonamide was obtained as pale yellow oil (51.2 mg, 75 %).
1H-NMR(400MHz,CDCl3) δ: 7.41 (br s, 1H), 7.39-7.31 (m, 4H), 6.99 (dd, J = 8.8 Hz, 2.9 Hz, 1H), 6.91 (d, J = 8.6 Hz, 1H), 3.86 (s, 3H), 3.71 (br s, 1H), 3.50 (br s, 1H), 2.51 (q, J = 7.3 Hz, 6H), 2.36 (s, 3H), 1.81-1.73 (m, 2H), 0.99 (t, J = 7.0 Hz, 6H)
LCMS: m/z 436(M+H)⁺

### [Working Example 34]

### Compound No.: 4-7

### 2-methyl-N-[3-(diethylamino)propyl]-N-(4-methoxy-2-nitro-phenyl)benzensulfonamide

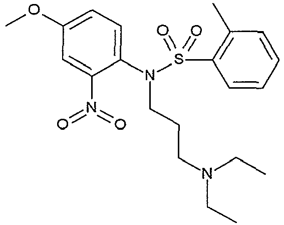

Under similar conditions to those in Working Example 28 using Compound No. 3-4, the title compound, 2-methyl-N-[3-(diethylamino)propyl]-N-(4-methoxy-2-nitrophenyl)benzensulfonamide was obtained as yellow oil (63.5 mg, 83 %).
1H-NMR(500MHz,CDCl3) δ: 7.60 (d, J = 8.0 Hz, 1H), 7.41 (dd, J = 7.6 Hz, 7.6 Hz, 1H), 7.25-7.22 (m, 3H), 7.18 (dd, J = 7.5 Hz, 7.5 Hz, 1H), 7.03 (dd, J = 9.2 Hz, 2.9 Hz, 1H), 3.84 (s, 3H), 3.75 (br s, 2H), 2.52-2.47 (m, 6H), 2.37 (s, 3H), 1.84-1.78 (m, 2H), 1.00-0.96 (m, 6H)
LCMS: m/z 436(M+H)⁺

### [Working Example 35]

### Compound No.: 4-8

### N-[3-(diethylamino)propyl]-N-(2-phenyl-phenyl)benzensulfonamide

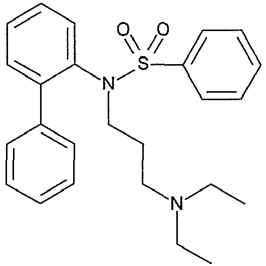

Under similar conditions to those in Working Example 28, using N-(2-phenyl-phenyl)benzensulfonamide synthesized according to a known synthetic method, the title compound, N-[3-(diethylamino)propyl]-N-(2-phenyl-phenyl)benzensulfonamide was obtained as colorless oil (65.2 mg, 95 %).
1H-NMR(500MHz,CDCl3) δ: 7.74 (d, J = 8.0 Hz, 2H), 7.59 (dd, J = 7.5 Hz, 7.5 Hz, 1H), 7.50-7.46 (m, 4H), 7.39-7.33 (m, 5H), 7.27 (m, 1H), 7.00 (d, J = 8.0 Hz, 1H), 3.23 (br s, 2H), 2.30 (q, J = 7.1 Hz, 4H), 2.07 (br s, 2H), 1.31 (br s, 2H), 0.85 (t, J = 7.2 Hz, 6H)
LCMS: m/z 423(M+H)⁺

### [Working Example 36]

### Compound No.: 4-9

### N-[3-(diethylamino)propyl]-N-(4-methoxy-2-nitro-phenyl)naphthalene-1-sulfonamide

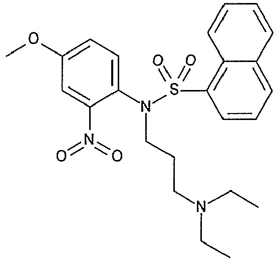

Under similar conditions to those in Working Example 28 using Compound No. 3-5, the title compound, N-[3-(diethylamino)propyl]-N-(4-methoxy-2-nitro-phenyl)naphthalene-1-sulfonamide was obtained as pale yellow oil (71.5 mg, 83 %).
1H-NMR(500MHz,CDCl3) δ: 8.37 (d, J = 8.6 Hz, 1H), 8.04 (dd, J = 8.9 Hz, 8.9 Hz, 2H), 7.90 (d, J = 7.5 Hz, 1H), 7.56 (ddd, J = 7.5 Hz, 7.4 Hz, 1.2 Hz, 1H), 7.49 (ddd, J = 7.7 Hz, 7.7 Hz, 1.2 Hz, 1H), 7.45 (dd, J = 7.7 Hz, 7.7 Hz, 1H), 7.27 (d, J = 2.9 Hz, 1H), 6.99 (d, J = 9.2 Hz, 1H), 6.90 (dd, J = 10.6 Hz, 3.2 Hz, 1H), 3.84 (s, 3H), 3.75 (t, J = 7.7 Hz, 2H), 2.52-2.44 (m, 6H), 1.82-1.75 (m, 2H), 0.96 (t, J = 7.2 Hz, 6H)
LCMS: m/z 472(M+H)⁺, 965(2M+Na)⁺

### [Working Example 37]

### Compound No.: 4-10

### N-[3-(diethylamino)propyl]-N-(4-methoxy-2-nitro-phenyl)naphthalene-2-sulfonamide

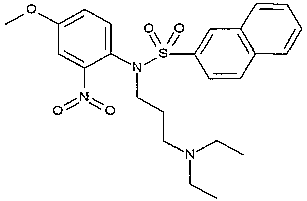

Under similar conditions to those in Working Example 28, using N-(4-methoxy-2-nitrophenyl)naphthalene-2-sulfonamide synthesized according to a known synthetic method, the title compound, N-[3-(diethylamino)propyl]-N-(4-methoxy-2-nitro-phenyl)naphthalene-2-sulfonamide was obtained as pale yellow oil (56.3 mg, 85 %).
1H-NMR(500MHz,CDCl3) δ: 8.20 (d, J = 1.2 Hz, 1H), 7.92-7.89 (m, 3H), 7.65 (dd, J = 7.5 Hz, 7.4 Hz, 1H), 7.61-7.58 (m, 2H), 7.37 (d, J = 2.9 Hz, 1H), 6.97 (dd, J = 9.2 Hz, 2.9 Hz, 1H), 6.91 (d, J = 9.2 Hz, 1H), 3.86 (s, 3H), 3.79 (br s, 1H), 3.56 (br s, 1H), 2.52-2.46 (m, 6H), 1.81 (br s, 1H), 1.75 (br s, 1H), 0.97 (t, J = 7.2 Hz, 6H)
LCMS: m/z 472(M+H)⁺

### [Working Example 38]

### Compound No.: 4-11

### 4-methoxy-N-[3-(diethylamino)propyl]-N-(4-methoxy-2-nitro-phenyl)benzensulfonamide

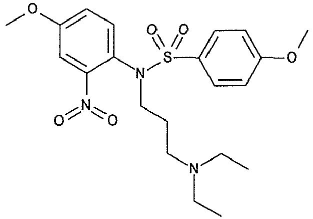

Under similar conditions to those in Working Example 28, using 4-methoxy-N-(4-methoxy-2nitro-phenyl)benzensulfonamide synthesized according to a known synthetic method, the title compound, 4-methoxy-N-[3-(diethylamino)propyl]-N-(4-methoxy-2-nitrophenyl)benzensulfonamide was obtained as pale yellow oil (11.6 mg, 17 %).
1H-NMR(500MHz,CDCl3) δ: 7.54 (ddd, J = 9.2 Hz, 2.6 Hz, 2.6 H, 2H), 7.34 (d, J = 2.9 Hz, 1H), 7.01 (dd, J = 9.2 Hz, 2.9 Hz, 1H), 6.95 (d, J = 9.2 Hz, 1H), 6.92 (ddd, J = 9.2 Hz, 2.6 Hz, 2.6 Hz, 2H), 3.86 (s, 6H), 3.70 (br s, 1H), 3.50 (br s, 1H), 2.55-2.51 (m, 6H), 1.78 (m, 2H), 1.00 (t, J 7.2 Hz, 6H)
LCMS: m/z 452(M+H)⁺, 925(2M+Na)⁺

### [Working Example 39]

### Compound No.: 4-12

### 3-methoxy-N-[3-(diethylamino)propyl]-N-(4-methoxy-2-nitro-phenyl)benzensulfonamide

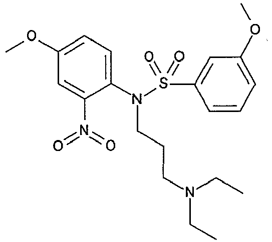

Under similar conditions to those in Working Example 28, using Compound No. 3-6, the title compound, 3-methoxy-N-[3-(diethylamino)propyl]-N-(4-methoxy-2-nitrophenyl)benzensulfonamide was obtained as pale yellow oil (23.5 mg, 35 %).
1H-NMR(400MHz,CDCl3) δ: 7.38-7.34 (m, 2H), 7.21 (ddd, J = 7.9 Hz, 1.4 Hz, 1.4 Hz, 1H), 7.12-7.09 (m, 2H), 7.02 (dd, J = 8.6 Hz, 2.7 Hz, 1H), 6.98 (d, J = 8.6 Hz, 1H), 3.87 (s, 3H), 3.79 (s, 3H), 3.70 (br s, 1H), 3.55 (br s, 1H), 2.48 (q, J = 7.1 Hz, 6H), 1.80-1.71 (m, 2H), 0.97 (t, J = 7.0 Hz, 6H)
LCMS: m/z 452(M+H)⁺

### [Working Example 40]

### Compound No.: 4-13

### N-[3-(diethylamino)propyl]-N-(2-phenyl-phenyl)naphthalene-1-sulfonamide

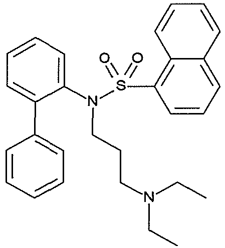

Under similar conditions to those in Working Example 28, using N-(2-phenyl-phenyl)naphthalene-1-sulfonamide synthesized according to a known synthetic method, the title compound, N-[3-(diethylamino)propyl]-N-(2-phenyl-phenyl)naphthalene-1-sulfonamide was obtained as colorless oil (62.0 mg, 94 %).
1H-NMR(500MHz,CDCl3) δ: 8.57 (d, J = 8.6 Hz, 1H), 8.09 (d, J = 7.5 Hz, 1H), 8.05 (d, J = 8.0 Hz, 1H), 7.90 (d, J = 8.0 Hz, 1H), 7.54 (dd, J = 7.5 Hz, 7.5 Hz, 1H), 7.49-7.46 (m, 2H), 7.34 (ddd, J = 7.5 Hz, 7.5 Hz, 1.2 Hz, 1H), 7.29 (dd, J = 7.5 Hz, 1.7 Hz, 1H), 7.26-7.19 (m, 6H), 7.09 (d, J = 8.0 Hz, 1H), 3.54 (br s, 1H), 3.22 (br s, 1H), 2.30 (q, J = 7.3 Hz, 4H), 2.12 (br s, 2H), 1.45-1.39 (m, 2H), 0.85 (t, J = 6.9 Hz, 6H)
LCMS: m/z 473(M+H)⁺

### [Working Example 41]

### Compound No.: 4-14

### 4-methoxy-N-[3-(diethylamino)propyl]-N-(2-phenyl-phenyl)benzensulfonamide

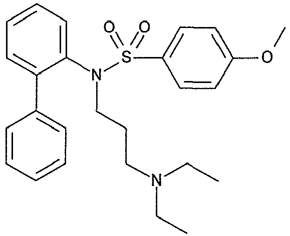

Under similar conditions to those in Working Example 28, using 4-methoxy-N-(2-phenyl-phenyl)benzensulfonamide synthesized according to a known synthetic method, the title compound, 4-methoxy-N-[3-(diethylamino)propyl]-N-(2-phenyl-phenyl)benzensulfonamide was obtained as colorless oil (48.7 mg, 73 %).
1H-NMR(500MHz,CDCl3) δ: 7.64 (ddd, J = 9.2 Hz, 2.6 Hz, 2.6 Hz, 2H), 7.49 (dd, J = 8.0 Hz, 1.2 Hz, 2H), 7.40-7.33 (m, 5H), 7.27 (m, 1H), 7.03 (d, J = 8.0 Hz, 1H), 6.93 (ddd, J = 9.3 Hz, 2.4 Hz, 2.4 Hz, 2H), 3.87 (s, 3H), 3.21 (t, J = 7.5 Hz, 2H), 2.29 (q, J = 6.9 Hz, 4H), 2.06 (br s, 2H), 1.37-1.26 (m, 2H), 0.85 (t, J = 7.2 Hz, 6H)
LCMS: m/z 453(M+H)⁺

### [Working Example 42]

### Compound No.: 4-15

### N-[3-(diethylamino)propyl]-N-(2-isopropyl-phenyl)benzensulfonamide

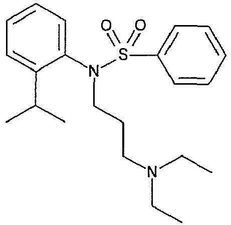

Under similar conditions to those in Working Example 28, using N-(2-isopropylphenyl)benzensulfonamide synthesized according to a known synthetic method, the title compound, N-[3-(diethylamino)propyl]-N-(2-isopropyl-phenyl)benzensulfonamide was obtained as colorless oil (52.9 mg, 75 %).
1H-NMR(400MHz,CDCl3) δ: 7.70 (dd, J = 7.6 Hz, 1.8 Hz, 2H), 7.58 (dddd, J = 7.4 Hz, 7.4 Hz, 1.5 Hz, 1.4 Hz, 1H), 7.48 (ddd, J = 6.8 Hz, 6.8 Hz, 1.6 Hz, 2H), 7.37 (dd, J = 7.9 Hz, 1.6 Hz, 1H), 7.28 (ddd, J = 7.6 Hz, 7.6 Hz, 1.2 Hz, 1H), 7.01 (ddd, J = 7.6 Hz, 7.6 Hz, 1.9 Hz, 1H), 6.55 (dd, J = 8.1 Hz, 1.4 Hz, 1H), 3.76 (m, 1H), 3.53 (m, 1H), 3.27 (m, 1H), 2.45-2.33 (m, 6H), 1.67 (m, 1H), 1.47 (m, 1H), 1.25 (d, J = 7.2 Hz, 3H), 1.18 (d, J = 6.7 Hz, 3H), 0.93 (t, J = 7.2 Hz, 6H)
LCMS: m/z 389(M+H)⁺

### [Working Example 43]

### Compound No.: 4-16

### N-[3-(diethylamino)propyl]-N-(2-t-butyl-phenyl)benzensulfonamide

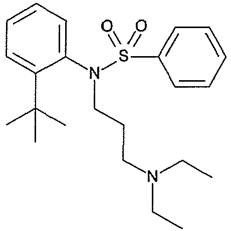

Under similar conditions to those in Working Example 28, using N-(2-t-butylphenyl)benzensulfonamide synthesized according to a known synthetic method, the title compound, N-[3-(diethylamino)propyl]-N-(2-t-butyl-phenyl)benzensulfonamide was obtained as colorless oil (40.8 mg, 58 %).
1H-NMR(500MHz,CDCl3) δ: 7.73(dd, J = 8.3 Hz, 1.4 Hz, 2H), 7.62-7.58 (m, 2H), 7.51 (dd, J = 7.5 Hz, 7.5 Hz, 2H), 7.25 (ddd, J = 7.6 Hz, 7.6 Hz, 1.3 Hz, 1H), 6.97 (ddd, J = 7.6 Hz, 7.6 Hz, 1.5 Hz, 1H), 6.40 (dd, J = 8.0 Hz, 1.7 Hz, 1H), 3.66 (m, 1H), 3.33 (m, 1H), 2.40 (q, J = 7.3 Hz, 4H), 2.34-2.25 (m, 2H), 1.77 (m, 1H), 1.55 (s, 9H), 1.47 (m, 1H), 0.93 (t, J = 7.2 Hz, 6H)
LCMS: m/z 403(M+H)⁺

### [Working Example 44]

### Compound No.: 4-17

### N-[3-(diethylamino)propyl]-N-(4-methoxy-2-phenyl-phenyl)benzensulfonamide

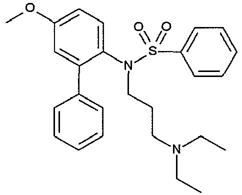

Under similar conditions to those in Working Example 28 using Compound No. 3-7, the title compound, N-[3-(diethylamino)propyl]-N-(4-methoxy-2-phenyl-phenyl)benzensulfonamide was obtained as colorless oil (27.5 mg, 56 %).
1H-NMR(400MHz,CDCl3) δ: 7.73 (d, J = 7.2 Hz, 2H), 7.58 (dd, J = 7.4 Hz, 7.4 Hz, 1H), 7.50-7.46 (m, 4H), 7.40-7.33 (m, 3H), 6.89 (d, J = 4.0 Hz, 1H), 6.87 (d, J = 1.8 Hz, 1H), 6.79 (dd, J = 9.0 Hz, 2.7 Hz, 1H), 3.82 (s, 3H), 3.21 (t, J = 7.6 Hz, 2H), 2.34-2.28 (m, 4H), 2.12-2.04 (m, 2H), 1.37-1.26 (m, 2H), 0.87 (t, J = 7.2 Hz, 6H)
LCMS: m/z 453(M+H)⁺

### [Working Example 45]

### Compound No.: 4-18

### t-butyl-4-[[N-(benzenesulfonyl)-4-methoxy-2-phenyl-anilino]methyl]piperidine-1-carboxylate

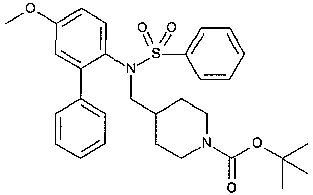

Under similar conditions to those in Working Example 28, using t-butyl-4-(bromomethyl)piperidine-1-carboxylate synthesized according to a known synthetic method and Compound No. 3-7 instead of diethylaminopropylchloride, the title compound, t-butyl-4-[[N-(benzenesulfonyl)-4-methoxy-2-phenyl-anilino]methyl]piperidine-1-carboxylate was obtained as colorless oil (60.0 mg, 75 %).
1H-NMR(500MHz,CDCl3) δ: 7.80 (d, J = 7.5 Hz, 2H), 7.64-7.52 (m, 5H), 7.41-7.34 (m, 3H), 6.88-6.72 (m,3H), 3.91 (br s, 1H), 3.82 (s, 3H), 3.67 (br s, 1H), 3.18 (br s, 1H), 2.86 (br s, 1H), 2.38 (br s, 1H), 2.10 (br t, J = 11.7 Hz, 1H), 1.40 (s, 9H), 1.19 (m, 1H), 1.09 (m, 1H), 0.88-0.80 (m, 2H), 0.63-0.61 (m, 1H)
LCMS: m/z 1095(2M+Na)⁺

### [Working Example 46]

### Compound No.: 4-19

### N-(4-methoxy-2-phenyl-phenyl)-N-(4-piperidylmethyl)benzensulfonamide

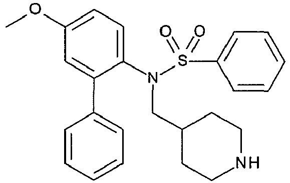

Compound No. 4-18 (112.0 mg, 0.20 mmol) was dissolved in dichloromethane (2 mL), and to this solution added trifluoroacetic acid (2 mL), which was stirred at room temperature for 1 hour.

The reaction solution was poured into saturated sodium bicarbonate aqueous solution (50 mL), which was extracted with chloroform (30 mL), and dried over sodium sulfate. After removing the desiccant, concentrating under reduced pressure gave the title compound, N-(4-methoxy-2-phenyl-phenyl)-N-(4-piperidylmethyl)benzensulfonamide as colorless oil (92.2 mg, 100 % yield).
1H-NMR(500MHz,CDCl3) δ: 7.82 (d, J = 8.0 Hz, 2H), 7.69 (m, 3H), 7.58 (dd, J = 7.7 Hz, 7.7 Hz, 2H), 7.43-7.36 (m, 3H), 6.91 (d, J = 2.9 Hz, 1H), 6.79 (dd, J = 9.2 Hz, 2.9 Hz, 1H), 6.65 (d, J = 8.6 Hz, 1H), 3.83 (s, 3H), 3.37 (br t, J = 11.2 Hz, 1H), 3.26 (br s, 1H), 2.98 (br s, 1H), 2.80 (br d, J = 12.0 Hz, 1H), 2.62 (br s, 1H), 2.23 (br s, 1H), 1.42 (br d, J = 11.5 Hz, 1H), 1.28-1.19 (m, 3H), 0.98 (br s, 2H)
LCMS: m/z 437(M+H)⁺

### [Working Example 47]

### Compound No.: 4-20

### N-(4-methoxy-2-phenyl-phenyl)-N-[1-methyl-4-piperidyl]methyl]benzensulfonamide

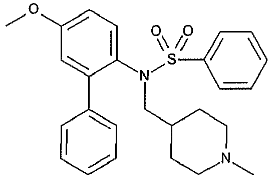

Compound No. 4-19 (18.0 mg, 0.04 mmol) was dissolved in anhydrous THF (1 mL), and to this solution added 60 % sodium hydride (4 mg, 0.1 mmol) and methyl iodide (0.006 mL, 0.1 mmol), which was stirred at room temperature for 1 hour.

The reaction solution was poured into water (10 mL), which was extracted with chloroform (30 mL), dried over sodium sulfate. After removing the desiccant, the residue obtained by concentration under reduced pressure was purified by silica gel column chromatography (Biotage Ultra, 10 g, 0 -> 10 % methanol/chloroform, elution with 15-column volume) to give the title compound, N-(4-methoxy-2-phenyl-phenyl)-N-[1-methyl-4-piperidyl]methyl]benzensulfonamide as colorless oil (3.8 mg, 20 % yield).
1H-NMR(500MHz,CDCl3) δ: 7.82 (d, J = 7.5 Hz, 2H), 7.68-7.63 (m, 3H), 7.58 (dd, J = 7.7 Hz, 7.7 Hz, 2H), 7.44-7.36 (m, 3H), 6.90 (d, J = 2.9 Hz, 1H), 6.79 (dd, J = 8.9 Hz, 3.2 Hz, 1H), 6.70 (d, J = 8.6 Hz, 1H), 3.83 (s, 3H), 3.38 (br t, J = 11.2 Hz, 1H), 3.19 (m, 1H), 2.98 (m, 1H), 2.82 (dd, J = 13.5 Hz, 3.7 Hz, 1H), 2.54 (br s, 3H), 2.30 (br s, 1H), 2.00 (br s, 1H), 1.60 (m, 1H), 1.37 (m, 1H), 1.19 (m, 1H), 1.09 (m, 1H), 0.86 (m, 1H)
LCMS: m/z 451 (M+H)⁺

### [Working Example 48]

### Compound No.: 4-21

### N-(4-methoxy-2-phenyl-phenyl)-N-[1-ethyl-4-piperidyl]methyl]benzensulfonamide

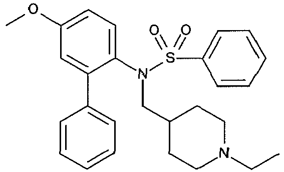

Under similar conditions to those in Working Example 47, using ethyl iodide instead of methyl iodide, the title compound, N-(4-methoxy-2-phenyl-phenyl)-N-[1-ethyl-4-piperidyl]methyl]benzensulfonamide was obtained as colorless oil (60.0 mg, 75 %).
1H-NMR(500MHz,CDCl3) δ: 7.80 (d, J = 7.5 Hz, 2H), 7.62 (dd, J = 7.5 Hz, 1H), 7.56-7.51 (m, 4H), 7.41-7.34 (m, 3H), 6.88 (d, J = 2.9 Hz, 1H), 6.85 (d, J = 8.6 Hz, 1H), 6.80 (dd, J = 8.6 Hz, 2.9 Hz, 1H), 3.82 (s, 3H), 3.14 (dd, J = 13.2 Hz, 8.6 Hz, 1H), 2.89 (dd, J = 13.2 Hz, 4.6 Hz, 1H), 2.83 (br d, J = 10.9 Hz, 1H), 2.65 (br d, J = 11.5 Hz, 1H), 2.38 (q, J = 7.3 Hz, 2H), 1.75 (m, 1H), 1.50 (br s, 1H), 1.27 (m, 1H), 1.14-1.01 (m, 1H), 1.05 (t, J = 7.2 Hz, 3H), 0.94-0.83 (m, 3H)
LCMS: m/z 465(M+H)⁺

### [Working Example 49]

### Compound No.: 4-22

### t-butyl-3-[[N-(benzenesulfonyl)-4-methoxy-2-phenyl-anilino]methyl]piperidine-1-carboxylate

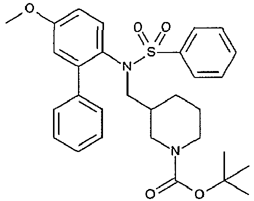

Under similar conditions to those in Working Example 45, using t-butyl-3-(bromomethyl)piperidine-1-carboxylate synthesized according to a known synthetic method and Compound No. 3-7 instead of t-butyl-4-(bromomethyl)piperidine-1-carboxylate, the title compound, t-butyl-3-[[N-(benzenesulfonyl)-4-methoxy-2-phenyl-anilino]methyl]piperidine-1-carboxylate was obtained as colorless oil (154.6 mg, 97 %).
1H-NMR(500MHz,CDCl3) Could not be analyzed for existing as a complex mixture of conformers.
LCMS: m/z 537(M+H)⁺

### [Working Example 50]

### Compound No.: 4-23

### N-(4-methoxy-2-phenyl-phenyl)-N-(3-piperidylmethyl)benzensulfonamide

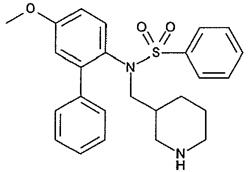

Under similar conditions to those in Working Example 46, using Compound No. 4-22 instead of Compound No. 4-18, the title compound, N-(4-methoxy-2-phenyl-phenyl)-N-(3-piperidylmethyl)benzensulfonamide was obtained as colorless waxy substance (113.0 mg, 99 %).
1H-NMR(500MHz,CDCl3) Could not be analyzed for existing as a complex mixture of conformers.
LCMS: m/z 537(M+H)⁺

### [Working Example 51]

### Compound No.: 4-24

### N-(4-methoxy-2-phenyl-phenyl)-N-[1-methyl-3-piperidyl]methyl]benzensulfonamide

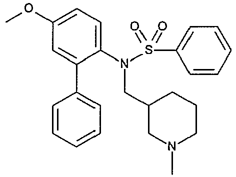

Under similar conditions to those in Working Example 47, using Compound No. 4-23 instead of Compound No. 4-19, the title compound, N-(4-methoxy-2-phenyl-phenyl)-N-[1-methyl-3-piperidyl]methyl]benzensulfonamide was obtained as colorless oil (18.4 mg, 63 % yield).
1H-NMR(500MHz,CDCl3) Could not be analyzed for existing as a complex mixture of conformers.
LCMS: m/z 451(M+H)⁺

### [Working Example 52]

### Compound No.: 4-25

### N-(4-methoxy-2-phenyl-phenyl)-N-[1-ethyl-3-piperidyl]methyl]benzensulfonamide

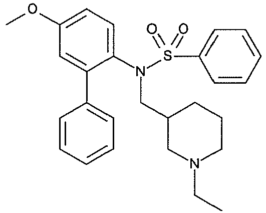

Under similar conditions to those in Working Example 48, using Compound No. 4-23 instead of Compound No. 4-19, the title compound, N-(4-methoxy-2-phenyl-phenyl)-N-[1-ethyl-3-piperidyl]methyl]benzensulfonamide was obtained as colorless oil (17.9 mg, 60 % yield).
1H-NMR(500MHz,CDCl3) Could not be analyzed for existing as a complex mixture of conformers.
LCMS: m/z 465(M+H)⁺

### [Working Example 53]

### Compound No.: 4-27

### N-[3-(dimethylamimo)propyl]-N-(4-methoxy-2-phenyl-phenyl)benzensulfonamide

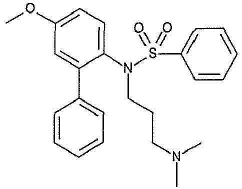

Under similar conditions to those in Working Example 45, using commercially obtained N,N-dimethylamimo-1-chloropropane instead of t-butyl-4-(bromomethyl)piperidine-1-carboxylate, the title compound, N-[3-(dimethylamimo)propyl]-N-(4-methoxy-2-phenyl-phenyl)benzensulfonamide was obtained as colorless oil (32.4 mg, 86 %).
1H-NMR(500MHz,CDCl3) δ: 7.72 (d, J = 7.5 Hz, 2H), 7.58 (dd, J = 7.5 Hz, 7.5 Hz, 1H), 7.51-7.46 (m, 4H), 7.40-7.34 (m, 3H), 6.88 (d, J = 2.9 Hz, 1H), 6.87 (d, J = 9.7 Hz, 1H), 6.79 (dd, J = 8.6 Hz, 2.9 Hz, 1H), 3.81 (s, 3H), 3.28-3.20 (m, 2H), 2.01 (s, 6H), 1.94 (m, 1H), 1.88 (m, 1H), 1.32 (m, 2H)
LCMS: m/z 425(M+H)⁺

### [Working Example 54]

### Compound No.: 4-28

### N-[3-(diethylamino)ethyl]-N-(4-methoxy-2-phenyl-phenyl)benzensulfonamide

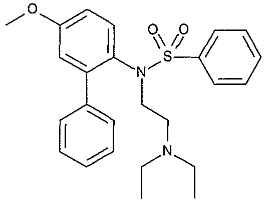

Under similar conditions to those in Working Example 45, using commercially obtained N,N-diethylamino-1-chloroethane instead of t-butyl-4-(bromomethyl)piperidine-1-carboxylate, the title compound, N-[3-(diethylamino)ethyl]-N-(4-methoxy-2-phenyl-phenyl)benzensulfonamide was obtained as colorless oil (13.1 mg, 33 %).
1H-NMR(500MHz,CDCl3) δ: 7.72 (d, J = 7.5 Hz, 2H), 7.58 (dd, J = 7.5 Hz, 7.5 Hz, 1H), 7.49-7.45 (m, 4H), 7.39-7.35 (m, 3H), 6.90 (d, J = 8.6 Hz, 1H), 6.88 (d, J = 2.9 Hz, 1H), 6.79 (dd, J = 8.9 Hz, 3.2 Hz, 1H), 3.82 (s, 3H), 3.27 (dd, J = 9.7 Hz, 6.3 Hz, 2H), 2.32 (q, J = 7.1 Hz, 4H), 2.24 (dd, J = 9.5 Hz, 6.6 Hz, 2H), 0.82 (t, J = 7.2 Hz, 6H)
LCMS: m/z 439(M+H)⁺

### [Working Example 55]

### Compound No.: 4-29

### t-butyl-N-[3-[N-(benzenesulfonyl)-4-methoxy-2-phenyl-anilino]propyl]-N-methylcarbamate

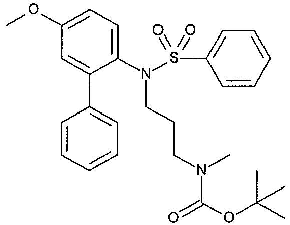

Under similar conditions to those in Working Example 45, using t-butyl-N-(3-chloropropyl)-N-methylcarbamate synthesized according to a known synthetic method instead of t-butyl-4-(bromomethyl)piperidine-1-carboxylate, the title compound, t-butyl-N-[3-[N-(benzenesulfonyl)-4-methoxy-2-phenyl-anilino]propyl]-N-methylcarbamate was obtained as colorless oil (26.3 mg, 58 %).
1H-NMR(400MHz,CDCl3) δ: 7.71 (d, J = 7.3 Hz, 2H), 7.59 (dd, J = 7.5 Hz, 7.5 Hz, 1H), 7.51-7.47 (m, 4H), 7.40-7.33 (m, 3H), 6.89-6.81 (m, 3H), 3.82 (s, 3H), 3.25 (br s, 1H), 3.16 (br s, 1H), 2.87 (br s, 2H), 2.62 (s, 3H), 1.40-1.34 (m, 11H)
LCMS: m/z 511(M+H)⁺, 533(M+Na)⁺

### [Working Example 56]

### Compound No.: 4-30

### N-(4-methoxy-2-phenyl-phenyl)-N-[3-(methylamino)propyl]-benzensulfonamide

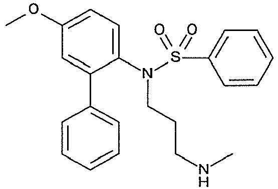

Under similar conditions to those in Working Example 46, using Compound No. 4-29 instead of Compound No. 4-18, the title compound, N-(4-methoxy-2-phenyl-phenyl)-N-[3-(methylamino)propyl]-benzensulfonamide was obtained as colorless powder (14.5 mg, 100 %).
1H-NMR(400MHz,CDCl3) δ: 8.78 (br s, 1H), 7.87 (d, J = 6.3 Hz, 2H), 7.70-7.56 (m, 5H), 7.46-7.39 (m, 3H), 6.88 (d, J = 3.1 Hz, 1H), 6.78 (dd, J = 8.5 Hz, 2.7 Hz, 1H), 6.56 (d, J = 9.0 Hz, 1H), 3.81 (m, 1H), 3.81 (s, 3H), 2.97 (br s, 1H), 2.50 (br s, 3H), 2.20 (m, 2H), 1.58 (br s, 1H), 1.35 (br s, 1H)
LCMS: m/z 411(M+H)⁺, 843(2M+Na)⁺, 433(M+Na)⁺

### [Working Example 57]

### Compound No.: 4-31

### t-butyl-N-[3-[N-(benzenesulfonyl)-4-methoxy-2-phenyl-anilino]propyl]carbamate

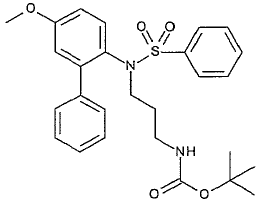

Under similar conditions to those in Working Example 45, using t-butyl-N-(3-chloropropyl)carbamate synthesized according to a known synthetic method instead of t-butyl-4-(bromomethyl)piperidine-1-carboxylate, the title compound, t-butyl-N-[3-[N-(benzenesulfonyl)-4-methoxy-2-phenyl-anilino]propyl]carbamate was obtained as colorless oil (34.5 mg, 78 %).
1H-NMR(500MHz,CDCl3) δ: 7.77 (d, J = 7.5 Hz, 2H), 7.62 (dd, J = 7.5 Hz, 7.5 Hz, 1H), 7.54-7.51 (m, 4H), 7.40-7.35 (m, 3H), 6.88 (d, J = 2.9 Hz, 1H), 6.79 (dd, J = 8.6 Hz, 2.9 Hz, 1H), 6.75 (d, J = 8.6 Hz, 1H), 4.53 (br s, 1H), 3.81 (s, 3H), 3.43 (m, 1H), 3.06 (m, 1H), 2.69 (br s, 1H), 2.47 (br s, 1H), 1.40 (s, 9H), 1.36 (br s, 1H), 1.20 (br s, 1H)
LCMS: m/z 1015(2M+Na)⁺, 519(M+Na)⁺, 497(M+H)⁺

### [Working Example 58]

### Compound No.: 4-32

### N-(3-aminopropyl)-N-(4-methoxy-2-phenyl-phenyl)benzensulfonamide

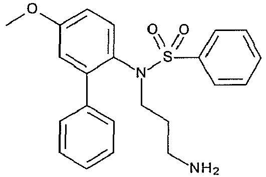

Under similar conditions to those in Working Example 46, using Compound No. 4-31 instead of Compound No. 4-18, the title compound, N-(3-aminopropyl)-N-(4-methoxy-2-phenyl-phenyl)benzensulfonamide was obtained as colorless powder (26.0 mg, 100 %).
1H-NMR(500MHz,CD3OD) δ: 7.77 (d, J = 6.9 Hz, 2H), 7.71 (dd, J = 7.5 Hz, 7.5 Hz, 1H), 7.60 (dd, J = 7.7 Hz, 7.7 Hz, 2H), 7.55-7.53 (m, 2H), 7.12-7.36 (m, 3H), 6.87 (d, J = 2.9 Hz, 1H), 6.85 (dd, J = 8.9 Hz, 3.2 Hz, 1H), 6.72 (d, J = 9.2 Hz, 1H), 3.80 (s, 3H), 3.58 (m, 1H), 3.15 (m, 1H), 2.46 (m, 1H), 2.24 (m, 1H), 1.49-1.42 (m, 2H)
LCMS: m/z 397(M+H)⁺, 815(2M+Na)⁺

### [Working Example 59]

### Compound No.: 4-33

### N-[3-(dimethylamimo)ethyl]-N-(4-methoxy-2-phenyl-phenyl)benzensulfonamide

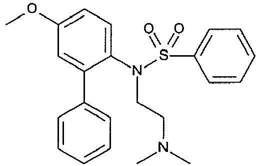

Under similar conditions to those in Working Example 45, using commercially obtained N,N-dimethylamimo-1-chloroethane instead of t-butyl-4-(bromomethyl)piperidine-1-carboxylate, the title compound, N-[3-(dimethylamimo)ethyl]-N-(4-methoxy-2-phenyl-phenyl)benzensulfonamide was obtained as colorless oil (29.7 mg, 82 %).
1H-NMR(500MHz,CDCl3) δ: 7.73 (d, J = 6.9 Hz, 2H), 7.58 (dd, J = 7.5 Hz, 7.5 Hz, 1H), 7.49-7.44 (m, 4H), 7.39-7.34 (m, 3H), 6.92 (d, J = 8.6 Hz, 1H), 6.88 (d, J = 2.9 Hz, 1H), 6.79 (dd, J = 8.6 Hz, 2.9 Hz, 1H), 3.82 (s, 3H), 3.35 (m, 1H), 3.34 (m, 1H), 2.10 (t, J = 7.7 Hz, 2H), 2.02 (s, 6H) LCMS: m/z 411(M+H)⁺, 843(2M+Na)⁺

### [Working Example 60]

### Compound No.: 4-34

### N-(4-methoxy-2-phenyl-phenyl)-N-(2-morpholinoethyl)benzensulfonamide

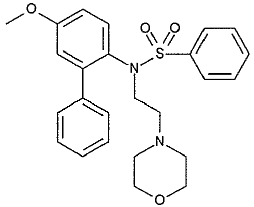

Under similar conditions to those in Working Example 45, using commercially obtained 2-morpholino-1-chloroethane instead of t-butyl-4-(bromomethyl)piperidine-1-carboxylate, the title compound, N-(4-methoxy-2-phenyl-phenyl)-N-(2-morpholinoethyl)benzensulfonamide was obtained as colorless oil (38.3 mg, 95 %).
1H-NMR(500MHz,CDCl3) δ: 7.73 (d, J = 7.5 Hz, 2H), 7.58 (dd, J = 7.5 Hz, 7.5 Hz, 1H), 7.49-7.45 (m, 4H), 7.39-7.34 (m, 3H), 6.95 (d, J = 9.2 Hz, 1H), 6.87 (d, J = 2.9 Hz, 1H), 6.79 (dd, J = 8.9 Hz, 3.2 Hz, 1H), 3.82 (s, 3H), 3.53 (t, J = 4.6 Hz, 4H), 3.38 (m, 1H), 3.24 (m, 1H), 2.18 (m, 6H) LCMS: m/z 453(M+H)⁺

### [Working Example 61]

### Compound No.: 4-35

### N-(4-methoxy-2-phenyl-phenyl)-N-[2-(1-piperidyl)ethyl]benzensulfonamide

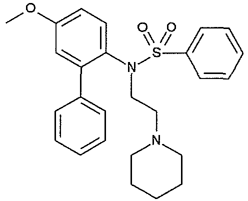

Under similar conditions to those in Working Example 45, using commercially obtained 2-piperidino-1-chloroethane instead of t-butyl-4-(bromomethyl)piperidine-1-carboxylate, the title compound, N-(4-methoxy-2-phenyl-phenyl)-N-[2-(1-piperidyl)ethyl]benzensulfonamide was obtained as colorless oil (37.1 mg, 93 %).
1H-NMR(500MHz,CDCl3) δ: 7.73(d, J = 7.5 Hz, 2H), 7.57 (dd, J = 7.5 Hz, 7.5 Hz, 1H), 7.51-7.45 (m, 4H), 7.40-7.34 (m, 3H), 6.90 (d, J = 8.6 Hz, 1H), 6.88 (d, J = 2.9 Hz, 1H), 6.78 (dd, J = 8.6 Hz, 2.9 Hz, 1H), 3.82 (s, 3H), 3.40-3.28 (m, 2H), 2.17-2.10 (m, 6H), 1.44-1.40 (m, 4H), 1.35-1.31 (m, 2H)
LCMS: m/z 451(M+H)⁺

### [Working Example 62]

### Compound No.: 4-36

### N-[1-isopropyl-4-piperidyl]methyl]-N-(4-methoxy-2-phenyl-phenyl)benzensulfonamide

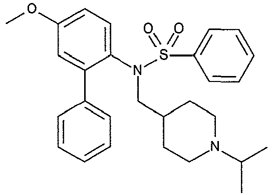

Under similar conditions to those in Working Example 47, using 2-propane iodide instead of methyl iodide, the title compound, N-[1-isopropyl-4-piperidyl]methyl]-N-(4-methoxy-2-phenyl-phenyl)benzensulfonamide was obtained as colorless oil (16.0 mg, 30 %).
1H-NMR(400MHz,CDCl3) δ: 7.78 (d, J = 7.2 Hz, 2H), 7.59 (dd, J = 7.2 Hz, 7.2 Hz, 1H), 7.54-7.48 (m, 4H), 7.40-7.33 (m, 3H), 6.89-6.86 (m, 2H), 6.79 (br d, J = 9.0 Hz, 1H), 3.81 (s, 3H), 3.05 (dd, J = 13.0 Hz, 7.2 Hz, 1H), 2.88 (br d, J = 13.0 Hz, 1H), 2.68 (br d, J = 10.3 Hz, 1H), 2.60 (br s, 1 H), 2.50 (br d, J = 10.8 Hz, 1H), 1.84 (br s, 1H), 1.60 (dd, J = 11.0 Hz, 11.0 Hz, 1H), 1.23 (br s, 2H), 1.01-0.79 (m, 3H), 0.94 (d, J = 5.8 Hz, 6H)
LCMS: m/z 479(M+H)⁺

### [Working Example 63]

### Compound No.: 4-37

### t-butyl-4-[[N-(benzenesulfonyl)-4-ethoxy-2-phenyl-anilino]methyl]piperidine-1-carboxylate

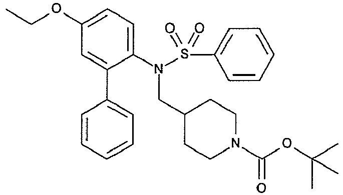

Under similar conditions to those in Working Example 45, using Compound No. 3-8 instead of Compound No. 3-7, the title compound, t-butyl-4-[[N-(benzenesulfonyl)-4-ethoxy-2-phenyl-anilino]methyl]piperidine-1-carboxylate was obtained as colorless oil (71.9 mg, 68 %).
1H-NMR(500MHz,CDCl3) δ: 7.80 (d, J = 7.5 Hz, 2H), 7.64-7.52 (m, 5H), 7.38 (m, 3H), 6.88-6.70 (m, 3H), 4.08-3.99 (m, 2H), 3.92 (br s, 1H), 3.66 (br s, 1H), 3.18 (br s, 1H), 2.86 (br s, 1H), 2.38 (br s, 1H), 2.09 (dd, J = 11.7 Hz, 11.7 Hz, 1H), 1.41 (t, J = 6.6 Hz, 3H), 1.40 (s, 9H), 1.19 (br d, J = 11.5 Hz, 1H), 1.08 (m, 1H), 0.88-0.79 (m, 2H), 0.62 (m, 1H)
LCMS: m/z 1124(2M+Na)⁺

### [Working Example 64]

### Compound No.: 4-38

### N-(4-ethoxy-2-phenyl-phenyl)-N-(4-piperidylmethyl)benzensulfonamide

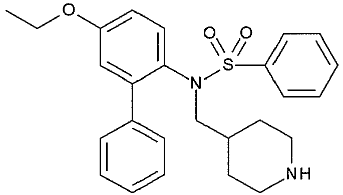

Under similar conditions to those in Working Example 46, using Compound No. 4-37instead of Compound No. 4-18, the title compound, N-(4-ethoxy-2-phenyl-phenyl)-N-(4-piperidylmethyl)benzensulfonamide was obtained as colorless oil (58.0 mg, 98 %).
1H-NMR(500MHz,CDCl3) δ: 7.79 (d, J = 6.9 Hz, 2H), 7.61 (dd, J = 7.5 Hz, 7.5 Hz, 1H), 7.55-7.50 (m, 4H), 7.39-7.32 (m, 3H), 6.86 (d, J = 2.9 Hz, 1H), 6.80 (d, J = 8.6 Hz, 1H), 6.77 (dd, J = 8.6 Hz, 2.9 Hz, 1H), 4.08-3.99 (m, 2H), 3.11 (dd, J = 13.2 Hz, 9.2 Hz, 1H), 2.87-2.83 (m, 2H), 2.66 (d, J = 12.6 Hz, 1H), 2.28 (ddd, J = 11.5 Hz, 11.5 Hz, 2.3 Hz, 1H), 2.02 (ddd, J = 12.0 Hz, 12.0 Hz, 2.3 Hz, 1H), 1.80 (br s, 1H), 1.41 (t, J = 6.9 Hz, 3H), 1.20 (d, J = 12.6 Hz, 1H), 1.06 (m, 1H), 0.85-0.76 (m, 2H), 0.60 (m, 1H)
LCMS: m/z 451(M+H)⁺, 901(2M+H)⁺

### [Working Example 65]

### Compound No.: 4-39

### N-(4-ethoxy-2-phenyl-phenyl)-N-[1-ethyl-4-piperidyl]methyl]benzensulfonamide

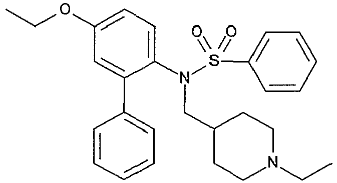

Under similar conditions to those in Working Example 48, using Compound No. 4-38 instead of Compound No. 4-19, the title compound, N-(4-ethoxy-2-phenyl-phenyl)-N-[1-ethyl-4-piperidyl]methyl]benzensulfonamide was obtained as colorless oil (42.0 mg, 68 %).
1H-NMR(400MHz,CDCl3) δ: 7.79 (d, J = 7.2 Hz, 2H), 7.61 (dddd, J = 7.4 Hz, 7.4 Hz, 1.6 Hz, 1.6 Hz, 1H), 7.56-7.49 (m, 4H), 7.41-7.32 (m, 3H), 6.87 (d, J = 1.8 Hz, 1H), 6.85 (d, J = 4.0 Hz, 1H), 6.79 (dd, J = 9.0 Hz, 2.7 Hz, 1H), 4.09-3.98 (m, 2H), 3.09 (d, J = 13.0 Hz, 8.1 Hz, 1H), 2.89 (d, J = 13.2 Hz, 4.3 Hz, 1H), 2.74 (br d, J = 10.8 Hz, 1H), 2.56 (br d, J = 11.7 Hz, 1H), 2.27 (q, J = 7.3 Hz, 2H), 1.61 (dd, J = 10.1 Hz, 1H), 1.42 (m, 1H), 1.41 (t, J = 7.2 Hz, 3H), 1.23 (d, J = 9.4 Hz, 1H), 1.05-0.94 (m, 2H), 0.99 (t, J = 7.2 Hz, 3H), 0.85-0.73 (m, 2H)
LCMS: m/z 479(M+H)⁺, 957(2M+H)⁺

### [Working Example 66]

### Compound No.: 4-40

### t-butyl-4-[[N-(benzenesulfonyl)-4-ethyl-2-phenyl-anilino]methyl]piperidine-1-carboxylate

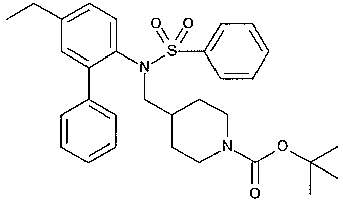

Under similar conditions to those in Working Example 45, using Compound No. 3-9 instead of Compound No. 3-7, the title compound, t-butyl-4-[[N-(benzenesulfonyl)-4-ethyl-2-phenyl-anilino]methyl]piperidine-1-carboxylate was obtained as colorless oil (84.4 mg, 71 %).
1H-NMR(400MHz,CDCl3) δ: 7.81 (d, J = 7.3 Hz, 2H), 7.65-7.52 (m, 5H), 7.41-7.33 (m, 3H), 7.21 (d, J = 2.3 Hz, 1H), 7.10 (d, J = 8.2 Hz, 1H), 6.77 (br s, 1H), 3.92 (br s, 1H), 3.67 (br s, 1H), 3.18 (br s, 1H), 2.89 (br s, 1H), 2.68 (q, J = 7.7 Hz, 2H), 2.38 (br s, 1H), 2.10 (br t, J = 12.0 Hz, 1H), 1.40 (s, 9H), 1.26 (t, J = 7.5 Hz, 3H), 1.19 (m, 1H), 1.09 (m, 1H), 0.88-0.78 (m, 2H), 0.63 (m, 1H) LCMS: m/z 1091(2M+Na)⁺, 557(M+Na)⁺

### [Working Example 67]

### Compound No.: 4-41

### N-(4-ethyl-2-phenyl-phenyl)-N-(4-piperidylmethyl)benzensulfonamide

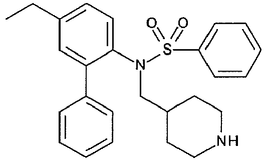

Under similar conditions to those in Working Example 46, using Compound No. 4-40 instead of Compound No. 4-18, the title compound, N-(4-ethyl-2-phenyl-phenyl)-N-(4-piperidylmethyl)benzensulfonamide was obtained as colorless oil (67.0 mg, 98 %).
1H-NMR(500MHz,CDCl3) δ: 7.81 (d, J = 7.5 Hz, 2H), 7.61 (dd, J = 7.5 Hz, 7.5 Hz, 1H), 7.55-7.51 (m, 4H), 7.39-7.32 (m, 3H), 7.19 (d, J = 1.7 Hz, 1H), 7.10 (dd, J = 8.3 Hz, 2.0 Hz, 1H), 6.84 (d, J = 8.6 Hz, 1H), 3.12 (dd, J = 13.2 Hz, 9.2 Hz, 1H), 2.90-2.85 (m, 2H), 2.71-2.64 (m, 3H), 2.29 (br t, J = 10.9 Hz, 1H), 2.04 (br t, J = 11.2 Hz, 1H), 1.92 (br s, 1H), 1.26 (t, J = 7.7 Hz, 3H), 1.22 (br d, J = 13.2 Hz, 1H), 1.07 (m, 1H), 0.87-0.77 (m, 2H), 0.61 (m, 1H)
LCMS: m/z 435(M+H)⁺, 869(2M+H)⁺

### [Working Example 68]

### Compound No.: 4-42

### N-(4-methoxy-2-phenyl-phenyl)-N-[1-methyl-3-piperidyl]methyl]benzensulfonamide

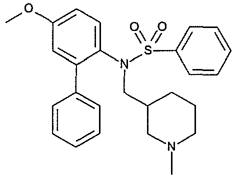

Under similar conditions to those in Working Example 47, using Compound No. 4-41 instead of Compound No. 4-19, the title compound, N-(4-methoxy-2-phenyl-phenyl)-N-[1-methyl-3-piperidyl]methyl]be,nzensulfonamide was obtained as colorless oil (28.2 mg, 40 %).
1H-NMR(500MHz,CDCl3) δ: 7.81 (d, J = 7.5 Hz, 2H), 7.60 (dd, J = 7.5 Hz, 7.5 Hz, 1H), 7.54-7.50 (m, 4H), 7.40-7.33 (m, 3H), 7.19 (d, J = 1.7 Hz, 1H), 7.12 (dd, J = 8.3 Hz, 2.0 Hz, 1H), 6.92 (d, J = 8.6 Hz, 1H), 3.06 (dd, J = 13.2 Hz, 8.0 Hz, 1H), 2.94 (dd, J = 13.2 Hz, 4.6 Hz, 1H), 2.70 (m, 1H), 2.69 (q, J = 7.6 Hz, 2H), 2.54 (br d, J = 11.5 Hz, 1H), 2.23 (q, J = 7.3 Hz, 2H), 1.58 (br t, J = 10.1 Hz, 1H), 1.37 (br t, J = 11.2 Hz, 1H), 1.27 (t, J = 7.7 Hz, 3H), 1.24 (m, 1H), 1.00-0.91 (m, 2H), 0.97 (t, J = 7.2 Hz, 3H), 0.86 (m, 1H), 0.74 (m, 1H)
LCMS: m/z 463(M+H)⁺, 925(2M+H)⁺

### [Working Example 69]

### Compound No.: 4-43

### t-butyl-4-[[N-(benzenesulfonyl)-4-isopropyl-2-phenyl-anilino]methyl]piperidine-1-carboxylate

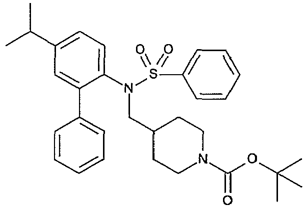

Under similar conditions to those in Working Example 45, using Compound No. 3-10 instead of Compound No. 3-7, the title compound, t-butyl-4-[[N-(benzenesulfonyl)-4-isopropyl-2-phenyl-anilino]methyl]piperidine-1-carboxylate was obtained as colorless oil (103.7 mg, 78 %).
1H-NMR(500MHz,CDCl3) δ: 7.81 (d, J = 7.5 Hz, 2H), 7.64-7.52 (m, 5H), 7.41-7.34 (m, 3H), 7.22 (d, J = 2.3 Hz, 1H), 7.12 (d, J = 8.6 Hz, 1H), 6.74 (br s, 1H), 3.92 (br s, 1H), 3.66 (br s, 1H), 3.18 (br s, 1H), 2.97-2.85 (m, 2H), 2.39 (br s, 1H), 2.10 (br t, J = 12.0 Hz, 1H), 1.40 (s, 9H), 1.27 (dd, J = 6.9 Hz, 2.3 Hz, 6H), 1.19 (br d, J = 11.5 Hz, 1H), 1.10 (m, 1H), 0.89-0.79 (m, 2H), 0.62 (br d, J = 9.2 Hz, 1H)
LCMS: m/z 1119(2M+Na)⁺, 571(M+Na)⁺

### [Working Example 70]

### Compound No.: 4-44

### N-(4-isopropyl-2-phenyl-phenyl)-N-(4-piperidylmethyl)benzensulfonamide

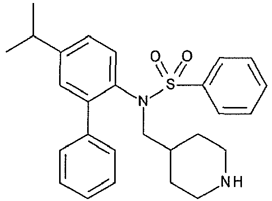

Under similar conditions to those in Working Example 46, using Compound No. 4-43 instead of Compound No. 4-18, the title compound, N-(4-isopropyl-2-phenyl-phenyl)-N-(4-piperidylmethyl)benzensulfonamide was obtained as colorless oil (77.2 mg, 94 %).
1H-NMR(500MHz,CDCl3) δ: 7.81 (d, J = 7.5 Hz, 2H), 7.61 (dd, J = 7.5 Hz, 7.5 Hz, 1H), 7.55-7.51 (m, 4H), 7.40-7.32(m,3H), 7.21 (d, J = 2.3 Hz, 1H), 7.12 (dd, J = 8.3 Hz, 2.0 Hz, 1H), 6.84 (d, J = 8.0 Hz, 1H), 3.11 (dd, J = 13.2 Hz, 9.2 Hz, 1H), 2.98 (m, 3H), 2.66 (br d, J = 12.0 Hz, 1H), 2.29 (dd, J = 11.2 Hz, 11.2 Hz, 1H), 2.03 (dd, J = 11.5 Hz, 11.5 Hz, 1H), 1.51 (br s, 1H), 1.27 (d, J = 5.7 Hz, 6H), 1.22 (m, 1H), 1.08 (m, 1H), 0.87-0.76 (m,2H), 0.60 (m, 1H)
LCMS: m/z 449(M+H)⁺, 897(2M+H)⁺

### [Working Example 71]

### Compound No.: 4-45

### N-(4-isopropyl-2-phenyl-phenyl)-N-(1-ethyl-4-piperidylmethyl)benzensulfonamide

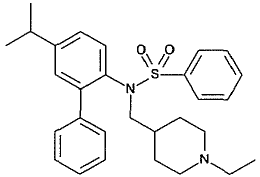

Under similar conditions to those in Working Example 48, using Compound No. 4-44 instead of Compound No. 4-19 ,the title compound, N-(4-isopropyl-2-phenyl-phenyl)-N-(1-ethyl-4-piperidylmethyl)benzensulfonamide was obtained as colorless oil (70.2 mg, 85 %).
1H-NMR(500MHz,CDCl3) δ: 7.80 (d, J = 7.5 Hz, 2H), 7.61 (dd, J = 7.5 Hz, 7.5 Hz, 1H), 7.55-7.50 (m, 4H), 7.40-7.33 (m, 3H), 7.20 (d, J = 1.7 Hz, 1H), 7.13 (dd, J = 8.6 Hz, 2.3 Hz, 1H), 6.88 (d, J = 8.0 Hz, 1H), 3.10 (dd, J = 13.5 Hz, 8.3 Hz, 1H), 2.96-2.90 (m, 2H), 2.77 (br d, J = 10.9 Hz, 1H), 2.59 (br d, J = 10.9 Hz, 1H), 2.31 (q, J = 7.3 Hz, 2H), 1.67 (br t, J = 10.3 Hz, 1H), 1.44 (br t, J = 10.9 Hz, 1H), 1.28-1.23 (m, 1H), 1.27 (d, J = 1.7 Hz, 3H), 1.26 (d, J = 2.3 Hz, 3H), 1.06-0.99 (m, 2H), 1.01 (t, J = 7.2 Hz, 3H), 0.90-0.78 (m, 2H)
LCMS: m/z 477(M+H)⁺

### [Working Example 72]

### Compound No.: 4-46

### t-butyl-4-[[N-(benzenesulfonyl)-4-t-butyl-2-phenyl-anilino]methyl]piperidine-1-carboxylate

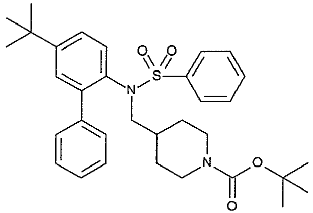

Under similar conditions to those in Working Example 45, using Compound No. 3-11 instead of Compound No. 3-7, the title compound, t-butyl-4-[[N-(benzenesulfonyl)-4-t-butyl-2-phenyl-anilino]methyl]piperidine-1-carboxylate was obtained as colorless oil (147.1 mg, 100 %).
1H-NMR(500MHz,CDCl3) δ: 7.81 (d, J = 7.5 Hz, 2H), 7.70-7.52 (m, 5H), 7.41-7.34 (m, 4H), 7.26 (dd, J = 8.3 Hz, 2.0 Hz, 1H), 6.75 (br s, 1H), 3.91 (br s, 1H), 3.65 (br s, 1H), 3.17 (br s, 1H), 2.88 (br s, 1H), 2.39 (br s, 1H), 2.10 (br t, J = 12.0 Hz, 1H), 1.39 (s, 9H), 1.33 (s, 9H), 1.19 (m, 1H), 1.09 (m, 1H), 0.84-0.82 (m, 2H), 0.62 (m, 1H)
LCMS: m/z 1147(2M+Na)⁺, 585(M+Na)⁺

### [Working Example 73]

### Compound No.: 4-47

### N-(4-t-butyl-2-phenyl-phenyl)-N-(4-piperidylmethyl)benzensulfonamide

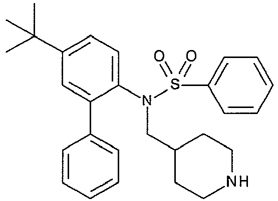

Under similar conditions to those in Working Example 46, using Compound No. 4-46 instead of Compound No. 4-18, the title compound, N-(4-t-butyl-2-phenyl-phenyl)-N-(4-piperidylmethyl)benzensulfonamide was obtained as colorless oil (109.0 mg, 90 %).
1H-NMR(400MHz,CDCl3) δ: 7.81 (d, J = 7.2 Hz, 2H), 7.62 (dddd, J = 7.4 Hz, 7.4 Hz, 1.6 Hz, 1.6 Hz, 1H), 7.57-7.51 (m, 4H), 7.39 (dddd, J = 7.0 Hz, 7.0 Hz, 1.8 Hz, 1.8 Hz, 2H), 7.36-7.34 (m, 2H), 7.27 (dd, J = 8.3 Hz, 2.5 Hz, 1H), 6.83 (d, J = 8.1 Hz, 1H), 3.12 (dd, J = 13.2 Hz, 9.2 Hz, 1H), 2.89-2.84 (m, 2H), 2.65 (br d, J = 12.6 Hz, 1H), 2.29 (br t, J = 11.0 Hz, 1H), 2.02 (br t, J = 11.0 Hz, 1H), 1.55 (br s, 1H), 1.33 (s, 9H), 1.21 (m, 1H), 1.06 (m, 1H), 0.85-0.74 (m, 2H), 0.59 (m, 1H)
LCMS: m/z 463(M+H)⁺, 925(2M+H)⁺

### [Working Example 74]

### Compound No.: 4-48

### N-(4-t-butyl-2-phenyl-phenyl)-N-(1-ethyl-4-piperidylmethyl)benzensulfonamide

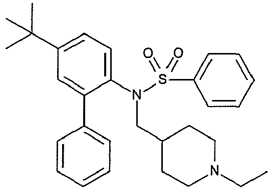

Under similar conditions to those in Working Example 48, instead of Compound No. 4-19, the title compound, N-(4-t-butyl-2-phenyl-phenyl)-N-(1-ethyl-4-piperidylmethyl)benzensulfonamide was obtained as colorless oil (32.8 mg, 34 %).
1H-NMR(400MHz,CDCl3) δ: 7.82 (d, J = 7.2 Hz, 2H), 7.68-7.55 (m, 5H), 7.44-7.34 (m, 4H), 7.26 (dd, J = 8.3 Hz, 2.5 Hz, 1H), 6.70 (d, J = 8.1 Hz, 1H), 3.34 (dd, J = 13.2 Hz, 10.1 Hz, 1H), 3.17 (br d, J = 9.4 Hz, 1H), 2.96 (br d, J = 10.8 Hz, 1H), 2.85-2.76 (m, 3H), 2.24 (br s, 1H), 1.89 (br s, 1H), 1.54 (m, 1H), 1.39-1.24 (m, 2H), 1.32 (s, 9H), 1.29 (t, J = 7.4 Hz, 3H), 1.16 (m, 1H), 1.05 (br d, J = 13.5 Hz, 1H)
LCMS: m/z 491(M+H)⁺

### [Working Example 75]

### Compound No.: 4-49

### t-butyl-4-[[N-(benzenesulfonyl)-2-(4-fluorophenyl)-4-methoxy-anilino]methyl]piperidine-1-carboxylate

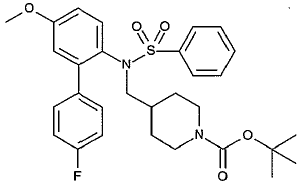

Under similar conditions to those in Working Example 45, using Compound No. 3-12 instead of Compound No. 3-7, the title compound, t-butyl-4-[[N-(benzenesulfonyl)-2-(4-fluorophenyl)-4-methoxy-anilino]methyl]piperidine-1-carboxylate was obtained as colorless oil (169.1 mg, 94 %).
1H-NMR(400MHz,CDCl3) δ: 7.79 (d, J = 7.2 Hz, 2H), 7.65 (dd, J = 7.4 Hz, 7.4 Hz, 2H), 7.55 (dd, J = 7.6 Hz, 7.6 Hz, 3H), 7.11 (dd, J = 8.1 Hz, 8.1 Hz, 2H), 6.85 (d, J = 2.7 Hz, 1H), 6.79 (d, J = 7.2 Hz, 1H), 6.68 (br s, 1H), 3.94 (br s, 1H), 3.82 (s, 3H), 3.73 (br s, 1H), 3.23 (br s, 1H), 2.85 (br s, 1H), 2.39 (br s, 1H), 2.13 (dd, J = 11.4 Hz, 11.4 Hz, 1H), 1.41 (s, 9H), 1.19 (d, J = 12.6 Hz, 1H), 1.07 (d, J = 12.1 Hz, 1H), 0.87 (dd, J = 12.1 Hz, 7.2 Hz, 2H), 0.71 (dd, J = 10.6 Hz, 10.5 Hz, 1H) LCMS: m/z No peaks.

### [Working Example 76]

### Compound No.: 4-50

### N-[2-(4-fluorophenyl)-4-methoxy-phenyl)-N-(4-piperidylmethyl)benzensulfonamide

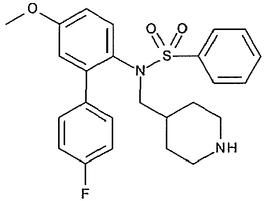

Under similar conditions to those in Working Example 46, using Compound No. 4-49 instead of Compound No. 4-18, the title compound, N-[2-(4-fluorophenyl)-4-methoxy-phenyl)-N-(4-piperidylmethyl)benzensulfonamide was obtained as colorless oil (138.4 mg, 100 %).
1H-NMR(400MHz,CDCl3) δ: 7.80 (d, J = 7.9 Hz, 2H), 7.67-7.54 (m, 5H), 7.11 (dd, J = 8.6 Hz, 8.6 Hz, 2H), 6.85 (d, J = 2.7 Hz, 1H), 6.78 (dd, J = 9.1 Hz, 3.2 Hz, 1H), 6.68 (d, J = 9.1 Hz, 1H), 5.78 (br s, 1H), 3.82 (s, 3H), 3.29 (dd, J = 13.1 Hz, 8.6 Hz, 1H), 3.09 (d, J = 13.1 Hz, 1H), 2.94 (d, J = 12.7 Hz, 1H), 2.80 (dd, J = 13.4 Hz, 3.4 Hz, 1H), 2.44 (dd, J = 11.3 Hz, 11.3 Hz, 1H), 2.14 (m, 1H), 1.31 (d, 10.9 Hz, 1H), 1.21-1.12 (m, 2H), 0.99 (br s, 2H)
LCMS: m/z 455(M+H)⁺, 909(2M+H)⁺

### [Working Example 77]

### Compound No.: 4-51

### N-[2-(4-fluorophenyl)-4-methoxy-phenyl)-N-(1-ethyl-4-piperidylmethyl)benzensulfonamide

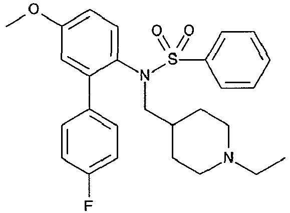

Under similar conditions to those in Working Example 48, using Compound No. 4-50 instead of Compound No. 4-19, the title compound, N-[2-(4-fluorophenyl)-4-methoxy-phenyl)-N-(1-ethyl-4-piperidylmethyl)benzensulfonamide was obtained as colorless oil (74.8 mg, 50 %).

1H-NMR(500MHz,CDCl3) δ: 7.79 (d, J = 8.0 Hz, 2H), 7.62 (dd, J = 7.5 Hz, 7.4 Hz, 1H), 7.57-7.52 (m, 4H), 7.10 (dd, J = 8.9 Hz, 8.8 Hz, 2H), 6.85-6.78 (m, 3H), 3.83 (s, 3H), 3.13 (dd, J = 13.2 Hz, 8.0 Hz, 1H), 2.89 (dd, J = 13.2 Hz, 4.6 Hz, 1H), 2.75 (d, J = 10.9 Hz, 1H), 2.63 (d, J = 10.9 Hz, 1H), 2.28 (dd, J = 14.3 Hz, 6.9 Hz, 2H), 1.63 (dd, J = 10.3 Hz, 10.3 Hz, 1H), 1.42 (dd, J = 10.6 Hz, 10.6 Hz, 1H), 1.23 (d, J = 11.5 Hz, 1H), 1.04-0.84 (m, 4H), 1.01 (t, J = 7.2 Hz, 3H)
LCMS: m/z 483(M+H)⁺, 965(2M+H)⁺

### [Working Example 78]

### Compound No.: 4-52

### t-butyl-4-[[N-(benzenesulfonyl)-2-(3-fluorophenyl)-4-methoxy-anilino]methyl]piperidine-1-carboxylate

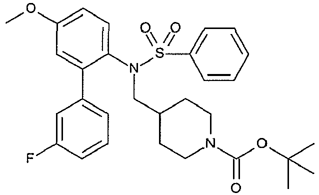

Under similar conditions to those in Working Example 45, using Compound No. 3-13 instead of Compound No. 3-7, the title compound, t-butyl-4-[[N-(benzenesulfonyl)-2-(3-fluorophenyl)-4-methoxy-anilino]methyl]piperidine-1-carboxylate was obtained as colorless oil (200.7 mg, 96 %).
1H-NMR(500MHz,CDCl3) δ: 7.79 (d, J = 8.0 Hz, 2H), 7.64 (dd, J = 7.5 Hz, 7.5 Hz, 1H), 7.54 (dd, J = 7.7 Hz, 7.7 Hz, 2H), 7.48-7.18 (m, 3H), 7.08 (br s, 1H), 6.86 (br s, 1H), 6.81 (d, J = 5.7 Hz, 1H), 6.72 (br s, 1H), 3.97 (br s, 1H), 3.83 (s, 3H), 3.73 (br s, 1H), 3.22 (br s, 1H), 2.88 (br s, 1H), 2.42 (br s, 1H), 2.15 (dd, J = 11.7 Hz, 11.7 Hz, 1H), 1.41 (s, 9H), 1.21 (d, J = 11.5 Hz, 1H), 1.10 (d, J = 8.6 Hz, 1H), 0.86 (d, J = 12.0 Hz, 2H), 0.68 (br s, 1H)
LCMS: m/z No peaks.

### [Working Example 79]

### Compound No.: 4-53

### N-[2-(3-fluorophenyl)-4-methoxy-phenyl)-N-(4-piperidylmethyl)benzensulfonamide

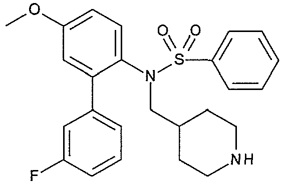

Under similar conditions to those in Working Example 46, using Compound No. 4-52 instead of Compound No. 4-18, the title compound, N-[2-(3-fluorophenyl)-4-methoxy-phenyl)-N-(4-piperidylmethyl)benzensulfonamide was obtained as colorless oil (171.2 mg, 100 %).
1H-NMR(500MHz,CDCl3) δ: 7.80 (d, J = 7.5Hz, 2H), 7.65 (dd, J = 7.5 Hz, 7.5 Hz, 1H), 7.55 (dd, J = 7.7 Hz, 7.7 Hz, 2H), 7.40-7.37 (m, 2H), 7.35 (d, J = 7.5 Hz, 1H), 7.07 (m, 1H), 6.86 (d, J = 2.9 Hz, 1H), 6.81 (dd, J = 8.6 Hz, 2.9 Hz, 1H), 6.74 (d, J = 8.6 Hz, 1H), 5.03 (br s, 1H), 3.83 (s, 3H), 3.29 (dd, J = 13.2 Hz, 9.2 Hz, 1H), 3.10 (d, J = 12.6 Hz, 1H), 2.92 (d, J = 12.6 Hz, 1H), 2.84 (dd, J = 13.2 Hz, 3.4 Hz, 1H), 2.44 (dd, J = 12.0 Hz, 12.0 Hz, 1H), 2.15 (dd, J = 12.0 Hz, 12.0 Hz, 1H), 1.33 (d, J = 12.0 Hz, 1H), 1.21-1.09 (m, 2H), 0.98 (br s, 2H),
LCMS: m/z 455(M+H)⁺, 909(2M+H)⁺

### [Working Example 80]

### Compound No.: 4-54

### N-[2-(3-fluorophenyl)-4-methoxy-phenyl)-N-(1-ethyl-4-piperidylmethyl)benzensulfonamide

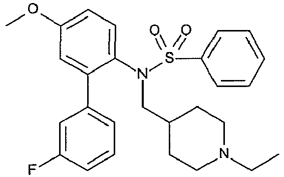

Under similar conditions to those in Working Example 48, using Compound No. 4-53 instead of Compound No. 4-19, the title compound, N-[2-(3-fluorophenyl)-4-methoxy-phenyl)-N-(1-ethyl-4-piperidylmethyl)benzensulfonamide was obtained as colorless oil (35.5 mg, 20 %).
1H-NMR(500MHz,CDCl3) δ: 7.79 (d, J = 8.0 Hz, 2H), 7.62 (dd, J = 7.5 Hz, 7.5 Hz, 1H), 7.53 (dd, J = 7.7 Hz, 7.7 Hz, 2H), 7.41-7.35 (m, 2H), 7.23 (d, J = 10.3 Hz, 1H), 7.07 (dd, J = 9.5 Hz, 9.5 Hz, 1H), 6.89-6.81 (m, 3H), 3.83 (s, 3H), 3.15 (m, 1H), 2.91 (dd, J = 13.2 Hz, 4.0 Hz, 1H), 2.79 (br s, 1H), 2.63 (br s, 1H), 2.31 (br s, 2H), 1.68 (br s, 1H), 1.46 (br s, 1H), 1.27-1.25 (m, 2H), 1.02 (br s, 1H), 1.02 (t, J = 6.6 Hz, 3H), 0.92-0.82 (m, 2H)
LCMS: m/z 483(M+H)⁺, 965(2M+H)⁺

### [Working Example 81]

### Compound No.: 4-55

### t-butyl-4-[[N-(benzenesulfonyl)-2-(2-fluorophenyl)-4-methoxy-anilino]methyl]piperidine-1-carboxylate

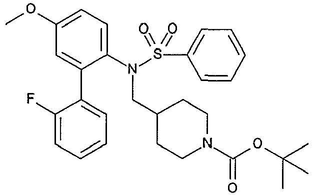

Under similar conditions to those in Working Example 45, using Compound No. 3-14 instead of Compound No. 3-7, the title compound, t-butyl-4-[[N-(benzenesulfonyl)-2-(2-fluorophenyl)-4-methoxy-anilino]methyl]piperidine-1-carboxylate was obtained as colorless oil (108.2 mg, 88 %).
1H-NMR(400MHz,CDCl3) δ: 7.89 (br s, 1H), 7.75 (d, J = 7.6 Hz, 2H), 7.64 (dd, J = 7.4 Hz, 7.4 Hz, 1H), 7.53 (dd, J = 7.4 Hz, 7.4 Hz, 2H), 7.37 (br s, 1H), 7.24 (br s, 1H), 7.10 (dd, J = 9.0 Hz, 9.0 Hz, 1H), 6.88 (br s, 1H), 6.84 (d, J = 8.1 Hz, 1H), 6.74 (br s, 1H), 4.00 (br s, 1H), 3.81 (s, 3H), 3.73 (br s, 1H), 3.23 (br s, 1H), 2.89 (br s, 1H), 2.46 (br s, 1H), 2.26 (br s, 1H), 1.40 (s, 9H), 1.26 (br s, 2H), 0.92-0.82 (m, 2H), 0.67 (br s, 1H)
LCMS: m/z 1109(2M+H)⁺, 1131(2M+Na)⁺, 555(M+H)⁺, 577(M+Na)⁺

### [Working Example 82]

### Compound No.: 4-56

### N-[2-(2-fluorophenyl)-4-methoxy-phenyl)-N-(4-piperidylmethyl)benzensulfonamide

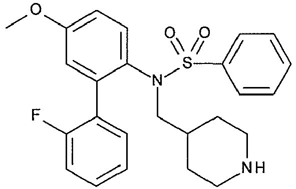

Under similar conditions to those in Working Example 46, using Compound No. 4-55 instead of Compound No. 4-18, the title compound, N-[2-(2-fluorophenyl)-4-methoxy-phenyl)-N-(4-piperidylmethyl)benzensulfonamide was obtained as colorless oil (82.2 mg, 92 %).
1H-NMR(500MHz,CDCl3) δ: 7.86 (dd, J = 6.9 Hz, 6.9 Hz, 1H), 7.76 (d, J = 7.5 Hz, 1H), 7.63 (dd, J = 7.5 Hz, 7.5 Hz, 1H), 7.54 (dd, J = 7.7 Hz, 7.7 Hz, 2H), 7.36 (dd, J = 13.8 Hz, 5.7 Hz, 1H), 7.23 (dd, J = 7.7 Hz, 7.7 Hz, 1H), 7.10 (dd, J = 8.6 Hz, 8.6 Hz, 1H), 6.88 (d, J = 1.7 Hz, 1H), 6.84 (dd, J = 8.9 Hz, 3.2 Hz, 1H), 6.78 (d, J = 9.2 Hz, 1H), 3.82 (s, 3H), 3.27 (dd, J = 12.9 Hz, 9.5 Hz, 1H), 3.10 (d, J = 12.0 Hz, 1H), 2.92 (d, J = 12.6 Hz, 1H), 2.86 (dd, J = 13.5 Hz, 3.7 Hz, 1H), 2.48 (dd, J = 11.2 Hz, 11.2 Hz, 1H), 2.27 (dd, J = 10.9 Hz, 10.9 Hz, 1H), 1.36 (d, J = 12.6 Hz, 1H), 1.28 (m, 1H), 1.14 (m, 1H), 1.01-0.89 (m, 2H)
LCMS: m/z 455(M+H)⁺, 909(2M+H)⁺

### [Working Example 83]

### Compound No.: 4-57

### N-[2-(2-fluorophenyl)-4-methoxy-phenyl)-N-(1-ethyl-4-piperidylmethyl)benzensulfonamide

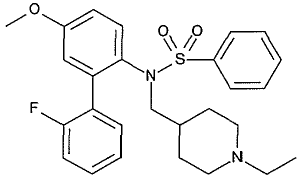

Under similar conditions to those in Working Example 48, using Compound No. 4-56 instead of Compound No. 4-19, the title compound, N-[2-(2-fluorophenyl)-4-methoxy-phenyl)-N-(1-ethyl-4-piperidylmethyl)benzensulfonamide was obtained as pale yellow oil (27.8 mg, 32 %).
1H-NMR(500MHz,CDCl3) δ: 7.76 (m, 3H), 7.60 (dd, J = 7.5 Hz, 7.5 Hz, 1H), 7.50 (dd, J = 7.7 Hz, 7.7 Hz, 2H), 7.36 (m, 1H), 7.22 (dd, J = 7.2 Hz, 7.2 Hz, 1H), 7.10 (dd, J = 9.2 Hz, 9.2 Hz, 1H), 6.92 (d, J = 8.6 Hz, 1H), 6.87 (br s, 1H), 6.86 (dd, J = 8.6 Hz, 2.9 Hz, 1H), 3.82 (s, 3H), 3.13 (dd, J = 10.9 Hz, 10.9 Hz, 1H), 2.94 (dd, J = 13.5 Hz, 4.9 Hz, 1H), 2.77 (br s, 1H), 2.62 (br s, 1H), 2.29 (br s, 2H), 1.67 (br s, 1H), 1.51 (br s, 1H), 1.30-1.24 (m, 2H), 1.13 (br s, 1H), 1.01 (t, J = 7.5 Hz, 3H), 0.93 (d, J = 13.8 Hz, 1H), 0.83 (br s, 1H)
LCMS: m/z 483(M+H)⁺, 965(2M+H)⁺

### [Working Example 84]

### Compound No.: 4-58

### t-butyl-4-[(N-(4-fluorophenyl)sulfonyl-4-methoxy-2-phenyl-anilino)methyl]piperidine-1-carboxylate

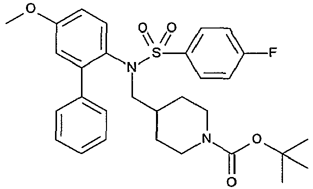

Under similar conditions to those in Working Example 45, using Compound No. 3-15 instead of Compound No. 3-7, the title compound, t-butyl-4-[(N-(4-fluorophenyl)sulfonyl-4-methoxy-2-phenyl-anilino)methyl]piperidine-1-carboxylate was obtained as colorless oil (298.1 mg, 81 %).
1H-NMR(500MHz,CDCl3) δ: 7.79 (dd, J = 9.2 Hz, 5.2 Hz, 2H), 7.57 (br s, 2H), 7.42-7.38 (m, 3H), 7.21 (dd, J = 8.6 Hz, 8.6 Hz, 2H), 6.89 (d, J = 2.3 Hz, 1H), 6.81 (d, J = 5.7 Hz, 1H), 6.73 (br s, 1H), 3.97 (br s, 1H), 3.83 (s, 3H), 3.67 (br s, 1H), 3.17 (br s, 1H), 2.87 (br s, 1H), 2.40 (br s, 1H), 2.10 (dd, J = 11.7 Hz, 11.7 Hz, 1H), 1.40 (s, 9H), 1.21 (d, J = 11.5 Hz, 1H), 1.10 (dd, J = 8.9 Hz, 3.7 Hz, 1H), 0.89-0.79 (m, 2H), 0.62 (d, J = 9.2 Hz,1H)
LCMS: m/z 1109(2M+H)⁺, 1131(2M+Na)⁺, 555(M+H)⁺, 577(M+Na)⁺

### [Working Example 85]

### Compound No.: 4-59

### 4-fluoro-N-(4-methoxy-2-phenyl-phenyl)-N-(4-piperidylmethyl)benzensulfonamide

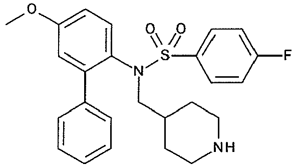

Under similar conditions to those in Working Example 46, using Compound No. 4-58 instead of Compound No. 4-18, the title compound, 4-fluoro-N-(4-methoxy-2-phenyl-phenyl)-N-(4-piperidylmethyl)benzensulfonamide was obtained as pale yellow oil (78.2 mg, 95 %).
1H-NMR(400MHz,CDCl3) δ: 7.82-7.80 (m, 2H), 7.58 (d, J = 7.5 Hz, 2H), 7.43-7.34 (m, 3H), 7.21 (dd, J = 8.6 Hz, 8.6 Hz, 2H), 6.89 (d, J = 3.2 Hz, 1H), 6.81 (dd, J = 8.8 Hz, 2.9 Hz, 1H), 6.74 (d, J = 9.1 Hz, 1H), 5.07 (br s, 1H), 3.82 (s, 3H), 3.21 (dd, J = 13.4 Hz, 8.8Hz, 1H), 3.01 (d, J = 12.7 Hz, 1H), 2.85-2.77 (m, 2H), 2.38 (dd, J = 11.3 Hz, 11.3 Hz, 1H), 2.08 (dd, J = 12.2 Hz, 3.2 Hz, 1H), 1.25 (m, 1H), 1.13-0.99 (m, 2H), 0.94-0.76 (m, 2H)
LCMS: m/z 455(M+H)⁺, 909(2M+H)⁺

### [Working Example 86]

### Compound No.: 4-60

### 4-fluoro-N-(4-methoxy-2-phenyl-phenyl)-N-(1-ethyl-4-piperidylmethyl)benzensulfonamide

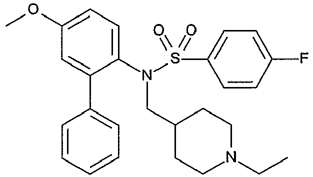

Under similar conditions to those in Working Example 48, using Compound No. 4-59 instead of Compound No. 4-19, the title compound, 4-fluoro-N-(4-methoxy-2-phenyl-phenyl)-N-(1-ethyl-4-piperidylmethyl)benzensulfonamide was obtained as pale yellow oil (14.9 mg, 18 %).
1H-NMR(500MHz,CDCl3) δ: 7.80-7.77 (m, 2H), 7.54 (d, J = 6.9 Hz, 2H), 7.42-7.35 (m, 3H), 7.19 (dd, J = 8.6 Hz, 8.6 Hz, 2H), 6.88 (d, J = 2.9 Hz, 2H), 6.83 (dd, J = 8.6 Hz, 2.9 Hz, 1H), 3.83 (s, 3H), 3.10 (br s, 1H), 2.91 (d, J = 10.3 Hz,1H), 2.80 (br s, 1H), 2.60 (br s, 1H), 2.30 (br s, 2H), 1.65 (br s, 1H), 1.43 (br s, 1H), 1.25 (br s, 2H), 1.08-0.97 (m, 4H), 0.88 (m, 2H)
LCMS: m/z 483(M+H)⁺, 965(2M+H)⁺

### [Working Example 87]

### Compound No.: 4-61

### t-butyl-4-[(N-(3-fluorophenyl)sulfonyl-4-methoxy-2-phenyl-anilino)methyl]piperidine-1-carboxylate

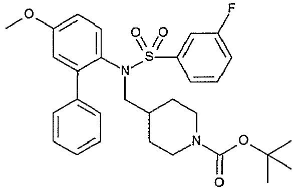

Under similar conditions to those in Working Example 45, using Compound No. 3-16 instead of Compound No. 3-7, the title compound, t-butyl-4-[(N-(3-fluorophenyl)sulfonyl-4-methoxy-2-phenyl-anilino)methyl]piperidine-1-carboxylate was obtained as colorless oil (326.2 mg, 90 %).
1H-NMR(400MHz,CDCl3) δ: 7.60-7.48 (m, 5H), 7.41-7.32 (m, 4H), 6.89 (d, J = 2.3 Hz, 1H), 6.82 (d, J = 6.8 Hz, 1H), 6.77 (br s, 1H), 3.94 (br s, 1H), 3.83 (s, 3H), 3.69 (br s, 1H), 3.17 (br s, 1H), 2.90 (br s, 1H), 2.40 (br s, 1H), 2.11 (dd, J = 11.6 Hz, 11.6 Hz, 1H), 1.40 (s, 9H), 1.22 (d, J = 15.9 Hz, 1H), 1.11 (dd, J = 8.6Hz, 3.6 Hz, 1H), 0.91-0.80 (m, 2H), 0.64 (d, J = 9.06 Hz, 1H)
LCMS: m/z 1109(2M+H)⁺, 555(M+H)⁺

### [Working Example 88]

### Compound No.: 4-62

### 3-fluoro-N-(4-methoxy-2-phenyl-phenyl)-N-(4-piperidylmethyl)benzensulfonamide

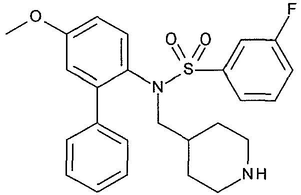

Under similar conditions to those in Working Example 46, using Compound No. 4-61 instead of Compound No. 4-18, the title compound, 4-fluoro-N-(4-methoxy-2-phenyl-phenyl)-N-(4-piperidylmethyl)benzensulfonamide was obtained as colorless oil (96.6 mg, 100 %).
1H-NMR(500MHz,CDCl3) δ: 7.61 (dd, J = 6.9 Hz, 6.9 Hz, 3H), 7.55 (ddd, J = 8.0 Hz, 8.0 Hz, 5.2 Hz, 1H), 7.51 (ddd, J = 8.0 Hz, 2.0 Hz, 2.0 Hz, 1H), 7.42 (dd, J = 7.2 Hz, 7.2 Hz, 2H), 7.38-7.33 (m, 2H), 7.22 (br s, 1H), 6.90(d, J = 2.9 Hz, 1H), 6.80 (dd, J = 8.6 Hz, 2.9 Hz, 1H), 6.69 (d, J = 8.6 Hz, 1H), 3.83 (s, 3H), 3.30 (dd, J = 13.2 Hz, 9.7 Hz, 1H), 3.13 (d, J = 12.6 Hz, 1H), 2.89 (d, J = 13.2 Hz, 1H), 2.82 (dd, J = 13.5 Hz, 3.7 Hz, 1H), 2.46 (dd, J = 11.9 Hz, 11.9 Hz, 1H), 2.10 (ddd, J = 12.5 Hz, 12.5 Hz, 3.2 Hz, 1H), 1.34 (d, J = 12.6 Hz, 1H), 1.23 (m, 1H), 1.12 (d, J = 6.9 Hz, 1H), 0.98 (m, 2H)
LCMS: m/z 455(M+H)⁺, 909(2M+H)⁺

### [Working Example 89]

### Compound No.: 4-63

### 3-fluoro-N-(4-methoxy-2-phenyl-phenyl)-N-(1-ethyl-4-piperidylmethyl)benzensulfonamide

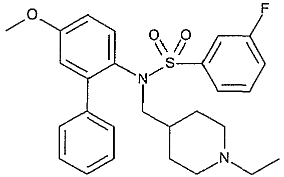

Under similar conditions to those in Working Example 48, using Compound No. 4-62 instead of Compound No. 4-19, the title compound, 3-fluoro-N-(4-methoxy-2-phenyl-phenyl)-N-(1-ethyl-4-piperidylmethyl)benzensulfonamide was obtained as pale yellow oil (47.0 mg, 49 %).
1H-NMR(500MHz,CDCl3) δ: 7.80-7.77 (m, 2H), 7.54 (d, J = 6.9 Hz, 2H), 7.42-7.35 (m, 3H), 7.19 (dd, J = 8.6 Hz, 8.6 Hz, 2H), 6.88 (d, J = 2.9 Hz, 2H), 6.83 (dd, J = 8.6 Hz, 2.9 Hz, 1H), 3.83 (s, 3H), 3.10 (br s, 1H), 2.91 (d, J = 10.3 Hz,1H), 2.80 (br s, 1H), 2.60 (br s, 1H), 2.30 (br s, 2H), 1.65 (br s, 1H), 1.43 (br s, 1H), 1.25 (br s, 2H), 1.08-0.97 (m, 4H), 0.88 (m, 2H)
LCMS: m/z 483(M+H)⁺, 965(2M+H)⁺

### [Working Example 90]

### Compound No.: 4-64

### t-butyl-4-[(N-(2-fluorophenyl)sulfonyl-4-methoxy-2-phenyl-anilino)methyl]piperidine-1-carboxylate

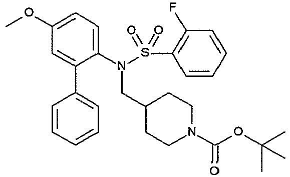

Under similar conditions to those in Working Example 45, using Compound No. 3-17 instead of Compound No. 3-7, the title compound, t-butyl-4-[(N-(2-fluorophenyl)sulfonyl-4-methoxy-2-phenyl-anilino)methyl]piperidine-1-carboxylate was obtained as colorless oil (181.1 mg, 95 %).
1H-NMR(400MHz,CDCl3) δ: 7.77 (ddd, J = 7.5 Hz, 7.5 Hz, 1.4 Hz, 1H), 7.61 (dd, J = 13.0 Hz, 7.5 Hz, 1H), 7.55 (d, J = 8.5 Hz, 1H), 7.36 (br s, 4H), 7.30-7.20 (m, 2H), 6.84 (br s, 1H), 6.80 (br s, 1H), 6.76 (d, J = 4.9 Hz, 1H), 3.95 (br s, 1H), 3.80 (s, 3H), 3.68 (br s, 1H), 3.32 (br s, 1H), 3.05 (br s, 1H), 2.40 (br s, 1H), 2.15 (dd, J = 12.7 Hz, 12.7 Hz, 1H), 1.40 (s, 9H), 1.28 (m, 1H), 1.13 (br s, 1H), 0.95-0.86 (m, 2H), 0.76 (m, 1H)
LCMS: m/z 1131(2M+Na)⁺, 577(M+Na)⁺

### [Working Example 91]

### Compound No.: 4-65

### 2-fluoro-N-(4-methoxy-2-phenyl-phenyl)-N-(4-piperidylmethyl)benzensulfonamide

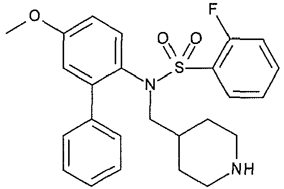

Under similar conditions to those in Working Example 46, using Compound No. 4-64 instead of Compound No. 4-18, the title compound, 2-fluoro-N-(4-methoxy-2-phenyl-phenyl)-N-(4-piperidylmethyl)benzensulfonamide was obtained as colorless oil (105.6 mg, 96 %).
1H-NMR(400MHz,CDCl3) δ: 7.78 (ddd, J = 7.6 Hz, 7.6 Hz, 1.5 Hz, 1H), 7.60 (m, 1H), 7.46 (d, J = 4.5 Hz, 1H), 7.44 (d, J = 1.8 Hz, 1H), 7.35 (dd, J = 4.5 Hz, 1.8 Hz, 3H), 7.29-7.19 (m, 2H), 6.86 (d, J = 9.0 Hz, 1H), 6.83 (d, J = 2.7 Hz, 1H), 6.78 (dd, J = 8.8 Hz, 2.9 Hz, 1H), 3.81 (s, 3H), 3.20 (dd, J = 13.4 Hz, 7.6 Hz, 1H), 3.08 (dd, J = 13.4 Hz, 5.8 Hz, 1H), 2.90 (d, J = 12.1 Hz, 1H), 2.74 (d, J = 12.6 Hz, 1H), 2.30 (ddd, J = 12.1 Hz, 12.1 Hz, 2.4 Hz, 1H), 2.09 (ddd, J = 12.1 Hz, 12.1 Hz, 2.4 Hz, 1H), 1.65 (br s, 1H), 1.35 (d, J = 12.6 Hz, 1H), 1.11 (m, 1H), 0.93 (d, 12.1 Hz, 1H), 0.87 (dd, J = 12.3 Hz, 3.8 Hz, 1H), 0.76 (m, 1H)
LCMS: m/z 455(M+H)⁺

### [Working Example 92]

### Compound No.: 4-66

### 2-fluoro-N-(4-methoxy-2-phenyl-phenyl)-N-(1-ethyl-4-piperidylmethyl)benzensulfonamide

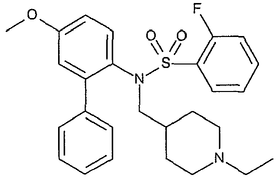

Under similar conditions to those in Working Example 48, using Compound No. 4-65 instead of Compound No. 4-19, the title compound, 2-fluoro-N-(4-methoxy-2-phenyl-phenyl)-N-(1-ethyl-4-piperidylmethyl)benzensulfonamide was obtained as pale yellow oil (64.2 mg, 66 %).
1H-NMR(400MHz,CDCl3) δ: 7.76 (ddd, J = 7.5 Hz, 7.5 Hz, 1.4 Hz, 1H), 7.61 (m, 1H), 7.49 (dd, J = 7.2 Hz, 2.2 Hz, 2H), 7.36 (dd, J = 4.9 Hz, 2.2 Hz, 3H), 7.29-7.20 (m, 2H), 6.83 (d, J = 2.7 Hz, 1H), 6.79 (d, J = 8.5 Hz, 1H), 6.76 (dd, J = 8.8 Hz, 2.9 Hz, 1H), 3.79 (s, 3H), 3.34 (dd, J = 13.5 Hz, 6.7 Hz, 1H), 3.06 (dd, J = 13.7 Hz, 3.8 Hz, 1H), 3.00 (d, J = 11.2 Hz, 1H),2.83 (d, J = 11.2 Hz, 1H), 2.58 (q, J = 7.2 Hz, 2H), 2.03 (br s, 1H), 1.78 (br s, 1H), 1.44 (d, J = 13.0 Hz, 1H), 1.30-1.22 (m, 2H), 1.19-1.02 (m, 2H), 1.13 (t, 7.4 Hz, 3H)
LCMS: m/z 483(M+H)+, 965(2M+H)⁺

### [Working Example 93]

### Compound No.: 4-67

### N-[3-(dimethylamimo)propyl]-N-[2-(4-fluorophenyl)-4-methoxy-phenyl)benzensulfonamide

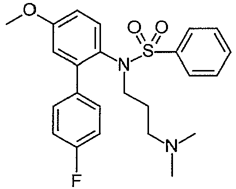

Under similar conditions to those in Working Example 45,using Compound No. 3-12 and commercially obtained N,N-dimethylamimo-1-chloropropane instead of Compound No. 3-7, the title compound, N-[3-(dimethylamimo)propyl]-N-[2-(4-fluorophenyl)-4-methoxyphenyl)benzensulfonamide was obtained as colorless oil (88.4 mg, 71 %).
1H-NMR(500MHz,CDCl3) δ: 7.74 (d, J = 6.9 Hz, 2H), 7.60 (dd, J = 7.5 Hz, 7.5 Hz, 1H), 7.52-7.48 (m, 4H), 7.08 (ddd, J = 9.2 Hz, 2.6 Hz, 2.6 Hz, 2H), 6.85 (d, J = 2.3 Hz, 1H), 6.79-6.78 (m, 2H), 3.82 (s, 3H), 3.32 (m, 1H), 3.18 (m, 1H), 2.01 (s, 6H), 1.95 (m, 1H), 1.90 (m, 1H), 1.33-1.25 (m, 2H)
LCMS: m/z 443(M+H)⁺

### [Working Example 94]

### Compound No.: 4-68

### N-[3-(diethylamino)propyl]-N-[2-(4-fluorophenyl)-4-methoxy-phenyl)benzensulfonamide

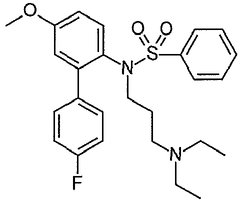

Under similar conditions to those in Working Example 45, using Compound No. 3-12 and N,N-diethylamino-1-chloropropane synthesized according to a known method instead of Compound No. 3-7, the title compound, N-[3-(diethylamino)propyl]-N-[2-(4-fluorophenyl)-4-methoxy-phenyl)benzensulfonamide was obtained as colorless oil (3.51g, 70 %).
1H-NMR(500MHz,CDCl3) δ: 7.74 (d, J =6.9 Hz, 2H), 7.61 (dd, J = 7.5 Hz, 7.5 Hz, 1H), 7.52-7.48 (m, 4H), 7.08 (dd, J = 8.6 Hz, 8.6 Hz, 2H), 6.85(d, J = 2.9 Hz, 1H), 6.79-6.78 (m, 2H), 3.82 (s, 3H), 3.27 (br s, 1H), 3.20-3.14 (m, 1H), 2.33 (br s, 4H), 2.13 (br s, 1H), 2.07 (m, 1H), 1.32-1.29 (m, 2H), 0.89 (t, J = 6.9 Hz, 6H)
LCMS: m/z 471(M+H)⁺

### [Working Example 95]

### Measuring Ebola virus infection rate

Functions of the surface glycoproteins (GPs) of Ebola and Marburg virus (filovirus) are essential for the entry process to all cells and deeply involved in the virulence of the viruses. Therefore, by suppressing the cell invasion mediated by the surface glycoproteins, the establishment of infection can completely be inhibited.

Using a pseudotype system of vesicular stomatitis virus (VSV), the infection-inhibiting effect by a compound can be screened. The specificity for a pseudotype virus of a filovirus (the virus on the right in Fig.1) was confirmed using the presence/absence of inhibitory effect on the replication of VSV parent strain (the virus on the left in Fig.1) as an index. This is an already-established system and have been used in screening for neutralizing activity of monoclonal antibodies (Takada A et al. A system for functional analysis of Ebola virus glycoprotein. Proc Natl Acad Sci USA 94:14764-9,1997; Nakayama E et al. Antibody-dependent enhancement of Marburg virus infection. J Infect Dis 204 Suppl 3:S978-85, 2011). In brief, said system was prepared as follows. In the full-length cDNA clone of VSV genome, the G-protein-coding region was replaced with a modified GFP gene (VSVΔG*-G), which was used to infect 233T cells that have been transfected with a plasmid that expresses filovirus GFP, and, after incubation, the supernatant was collected.

The pseudotype system described as above was used to assess the activity of the compounds which inhibit the cell invasion processes by Ebola virus.

The procedures are as follows:
1. The compounds synthesized in Working Examples described above were serially diluted and each was mixed with the pseudotype VSV (expressing GFP) having the surface glycoprotein of Ebola virus (Zaire sp.).
2. The pseudotype VSV mixed with the compound was added to VeroE6 cells in 96-well plate in approximately 1000 IU/well and allowed to infect the cells.
3. After 18 hours, the number of the cells expressing GFP was counted using IN cell Analyzer, and infection-inhibiting efficacy was calculated referring to the number of GFP-positive cells in the control well (no compound; the negative control) as 100 %.

The results are shown in the tables below. In all compounds examined, 50 % or higher inhibition of infection was confirmed at 5 µM.

**[Table 6-1]**

| Compound No. | Viral infection rate (%) | | | | | | | |
|---|---|---|---|---|---|---|---|---|
| | Compound Conc. 5 uM | Compound Conc. 2.5 uM | Compound Conc. 1.25 uM | Compound Conc. 0.625 uM | Compound Conc. 0.312 uM | Compound Conc. 0.078 uM | Compound Conc. 0.020 uM | Compound Conc. 0.005 uM |
| 4-21 | 0.17 | 0.12 | 0.18 | 0.22 | 0.75 | 25.50 | 64.56 | 93.59 |
| 4-17 | 0.22 | 0.61 | 020 | 2.05 | 1.91 | 34.58 | 72.60 | 91.16 |
| 4-20 | 0.17 | 0.09 | 0.23 | 2.58 | 4.58 | 43.73 | 76.79 | 91.82 |
| 4-27 | 0.20 | 0.14 | 0.16 | 0.40 | 3.49 | 45.02 | 81.47 | 98.98 |
| 4-51 | 0.00 | | 0.51 | | 11.97 | 44.96 | 84.52 | 92.17 |
| 4-54 | 0.00 | | 0.10 | | 28.58 | 74.55 | 90.05 | 94.53 |
| 4-63 | 0.00 | | 0.81 | | 34.34 | 76.24 | 96.25 | 96.70 |
| 4-36 | 0.00 | | 0.32 | | 36.12 | 78.11 | 90.97 | 92.58 |
| 4-25 | 0.14 | 0.14 | 2.51 | 24.32 | 55.06 | | | |

**[Table 6-2]**

| Compound No. | Viral infection rate (%) | | | | | | | |
|---|---|---|---|---|---|---|---|---|
| | Compound Conc. 5 uM | Compound Conc. 2.5 uM | Compound Conc. 1.25 uM | Compound Conc. 0.625 uM | Compound Conc. 0.312 uM | Compound Conc. 0.078 uM | Compound Conc. 0.020 uM | Compound Conc. 0.005 uM |
| 4-57 | 0.83 | | 1.00 | | 58.25 | 93.30 | 99.47 | 100.97 |
| 4-66 | 0.25 | | 1.67 | | 52.78 | 88.32 | 102.58 | 99.94 |
| 4-42 | 0.09 | 0.25 | 4.15 | 29.37 | 61.17 | 101.89 | 101.35 | 95.59 |
| 4-60 | 0.00 | | 3.97 | | 61.85 | | | |
| 4-14 | 0.68 | 0.86 | 7.82 | 42.29 | 77.44 | | | |
| 4-1 | 1.63 | 2.83 | 12.59 | 38.56 | 57.52 | | | |
| 4-11 | 2.09 | 3.34 | 15.38 | 42.85 | 68.35 | | | |
| 4-33 | 0.10 | 1.51 | 17.08 | 71.32 | 81.07 | | | |
| 4-8 | 1.11 | 1.93 | 19.62 | 67.59 | 89.04 | | | |

**[Table 6-3]**

| Compound No. | Viral infection rate (%) | | | | | | | |
|---|---|---|---|---|---|---|---|---|
| | Compound Conc. 5uM | Compound Conc. 2.5 uM | Compound Conc. 1.25 uM | Compound Conc. 0.625 uM | Compound Conc. 0.312 uM | Compound Conc. 0.078 uM | Compound Conc. 0.020 uM | Compound Conc. 0.005 uM |
| 4-9 | 2.32 | 3.93 | 20.60 | 58.62 | 82.55 | | | |
| 4-42 | 0.00 | | 22.89 | | 93.02 | 102.88 | 103.88 | 95.97 |
| 4-28 | 0.09 | 3.36 | 29.08 | 52.49 | 78.03 | | | |
| 4-4 | 2.05 | 8.13 | 29.67 | 71.34 | 84.40 | | | |
| 4-30 | 0.12 | 4.17 | 31.07 | 60.75 | 80.87 | | | |
| 4-7 | 5.08 | 12.91 | 36.98 | 68.41 | 87.20 | | | |
| 4-11 | 1.95 | 12.35 | 40.46 | 67.41 | 84.57 | | | |
| 4-32 | 0.47 | 15.75 | 57.76 | 82.37 | 85.98 | | | |
| 4-26 | 1.76 | 16.78 | 54.23 | 82.22 | 96.90 | | | |

**[Table 6-4]**

| Com-pound No. | Viral infection rate (%) | | | | | | | |
|---|---|---|---|---|---|---|---|---|
| | Compound Conc. 5 uM | Compound Conc. 2.5 uM | Compound Conc. 1.25 uM | Compound Conc. 0.625 uM | Compound Conc. 0.312 uM | Compound Conc. 0.078 uM | Compound Conc. 0.020 uM | Compound Conc. 0.005 uM |
| 4-5 | 3.15 | 16.75 | 54.88 | 94.18 | 105.27 | | | |
| 4-3 | 3.80 | 18.41 | 52.76 | 88.12 | 105.33 | | | |
| 4-19 | 2.20 | 18.89 | 52.32 | 78.51 | 89.34 | | | |
| 4-6 | 3.80 | 20.14 | 57.10 | 91.12 | 91.17 | | | |
| 4-45 | 0.23 | | 69.47 | | 103.41 | 104.08 | 101.14 | 96.75 |
| 4-10 | 3.57 | 22.68 | 69.85 | 103.34 | 106.38 | | | |
| 4-23 | 1.79 | 24.46 | 69.66 | 90.07 | 100.01 | | | |
| 4-15 | 6.43 | 30.26 | 61.62 | 82.30 | 89.53 | | | |
| 4-35 | 0.96 | 30.42 | 65.59 | 78.88 | 83.98 | | | |

**[Table 6-5]**

| Compound No. | Viral infection rate (%) | | | | | | | |
|---|---|---|---|---|---|---|---|---|
| | Compound Conc. 5 uM | Compound Conc. 2.5 uM | Compound Conc. 1.25 uM | Compound Conc. 0.625 uM | Compound Conc. 0.312 uM | Compound Conc. 0.078 uM | Compound Conc. 0.020 uM | Compound Conc. 0.005 uM |
| 4-29 | 5.94 | 35.83 | 78.71 | 102.84 | 99.83 | | | |
| 4-34 | 4.56 | 40.23 | 76.88 | 91.43 | 94.30 | | | |
| 4-16 | 11.03 | 45.56 | 73.56 | 87.61 | 97.33 | | | |
| 4-31 | 8.51 | 51.23 | 67.80 | 95.56 | 100.50 | | | |
| 4-22 | 8.71 | | | | | | | |
| 4-39 | 9.80 | | 115.92 | | 108.42 | 99.87 | 102.72 | 96.42 |
| 4-13 | 10.78 | 50.76 | 76.09 | 90.23 | 97.27 | | | |
| 4-18 | 23.29 | | | | | | | |
| 4-2 | 47.14 | | | | | | | |
| 4-48 | 47.57 | | 112.00 | | 103.37 | 96.33 | 101.79 | 97.14 |

### [Working Example 96]

### Measurement of infection-inhibiting efficacy against multiple Ebola virus species and Marburg virus

The pseudotype VSVs for Ebola virus other than Zaire (Sudan, Bundibugyo, Tai Forest and Reston species) and Marburg virus (Angola strain) were also generated, and the inhibitory effect was confirmed by a similar method as that in Working Example 95 (Fig.2).

In viral infection-inhibiting examination (pseudotype virus) using cultured cells, Compound Nos. 4-17, 4-20, 4-21 and 4-27 showed IC₅₀ values of about 50 nM against Zaire Ebola virus, about 300 nM against Sudan Ebola virus, about 50 nM against Bundibugyo Ebola virus, about 0.5-1 µM against Tai Forest Ebola virus, about 1 µM against Reston Ebola virus, and about 1-5 µM against Marburg virus.

## Claims

1. A compound of Formula (I): 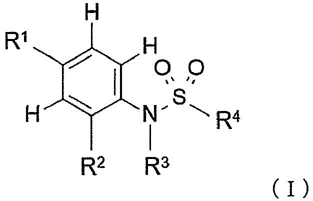 wherein,
R¹ is H, Hal, Alk or OAlk,
R² is NO₂, Alk or Ar,
R³ is 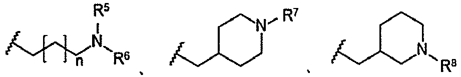
or 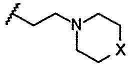 wherein,
n is any of 0 to 5,
R⁵ to R⁸ are each independently H, Alk or COOAlk,
X is CH₂ or O,
R⁴ is 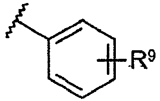
or a naphthyl group, wherein,
R⁹ is H, Hal, Alk or OAlk,
wherein
Alk is each independently a linear or branched alkyl group having 1 to 10 carbon atoms,
Hal is each independently a halogen,
Ar is each independently an aryl group which may optionally be substituted with Hal,
or a hydrate thereof or a pharmaceutically acceptable salt of the compound or a hydrate thereof.

2. The compound of claim 1 or a hydrate thereof or a pharmaceutically acceptable salt of the compound or a hydrate thereof, wherein n is 0 or 1.

3. The compound of claim 1 or 2 or a hydrate thereof or a pharmaceutically acceptable salt of the compound or a hydrate thereof, wherein Alk is a linear or branched alkyl group having 1 to 4 carbon atoms.

4. The compound of any one of claims 1 to 3 or a hydrate thereof or a pharmaceutically acceptable salt of the compound or a hydrate thereof, wherein Ar is an aryl group substituted with Hal.

5. The compound of any one of claims 1 to 4 or a hydrate thereof or a pharmaceutically acceptable salt of the compound or a hydrate thereof, wherein Hal is F.

6. The compound of any one of claims 1 to 5 or a hydrate thereof or a pharmaceutically acceptable salt of the compound or a hydrate thereof, wherein R⁴ is a phenyl group, a fluorinated phenyl group, a phenyl group substituted with a methyl group or methoxy group,

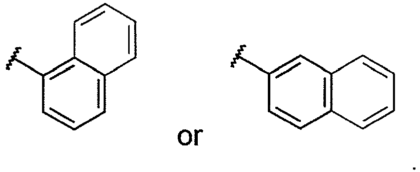

7. The compound of claims 1, wherein the compound is of the following chemical structure formulae:
| No. | Name | Chemical structural formula |
|---|---|---|
| 4-21 | N-(4-methoxy-2-phenyl-phenyl)-N-[1-ethyl-4-piperidyl]methyl]benzensulfonamide | 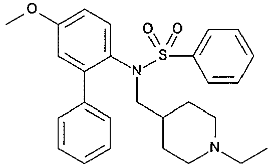 |
| 4-17 | N-[3-(diethylamino)propyl]-N-(4-methoxy-2-phenyl-phenyl)benzensulfonamide | 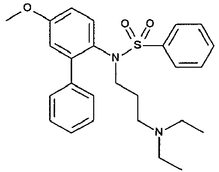 |
| 4-20 | N-(4-methoxy-2-phenyl-phenyl)-N-[1-methyl-4-piperidyl]methyl]benzensulfonamide | 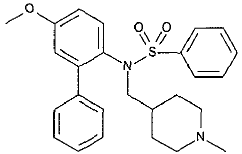 |
| 4-27 | N-[3-(dimethylamino)propyl]-N-(4-methoxy-2-phenyl-phenyl)benzensulfonamide | 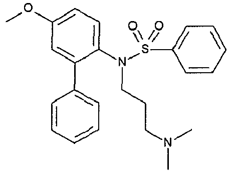 |
| 4-51 | N-[2-(4-fluorophenyl)-4-methoxyphenyl)-N-(1-ethyl-4-piperidylmethyl)benzensulfonamide | 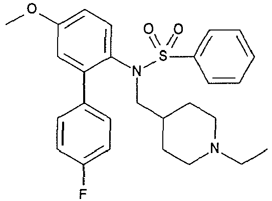 |
| 4-54 | N-[2-(3-fluorophenyl)-4-methoxyphenyl)-N-(-ethyl-4-piperidy[methyl)benzensulfonamide | 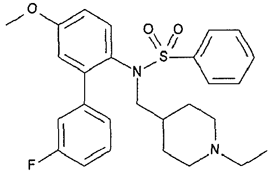 |
| 4-63 | 3-fluoro-N-(4-methoxy-2-phenylphenyl)-N-(1-ethyl-4-piperidylmethyl)benzensulfonamide | 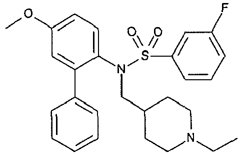 |
| 4-36 | N-[1-isopropyl-4-piperidyl]methyl]-N-(4-methoxy-2-phenylphenyl)benzensulfonamide | 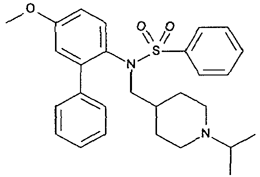 |
| 4-25 | N-(4-methoxy-2-phenyl-phenyl)-N-[1 - ethyl-3-piperidyl]methyl]benzensulfonamide | 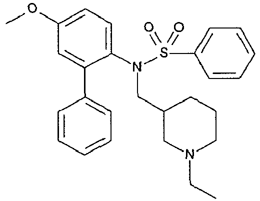 |
| 4-57 | N-[2-(2-fluorophenyl)-4-methoxyphenyl)-N-(1-ethyl-4-piperidylmethyl)benzensulfonamide | 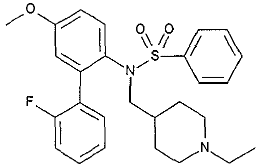 |
| 4-66 | 2-fluoro-N-(4-methoxy-2-phenylphenyl)-N-(1-ethyl-4-piperidylmethyl)benzensulfonamide | 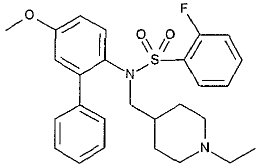 |
| 4-42 | N-(4-methoxy-2-phenyl-phenyl)-N-[1-methyl-3-piperidyl]methyl]benzensulfonamide | 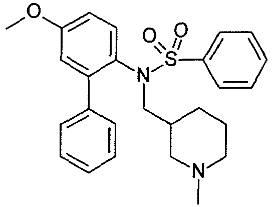 |
| 4-60 | 4-fluoro-N-(4-methoxy-2-phenylphenyl)-N-(1-ethyl-4-piperidylmethyl)benzensulfonamide | 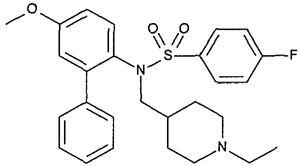 |
| 4-14 | 4-methoxy-N-[3-(diethylamino)propyl]-N-(2-phenylphenyl)benzensulfonamide | 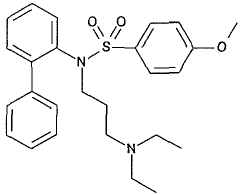 |
| 4-1 | N-[3-(diethylamino)propyl]-N-(4-methoxy-2-nitrophenyl)benzensulfonamide | 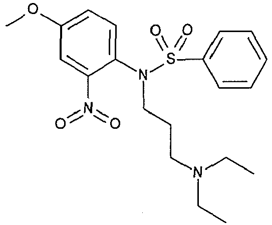 |
| 4-11 | 4-methoxy-N-[3-(diethylamino)propyl]-N-(4-methoxy-2-nitrophenyl)benzensulfonamide | 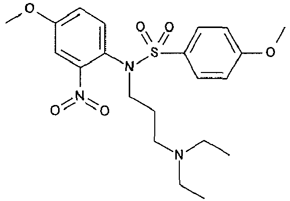 |
| 4-33 | N-[3-(dimethylamino)ethyl]-N-(4-methoxy-2-phenylphenyl)benzensulfonamide | 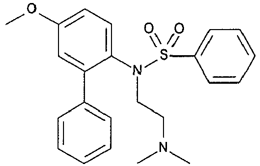 |
| 4-8 | N-[3-(diethylamino)propyl]-N-(2-phenyl-phenyl)benzensulfonam ide | 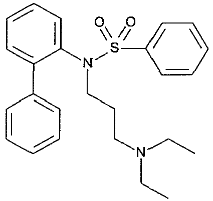 |
| 4-9 | N-[3-(diethylamino)propyl]-N-(4-methoxy 2-nitro-phenyl)naphthalene-1-sulfonamide | 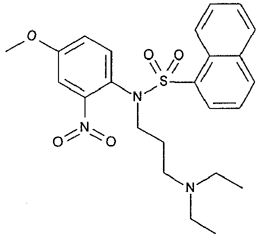 |
| 4-28 | N-[3-(diethylamino)ethyl]-N-(4-methoxy-2-phenylphenyl)benzensulfonamide | 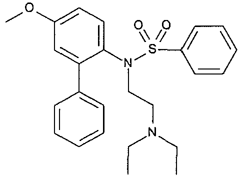 |
| 4-4 | 3-fluoro-N-[3-(diethylamino)propyl]-N-(4-methoxy-2-nitrophenyl)benzensulfonamide | 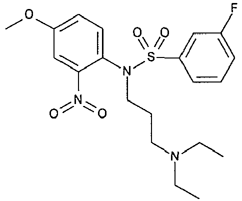 |
| 4-30 | N-(4-methoxy-2-phenyl-phenyl)-N-[3-(methylamino)propyl]-benzensulfonamide | 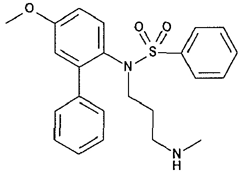 |
| 4-7 | 2-methyl-N-[3-(diethylamino)propyl]-N-(4-methoxy-2-nitrophenyl)benzensulfonamide | 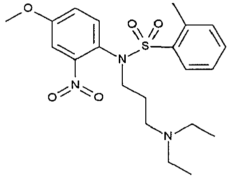 |
| 4-32 | N-(3-aminopropyl)-N-(4-methoxy-2-phenyl-phenyl)benzensulfonam ide | 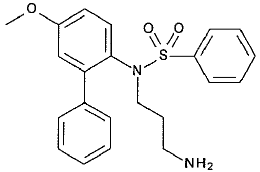 |
| 4-5 | 2-fluoro-N-[3-(diethylamino)propyl]-N-(4-methoxy-2-nitrophenyl)benzensulfonamide | 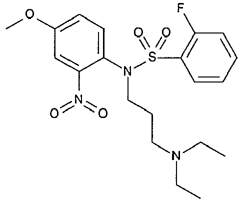 |
| 4-3 | 4-fluoro-N-[3-(diethylamino)propyl]-N-(4-methoxy-2-nitrophenyl)benzensulfonamide | 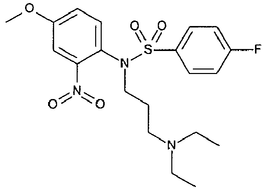 |
| 4-19 | N-(4-methoxy-2-phenyl-phenyl)-N-(4-piperidylmethyl)benzensulfonamide | 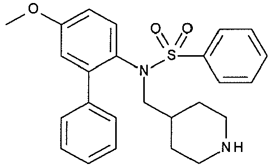 |
| 4-6 | 3-methyl-N-[3-(diethylamino)propyl]-N-(4-methoxy-2-nitrophenyl)benzensulfonamide | 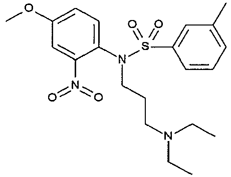 |
| 4-45 | N-(4-isopropyl-2-phenyl-phenyl)-N-(1-ethyl-4-piperidylmethyl)benzensulfonamide | 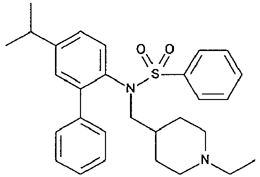 |
| 4-10 | N-[3-(diethylamino)propyl]-N-(4-methoxy 2-nitro-phenyl)naphthalene-2-sulfonamide | 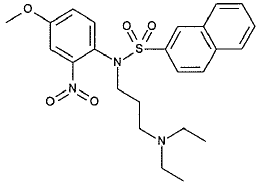 |
| 4-23 | N-(4-methoxy-2-phenyl-phenyl)-N-(3-piperidylmethyl)benzensulfonamide | 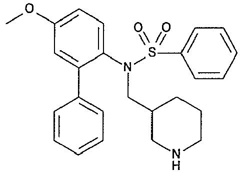 |
| 4-15 | N-[3-(diethylamino)propyl]-N-(2-isopropyl-phenyl)benzensulfonamide | 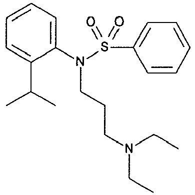 |
| 4-35 | N-(4-methoxy-2-phenyl-phenyl)-N-[2-(1-piperidyl)ethyl]benzensulfonamide | 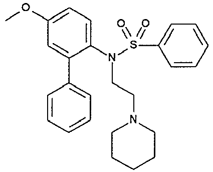 |
| 4-29 | t-butyl-N-[3-[N-(benzensulfonyl)-4-methoxy-2-phenyl-anilino]propyl]-N-methylcarbamate | 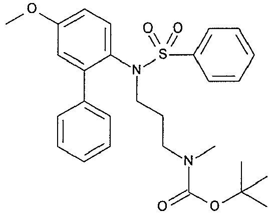 |
| 4-34 | N-(4-methoxy-2-phenyl-phenyl)-N-(2-morpholinoethyl)benzensulfonamide | 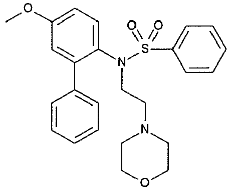 |
| 4-16 | N-[3-(diethylamino)propyl]-N-(2-t-butylphenyl)benzensulfonamide | 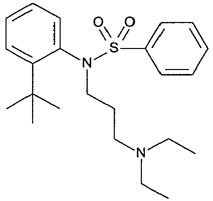 |
| 4-31 | t-butyl-N-[3-[N-(benzensulfonyl)-4-methoxy-2-phenylanilino]propyl]carbamate | 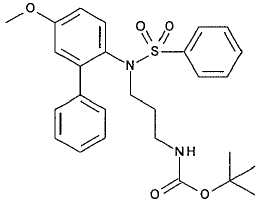 |
| 4-22 | t-butyl-3-[[N-(benzensulfonyl)-4-methoxy-2-phenylanilino]methyl]piperidine-1-carboxylate | 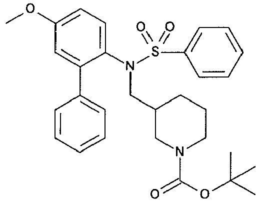 |
| 4-39 | N-(4-ethoxy-2-phenyl-phenyl)-N-[1-ethyl-4-piperidyl]methyl]benzensulfonamide | 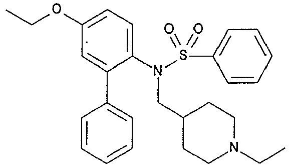 |
| 4-13 | N-[3-(diethylamino)propyl]-N-(2-phenyl-phenyl)naphthalene-1-sulfonamide | 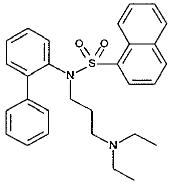 |
| 4-18 | t-butyl-4-[[N-(benzensulfonyl)-4-methoxy-2-phenylanilino]methyl]piperidine-1-carboxylate | 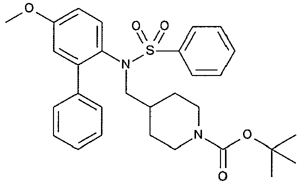 |
| 4-2 | N-[3-(diethylamino)propyl]-N-(2-nitrophenyl)benzensulfonamide | 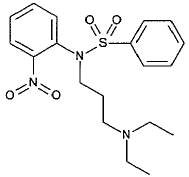 |
| 4-48 | N-(4-t-butyl-2-phenyl-phenyl)-N-(1-ethyl-4-piperidylmethyl)benzensulfonamide | 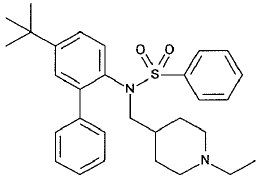 |
| 4-67 | N-[3-(dimethylamino)propyl]-N-[2-(4-fluorophenyl)-4-methoxyphenyl)benzensulfonamide | 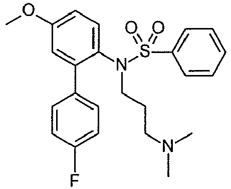 |
| 4-68 | N-[3-(diethylamino)propyl]-N-[2-(4-fluorophenyl)-4-methoxyphenyl)benzensulfonamide | 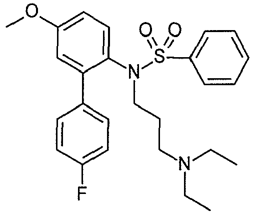 |
or a hydrate thereof or a pharmaceutically acceptable salt of the compound or a hydrate thereof.

8. A pharmaceutical composition comprising the compound of any one of claims 1 to 7 or a hydrate thereof or a pharmaceutically acceptable salt of the compound or a hydrate thereof.

9. The pharmaceutical composition of claim 8, wherein the pharmaceutical composition is for prophylaxis and/or treatment of a viral infection.

10. The pharmaceutical composition of claim 9, wherein the viral infection is an acute viral infection.

11. The pharmaceutical composition of claim 10, wherein the acute viral infection is Ebola hemorrhagic fever.

12. The pharmaceutical composition of claim 9, wherein the virus is a filovirus.

13. The pharmaceutical composition of claim 12, wherein the filovirus is Ebola virus.

14. An antiviral agent comprising the compound of any one of claims 1 to 7 or a hydrate thereof or a pharmaceutically acceptable salt of the compound or a hydrate thereof.

15. A viral cell-invasion inhibitor comprising the compound of any one of claims 1 to 7 or a hydrate thereof or a pharmaceutically acceptable salt of the compound or a hydrate thereof.

16. A method of inhibiting viral cell-invasion processes *in vitro* using the compound of any one of claims 1 to 7 or a hydrate thereof or a pharmaceutically acceptable salt of the compound or a hydrate thereof.
